(19) **Europäisches Patentamt / European Patent Office / Office européen des brevets**

(11) **EP 4 703 384 A1**

(12) **EUROPEAN PATENT APPLICATION**
published in accordance with Art. 153(4) EPC

(43) Date of publication:
04.03.2026 Bulletin 2026/10

(21) Application number: 24796213.7

(22) Date of filing: 26.04.2024

(51) International Patent Classification (IPC):
$C07K\ 16/28^{(2006.01)}$ $A61K\ 39/395^{(2006.01)}$
$A61P\ 35/02^{(2006.01)}$

(52) Cooperative Patent Classification (CPC):
A61K 39/395; A61P 35/02; C07K 16/28

(86) International application number:
PCT/CN2024/089962

(87) International publication number:
WO 2024/222839 (31.10.2024 Gazette 2024/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.04.2023 CN 202310484445

(71) Applicant: Chia Tai Tianqing Pharmaceutical
Group Co., Ltd.
Lianyungang, Jiangsu 222062 (CN)

(72) Inventors:
• ZHANG, Bing
Lianyungang, Jiangsu 222062 (CN)

• LU, Yamin
Lianyungang, Jiangsu 222062 (CN)
• DU, Min
Lianyungang, Jiangsu 222062 (CN)
• LU, Zhenzhen
Lianyungang, Jiangsu 222062 (CN)
• LU, Miaomiao
Lianyungang, Jiangsu 222062 (CN)

(74) Representative: Lavoix
Bayerstraße 83
80335 München (DE)

Remarks:
The complete document including Reference
Table(s) and the Sequence Listing(s) can be
downloaded from the EPO website

(54) **BISPECIFIC ANTIBODY TARGETING G PROTEIN-COUPLED RECEPTOR**

(57) Provided is a bispecific antibody targeting a G protein-coupled receptor. Specifically, provided are a bispecific antibody, a nucleic acid encoding same, a vector comprising the nucleic acid, a cell comprising the vector, and a pharmaceutical composition comprising a multi-specific antibody; and further provided is the use thereof in the treatment of related diseases in a subject.

EP 4 703 384 A1

## Description

### CROSS-REFERENCE

The present application claims the benefit to the Chinese Patent Application No. CN202310484445.0 filed on April 28, 2023, the content of which is incorporated herein in its entirety.

### SEQUENCE LISTING FILE SUBMITTED SIMULTANEOUSLY

The entire content of the following XML file is incorporated herein by reference in its entirety: a sequence listing in computer readable format (CRF) (name: TFH01011PCT-sequence listing.xml, date: April 24, 2024, size: 119KB).

### TECHNICAL FIELD

The present disclosure relates to a bispecific antibody, and in particular to a bispecific antibody targeting GPRC5D and CD3. The present disclosure further relates to a method for preparing the bispecific antibody and use thereof.

### BACKGROUND

Multiple myeloma (MM) is the second most common hematologic tumor following non-Hodgkin's lymphoma, and its pathological state is characterized by abnormal proliferation of plasmocytes in the bone marrow, which overwhelms the development of normal blood cells, resulting in cytopenia, bone damage and kidney injury. It is estimated that there were 16,500 new cases of multiple myeloma and 10,300 deaths in China in 2016, and the incidence of MM in China is estimated to be 1.6/100,000.

GPRC5D (G protein-coupled receptor class C group 5 member D) is a member of the retinoic acid-inducible orphan G protein-coupled receptor (RAIG) family. It is a 7-transmembrane protein consisting of 345 amino acid residues with its normal physiological function associated with the structure of hard keratin. However, no specific biological function or ligand of GPRC5D has been disclosed at present. Some studies have shown that GPRC5D has an expression threshold of over 50% on malignant bone marrow-derived plasmocytes in 65% of patients with multiple myeloma, and that its expression is independent of BCMA (B-cell maturation antigen). Other studies have shown that the overexpression of GPRC5D correlates with tumor burden and poor prognosis in patients with multiple myeloma. GPRC5D has no or very low expression in normal tissues, except for hair follicle tissues of the skin and bone marrow-derived plasmocytes. GPRC5D may provide a new therapeutic pathway with clinical prospects for patients with multiple myeloma.

As a potential therapeutic target, some antibodies targeting GPRC5D have been developed, which, however, are still limited. There is a need for more available options.

### BRIEF SUMMARY

The present disclosure provides a bispecific antibody targeting GPRC5D and CD3, and further provides related nucleic acids capable of encoding the provided antibody, vectors, cells, pharmaceutical compositions, preparation methods, and use.

In one aspect, the present disclosure provides a bispecific antibody comprising a first antigen-binding portion binding to GPRC5D and a second antigen-binding portion binding to CD3, wherein the first antigen-binding portion comprises:

(1) an HCDR1 comprising the amino acid sequence set forth in SEQ ID NO: 1, an HCDR2 comprising the amino acid sequence set forth in SEQ ID NO: 59, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;

(2) an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;

(3) an HCDR1 comprising the amino acid sequence set forth in SEQ ID NO: 1, an HCDR2 comprising the amino acid sequence set forth in SEQ ID NO: 60, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;

(4) an HCDR1 comprising the amino acid sequence set forth in SEQ ID NO: 1, an HCDR2 comprising the amino acid sequence set forth in SEQ ID NO: 61, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;

(5) an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14;

(6) an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22;

(7) an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30; or

(8) an HCDR1 comprising the amino acid sequence set forth in SEQ ID NO: 33, an HCDR2 comprising the amino acid sequence set forth in SEQ ID NO: 34, an HCDR3 comprising the amino acid sequence set forth in SEQ ID NO: 35, an LCDR1 comprising the amino acid sequence set forth in SEQ ID NO: 36, an LCDR2 comprising the amino acid sequence set forth in SEQ ID NO: 37, and an LCDR3 comprising the amino acid sequence set forth in SEQ ID NO: 38.

In one aspect, the present disclosure further provides a bispecific antibody comprising a first antigen-binding portion binding to GPRC5D and a second antigen-binding portion binding to CD3, wherein the first antigen-binding portion comprises:

(1) an HCDR1, an HCDR2, and an HCDR3 in a heavy chain variable region having the amino acid sequence set forth in SEQ ID NO: 7, and an LCDR1, an LCDR2, and an LCDR3 in a light chain variable region having the amino acid sequence set forth in SEQ ID NO: 8;

(2) an HCDR1, an HCDR2, and an HCDR3 in a heavy chain variable region having the amino acid sequence set forth in SEQ ID NO: 15, and an LCDR1, an LCDR2, and an LCDR3 in a light chain variable region having the amino acid sequence set forth in SEQ ID NO: 16;

(3) an HCDR1, an HCDR2, and an HCDR3 in a heavy chain variable region having the amino acid sequence set forth in SEQ ID NO: 23, and an LCDR1, an LCDR2, and an LCDR3 in a light chain variable region having the amino acid sequence set forth in SEQ ID NO: 24;

(4) an HCDR1, an HCDR2, and an HCDR3 in a heavy chain variable region having the amino acid sequence set forth in SEQ ID NO: 31, and an LCDR1, an LCDR2, and an LCDR3 in a light chain variable region having the amino acid sequence set forth in SEQ ID NO: 32;

(5) an HCDR1, an HCDR2, and an HCDR3 in a heavy chain variable region having the amino acid sequence set forth in SEQ ID NO: 39, and an LCDR1, an LCDR2, and an LCDR3 in a light chain variable region having the amino acid sequence set forth in SEQ ID NO: 40;

(6) an HCDR1, an HCDR2, and an HCDR3 in a heavy chain variable region having the amino acid sequence set forth in SEQ ID NO: 62, and an LCDR1, an LCDR2, and an LCDR3 in a light chain variable region having the amino acid sequence set forth in SEQ ID NO: 67;

(7) an HCDR1, an HCDR2, and an HCDR3 in a heavy chain variable region having the amino acid sequence set forth in SEQ ID NO: 63, and an LCDR1, an LCDR2, and an LCDR3 in a light chain variable region having the amino acid sequence set forth in SEQ ID NO: 67;

(8) an HCDR1, an HCDR2, and an HCDR3 in a heavy chain variable region having the amino acid sequence set forth in SEQ ID NO: 64, and an LCDR1, an LCDR2, and an LCDR3 in a light chain variable region having the amino acid sequence set forth in SEQ ID NO: 67;

(9) an HCDR1, an HCDR2, and an HCDR3 in a heavy chain variable region having the amino acid sequence set forth in SEQ ID NO: 65, and an LCDR1, an LCDR2, and an LCDR3 in a light chain variable region having the amino acid sequence set forth in SEQ ID NO: 67;

(10) an HCDR1, an HCDR2, and an HCDR3 in a heavy chain variable region having the amino acid sequence set forth in SEQ ID NO: 66, and an LCDR1, an LCDR2, and an LCDR3 in a light chain variable region having the amino acid sequence set forth in SEQ ID NO: 67; or

(11) an HCDR1, an HCDR2, and an HCDR3 in a heavy chain variable region having the amino acid sequence set forth in SEQ ID NO: 68, and an LCDR1, an LCDR2, and an LCDR3 in a light chain variable region having the amino acid sequence set forth in SEQ ID NO: 69.

In some embodiments, the first antigen-binding portion comprises:

(1) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;

(2) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;

(3) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 23, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24;

(4) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 31, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32;

(5) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 39, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40;

(6) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(7) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(8) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(9) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(10) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 66, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67; or

(11) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69.

In some embodiments, the second antigen-binding portion comprises: an HCDR1 comprising the amino acid sequence set forth in SEQ ID NO: 94, an HCDR2 comprising the amino acid sequence set forth in SEQ ID NO: 95, an HCDR3 comprising the amino acid sequence set forth in SEQ ID NO: 96, an LCDR1 comprising the amino acid sequence set forth in SEQ ID NO: 97, an LCDR2 comprising the amino acid sequence set forth in SEQ ID NO: 98, and an LCDR3 comprising the amino acid sequence set forth in SEQ ID NO: 99.

In some embodiments, the second antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 100, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 101.

In some specific embodiments, the bispecific antibody comprises a first antigen-binding portion binding to GPRC5D and a second antigen-binding portion binding to CD3, wherein the first antigen-binding portion comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6; the second antigen-binding portion comprises: an HCDR1 comprising the amino acid sequence set forth in SEQ ID NO: 94, an HCDR2 comprising the amino acid sequence set forth in SEQ ID NO: 95, an HCDR3 comprising the amino acid sequence set forth in SEQ ID NO: 96, an LCDR1 comprising the amino acid sequence set forth in SEQ ID NO: 97, an LCDR2 comprising the amino acid sequence set forth in SEQ ID NO: 98, and an LCDR3 comprising the amino acid sequence set forth in SEQ ID NO: 99.

In some other specific embodiments, the bispecific antibody comprises a first antigen-binding portion binding to GPRC5D and a second antigen-binding portion binding to CD3, wherein the first antigen-binding portion comprises: an HCDR1 comprising the amino acid sequence set forth in SEQ ID NO: 33, an HCDR2 comprising the amino acid sequence set forth in SEQ ID NO: 34, an HCDR3 comprising the amino acid sequence set forth in SEQ ID NO: 35, an LCDR1 comprising the amino acid sequence set forth in SEQ ID NO: 36, an LCDR2 comprising the amino acid sequence set forth in SEQ ID NO: 37, and an LCDR3 comprising the amino acid sequence set forth in SEQ ID NO: 38; the second antigen-binding portion comprises: an HCDR1 comprising the amino acid sequence set forth in SEQ ID NO: 94, an HCDR2 comprising the amino acid sequence set forth in SEQ ID NO: 95, an HCDR3 comprising the amino acid sequence set forth in SEQ ID NO: 96, an LCDR1 comprising the amino acid sequence set forth in SEQ ID NO: 97, an LCDR2 comprising the amino acid sequence set forth in SEQ ID NO: 98, and an LCDR3 comprising the amino acid sequence set forth in SEQ ID NO: 99.

In some specific embodiments, the bispecific antibody comprises a first antigen-binding portion binding to GPRC5D and a second antigen-binding portion binding to CD3, wherein the first antigen-binding portion comprises: a heavy chain variable region comprising the amino acid sequence set forth in SEQ ID NO: 62 and a light chain variable region comprising the amino acid sequence set forth in SEQ ID NO: 67; the second antigen-binding portion comprises: a heavy chain variable region comprising the amino acid sequence set forth in SEQ ID NO: 100 and a light chain variable region comprising the amino acid sequence set forth in SEQ ID NO: 101.

In some other specific embodiments, the bispecific antibody comprises a first antigen-binding portion binding to GPRC5D and a second antigen-binding portion binding to CD3, wherein the first antigen-binding portion comprises: a heavy chain variable region comprising the amino acid sequence set forth in SEQ ID NO: 68 and a light chain variable region comprising the amino acid sequence set forth in SEQ ID NO: 69; the second antigen-binding portion comprises: a heavy chain variable region comprising the amino acid sequence set forth in SEQ ID NO: 100 and a light chain variable region comprising the amino acid sequence set forth in SEQ ID NO: 101.

In some embodiments, the bispecific antibody of the present disclosure comprises an Fc domain composed of two Fc polypeptides.

In some embodiments, the first antigen-binding portion and the second antigen-binding portion are each independently Fab, scFv, scFab (single-chain Fab), or other structures. In some embodiments, the first antigen-binding portion is a Fab and the second antigen-binding portion is an scFv.

In some embodiments, for the bispecific antibody of the present disclosure, the first antigen-binding portion is a Fab, and the second antigen-binding portion is an scFv; the first antigen-binding portion, at the C-terminus of its Fab heavy chain or Fab light chain, is fused to the N-terminus of one of the Fc polypeptides of the Fc domain, and the second antigen-binding portion, at the C-terminus, is fused to the N-terminus of the other Fc polypeptide of the Fc domain. In further more specific embodiments, the bispecific antibody consists of three polypeptide chains, wherein the first polypeptide chain comprises a Fab light chain of the first antigen-binding portion described above, the second polypeptide chain comprises a Fab heavy chain of the first antigen-binding portion described above and one of the Fc polypeptides of the Fc domain, and the third polypeptide chain comprises the second antigen-binding portion described above and the other Fc polypeptide of the Fc domain. In some embodiments, the bispecific antibody is bivalent.

In another aspect, the present disclosure provides an antibody binding to GPRC5D or an antigen-binding portion thereof comprising:

(1) an HCDR1 comprising the amino acid sequence set forth in SEQ ID NO: 1, an HCDR2 comprising the amino acid sequence set forth in SEQ ID NO: 59, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;
(2) an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;
(3) an HCDR1 comprising the amino acid sequence set forth in SEQ ID NO: 1, an HCDR2 comprising the amino acid sequence set forth in SEQ ID NO: 60, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;
(4) an HCDR1 comprising the amino acid sequence set forth in SEQ ID NO: 1, an HCDR2 comprising the amino acid sequence set forth in SEQ ID NO: 61, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;
(5) an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14;
(6) an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22;
(7) an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30; or
(8) an HCDR1 comprising the amino acid sequence set forth in SEQ ID NO: 33, an HCDR2 comprising the amino acid sequence set forth in SEQ ID NO: 34, an HCDR3 comprising the amino acid sequence set forth in SEQ ID NO: 35, an LCDR1 comprising the amino acid sequence set forth in SEQ ID NO: 36, an LCDR2 comprising the amino acid sequence set forth in SEQ ID NO: 37, and an LCDR3 comprising the amino acid sequence set forth in SEQ ID NO: 38.

In one aspect, the present disclosure further provides an antibody binding to GPRC5D or an antigen-binding portion thereof comprising:

(1) an HCDR1, an HCDR2, and an HCDR3 in a heavy chain variable region having the amino acid sequence set forth in SEQ ID NO: 7, and an LCDR1, an LCDR2, and an LCDR3 in a light chain variable region having the amino acid sequence set forth in SEQ ID NO: 8;
(2) an HCDR1, an HCDR2, and an HCDR3 in a heavy chain variable region having the amino acid sequence set forth in SEQ ID NO: 15, and an LCDR1, an LCDR2, and an LCDR3 in a light chain variable region having the amino acid sequence set forth in SEQ ID NO: 16;
(3) an HCDR1, an HCDR2, and an HCDR3 in a heavy chain variable region having the amino acid sequence set forth in SEQ ID NO: 23, and an LCDR1, an LCDR2, and an LCDR3 in a light chain variable region having the amino acid sequence set forth in SEQ ID NO: 24;
(4) an HCDR1, an HCDR2, and an HCDR3 in a heavy chain variable region having the amino acid sequence set forth in SEQ ID NO: 31, and an LCDR1, an LCDR2, and an LCDR3 in a light chain variable region having the amino acid sequence set forth in SEQ ID NO: 32;
(5) an HCDR1, an HCDR2, and an HCDR3 in a heavy chain variable region having the amino acid sequence set forth in SEQ ID NO: 39, and an LCDR1, an LCDR2, and an LCDR3 in a light chain variable region having the amino acid sequence set forth in SEQ ID NO: 40;
(6) an HCDR1, an HCDR2, and an HCDR3 in a heavy chain variable region having the amino acid sequence set forth in SEQ ID NO: 62, and an LCDR1, an LCDR2, and an LCDR3 in a light chain variable region having the amino acid sequence set forth in SEQ ID NO: 67;

(7) an HCDR1, an HCDR2, and an HCDR3 in a heavy chain variable region having the amino acid sequence set forth in SEQ ID NO: 63, and an LCDR1, an LCDR2, and an LCDR3 in a light chain variable region having the amino acid sequence set forth in SEQ ID NO: 67;

(8) an HCDR1, an HCDR2, and an HCDR3 in a heavy chain variable region having the amino acid sequence set forth in SEQ ID NO: 64, and an LCDR1, an LCDR2, and an LCDR3 in a light chain variable region having the amino acid sequence set forth in SEQ ID NO: 67;

(9) an HCDR1, an HCDR2, and an HCDR3 in a heavy chain variable region having the amino acid sequence set forth in SEQ ID NO: 65, and an LCDR1, an LCDR2, and an LCDR3 in a light chain variable region having the amino acid sequence set forth in SEQ ID NO: 67;

(10) an HCDR1, an HCDR2, and an HCDR3 in a heavy chain variable region having the amino acid sequence set forth in SEQ ID NO: 66, and an LCDR1, an LCDR2, and an LCDR3 in a light chain variable region having the amino acid sequence set forth in SEQ ID NO: 67; or

(11) an HCDR1, an HCDR2, and an HCDR3 in a heavy chain variable region having the amino acid sequence set forth in SEQ ID NO: 68, and an LCDR1, an LCDR2, and an LCDR3 in a light chain variable region having the amino acid sequence set forth in SEQ ID NO: 69.

In some embodiments, the antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, 63, 64, 65, 66, or 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67 or 69.

In some embodiments, the antibody binding to GPRC5D or the antigen-binding portion thereof comprises:

(1) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(2) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(3) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(4) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(5) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 66, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69; or

(6) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69.

In some embodiments, the antibody binding to GPRC5D or the antigen-binding portion thereof comprises:

(1) a heavy chain variable region comprising the amino acid sequence set forth in SEQ ID NO: 62 and a light chain variable region comprising the amino acid sequence set forth in SEQ ID NO: 67;

(2) a heavy chain variable region comprising the amino acid sequence set forth in SEQ ID NO: 63 and a light chain variable region comprising the amino acid sequence set forth in SEQ ID NO: 67;

(3) a heavy chain variable region comprising the amino acid sequence set forth in SEQ ID NO: 64 and a light chain variable region comprising the amino acid sequence set forth in SEQ ID NO: 67;

(4) a heavy chain variable region comprising the amino acid sequence set forth in SEQ ID NO: 65 and a light chain variable region comprising the amino acid sequence set forth in SEQ ID NO: 67; or

(5) a heavy chain variable region comprising the amino acid sequence set forth in SEQ ID NO: 68 and a light chain variable region comprising the amino acid sequence set forth in SEQ ID NO: 69.

In one aspect, the present disclosure provides an isolated nucleic acid comprising a nucleotide sequence encoding the bispecific antibody or the antibody or the antigen-binding portion thereof described herein.

In one aspect, the present disclosure provides a vector comprising the nucleic acid described in the present disclosure.

In one aspect, the present disclosure provides a host cell comprising the nucleic acid or the vector described in the present disclosure.

In one aspect, the present disclosure provides an antibody-drug conjugate comprising the antibody binding to GPRC5D or the antigen-binding portion thereof of the present disclosure.

In one aspect, the present disclosure provides an anti-GPRC5D chimeric antigen receptor comprising the antibody binding to GPRC5D of the present disclosure.

In one aspect, the present disclosure provides an engineered immune effector cell comprising the chimeric antigen receptor of the present disclosure.

In another aspect, the present disclosure provides a method for preparing the bispecific antibody, or the antibody or the antigen-binding portion thereof described herein, comprising culturing the host cell to express the bispecific antibody, or the antibody or the antigen-binding portion thereof, and isolating and purifying the bispecific antibody, or the antibody or the antigen-binding portion thereof in a system.

In another aspect, the present disclosure provides a pharmaceutical composition comprising the antibody or the antigen-binding portion thereof, the antibody-drug conjugate, the engineered immune effector cell, or the bispecific antibody, and a pharmaceutically acceptable excipient.

In another aspect, the present disclosure provides use of the antibody or the antigen-binding portion thereof, the antibody-drug conjugate, the engineered immune effector cell, the bispecific antibody, or the pharmaceutical composition for preparing a medicament for treating a GPRC5D-expressing disease.

In another aspect, the present disclosure provides a method for treating a GPRC5D-expressing disease comprising administering to a subject in need the antibody or the antigen-binding portion thereof, the antibody-drug conjugate, the engineered immune effector cell, the bispecific antibody, or the pharmaceutical composition. In another aspect, the present disclosure provides a method for treating a GPRC5D-expressing disease comprising administering to a subject in need a therapeutically effective amount of the antibody or the antigen-binding portion thereof, the antibody-drug conjugate, the engineered immune effector cell, the bispecific antibody, or the pharmaceutical composition.

The bispecific antibody targeting GPRC5D and CD3 provided by the present disclosure exhibits good anti-tumor effects and/or safety.

## BRIEF DESCRIPTION OF THE DRAWINGS

FIG. 1 shows the $OD_{450\ nm}$ of the binding of some mouse anti-hGPRC5D antibodies to CHO-K1-hGPRC5D$^{hi}$ cells, CHO-K1-cynoGPRC5D cells, and CHO-K1-murineGPRC5D cells as determined by an ELISA method;

FIG. 2 shows the MFI and $EC_{50}$ values of the binding of chimeric antibodies to cells with high expression of hGPRC5D as measured by FACS;

FIG. 3 shows the MFI values of the binding of chimeric antibodies to cells with low expression of hGPRC5D as measured by FACS;

FIGs. 4A-4B show the MFI values of the binding of anti-GPRC5D antibodies to different cells as measured by FACS, wherein FIG. 4A shows NCI-H929 cells, and FIG. 4B shows the MFI values of the binding of MM.1S cells;

FIGs. 5A-5B show the MFI values of the binding of anti-GPRC5D antibodies to different cells as measured by FACS, wherein FIG. 5A shows CHO-K1-cynoGPRC5D cells, and FIG. 5B shows CHO-K1-murineGPRC5D cells;

FIGs. 6A-6C show the MFI values of the binding of anti-GPRC5D antibodies to different cells as measured by FACS, wherein FIG. 6A shows CHO-K1-GPRC5A cells, FIG. 6B shows CHO-K1-GPRC5B cells, and FIG. 6C shows CHO-K1-GPRC5C cells;

FIG. 7 shows the MFI values of the binding of anti-GPRC5D antibodies to CHO-K1 cells as measured by FACS;

FIG. 8 shows the MFI values of the binding of chimeric and humanized anti-GPRC5D antibodies to NCI-H929 cells as measured by FACS;

FIG. 9 shows a structural schematic diagram of the exemplary bispecific antibody of the present disclosure;

FIGs. 10A-10C show the MFI values of the binding of anti-GPRC5D/anti-CD3 bispecific antibodies to different cells as measured by FACS, wherein FIG. 10A shows RPMI-8226 cells, FIG. 10B shows MM.1S cells, and FIG. 10C shows NCI-H929 cells;

FIG. 11 shows the MFI values of the binding of anti-GPRC5D/anti-CD3 bispecific antibodies to CD3$^+$ T cells as measured by FACS;

FIGs. 12A-12C show the lysis rates of anti-GPRC5D/anti-CD3 bispecific antibodies to different cells, wherein FIG. 12A shows NCI-H929 cells, FIG. 12B shows MM.1S cells, and FIG. 12C shows RPMI-8226 cells;

FIGs. 13A-13D show the effect of anti-GPRC5D/anti-CD3 bispecific antibodies on T cell activation in the presence of target cells MM.1S, and CD69 and CD25 are T cell activation markers, wherein FIG. 13A shows the proportion of the number of CD25$^+$ cells among the number of CD4$^+$ cells, FIG. 13B shows the proportion of the number of CD69$^+$ cells among the number of CD4$^+$ cells, FIG. 13C shows the proportion of the number of CD25$^+$ cells among the number of CD8$^+$ cells, and FIG. 13D shows the proportion of the number of CD69$^+$ cells among the number of CD8$^+$ cells;

FIGs. 14A-14D show the release amount of different cytokines after different concentrations of anti-GPRC5D/anti-CD3 bispecific antibodies are co-incubated with target cells MM.1S and effector cells PBMC for 24 h, wherein FIG. 14A shows IL-2, FIG. 14B shows IL-6, FIG. 14C shows TNF-$\alpha$, and FIG. 14D shows IFN-$\gamma$;

FIG. 15 shows the effect of anti-GPRC5D/anti-CD3 bispecific antibodies on tumor volume in an NCI-H929 human multiple myeloma mouse xenograft tumor model.

## SUMMARY

### *I. Definitions and Description*

Unless otherwise stated, the following terms used in the present disclosure shall have the following meanings. A certain term, unless otherwise specifically defined, should not be considered uncertain or unclear, but construed according to its common meaning in the art. When referring to a trade name, it is intended to refer to its corresponding commercial product or its active ingredient.

The term "antibody" is used in its broadest sense and encompasses natural antibodies and artificial antibodies of various structures, including, but not limited to, various antibody structures such as monoclonal antibodies, polyclonal antibodies, monospecific antibodies, multispecific antibodies (e.g., bispecific antibodies or trispecific antibodies), and single-chain antibodies, so long as they exhibit the desired antigen-binding activity. The term "multispecific" means that an antibody is capable of specifically binding to a plurality of different antigenic determinants, e.g., capable of specifically binding to two (i.e., "bispecific") or more different antigenic determinants. The antigenic determinant is synonymous with an antigen epitope herein. Generally, a bispecific antibody comprises two antigen-binding sites, each of which is specific for a different antigenic determinant. Different antigenic determinants may be expressed on the same or different cells. Different antigenic determinants may be different depending on different types of antigens (e.g., binding to antigens GPRC5D and CD3) or may be present on the same antigen. An antigenic determinant is a special chemical group with a certain composition and structure on the surface or other parts of the antigenic substance molecule, which can specifically bind to its corresponding antibody or sensitized lymphocyte. An example of the antigenic determinant is GPRC5D, which is an antigenic substance having various structurally defined or structurally undefined antigenic determinants. A specific bispecific antibody can, for example, bind to GPRC5D and CD3.

The term "antigen-binding portion" refers to a polypeptide molecule specifically binding to an antigenic determinant. A specific antigen-binding portion may be, for example, Fab, scFv, scFab, or the like.

The term "first", "second", or "third" used herein with respect to an antigen-binding portion, an antigen, an Fc polypeptide, a peptide linker, a polypeptide chain, and the like are used for ease of distinction when more than one part of each type is present. Unless specifically stated, the use of these terms is not intended to confer a particular order or orientation.

The term "fusion" means that the components (e.g., antigen-binding portions, Fc polypeptides, etc.) are linked either directly or by peptide bonds via one or more peptide linkers. For example, some peptide linkers consist of 1 to 50 amino acids linked by peptide bonds, wherein the amino acids may be selected from 20 naturally occurring amino acids; in a more preferred embodiment, the 1 to 50 amino acids are selected from glycine, alanine, proline, serine, asparagine, glutamine, and lysine.

The term "variable domain" or "variable region" refers to a domain of an antibody that is involved in the binding of the antibody to an antigen. For example, a natural four-chain antibody (e.g., one derived from a human, a murine, or other mammals) comprises a heavy chain variable region (also referred to as heavy chain variable domain, VH, or VH domain) and a light chain variable region (also referred to as light chain variable domain, VL, or VL domain). In most cases, each variable domain of a natural antibody consists substantially of four "framework regions (FRs)" and three "complementarity determining regions (CDRs)". The four framework regions are referred to as framework region 1 (or FR1), framework region 2 (or FR2), framework region 3 (or FR3), and framework region 4 (or FR4). The framework regions are separated by three complementarity determining regions referred to in the art and hereinafter as complementarity determining region 1 (or CDR1), complementarity determining region 2 (or CDR2), and complementarity determining region 3 (or CDR3). Thus, the general structure of a variable domain can be represented as follows: FR1-CDR1-FR2-CDR2-FR3-CDR3-FR4. Variable domains impart specificity for an antigen to an antibody by virtue of having an antigen-binding site.

The term "complementarity determining region" (CDR) is also referred to as "hypervariable region" (HVR). A natural four-chain antibody typically comprises six CDRs, among which three are in the heavy chain variable region, i.e., heavy chain CDR1 (HCDR1), heavy chain CDR2 (HCDR2), and heavy chain CDR3 (HCDR3), and three are in the light chain variable region, i.e., light chain CDR1 (LCDR1), light chain CDR2 (LCDR2), and light chain CDR3 (LCDR3).

There are currently many ways to define CDRs. The Kabat scheme defines CDRs based on the sequence variability and is the most commonly used (Elvin A. Kabat, et al., Sequences of Proteins of Immunological Interest, 5th Edition, Public Health Service, National Institutes of Health, Bethesda, Md. (1991)), while the Chothia scheme defines CDRs based on the positions of structural loops (Cyrus Chothia, et al., Canonical Structures for the Hypervariable Regions of Immunoglobulins, J. Mol. Biol. 196:901-917(1987)). The AbM scheme, a compromise between the Kabat scheme and the Chothia scheme, is used by the Oxford Molecular's AbM antibody modeling software. The "Contact" defines CDRs based on the analysis of the crystal structure of available complexes. Additionally, there is a definition of CCG. However, it should be noted that boundaries of the CDRs of variable regions of the same antibody based on different methods and definitions may differ, that is, CDR sequences of the variable regions of the same antibody defined by different methods may differ. Thus, when reference is made to an antibody defined by a specific CDR sequence, the scope of the antibody also encompasses antibodies defined by CDR sequences under any other definitions (e.g., one of or a combination of several of the definitions such as Kabat, IMGT, Chothia, Contact, AbM, CCG).

The term "Fab" refers to a protein consisting of VH and CH1 domains of the heavy chain and VL and CL domains of the light chain of an immunoglobulin. The Fab herein refers to an Fab molecule in its natural form or a modified Fab molecule, i.e., comprising an Fab heavy chain consisting of a heavy chain variable region VH and a constant region CH1 (VH-CH1, in a direction from N-terminus to C-terminus), and an Fab light chain consisting of a light chain variable region VL and a constant region CL (VL-CL, in a direction from N-terminus to C-terminus). The modified Fab may be, for example, a Fab introducing an amino acid substitution into the CH1/CL domain or/and the VH/VL domain. As a specific example, the modified Fab may be a Fab introducing an amino acid substitution into the CL domain.

The term "scFv" includes the VH and VL domains of an immunoglobulin, wherein these domains are present in a single polypeptide chain. In some embodiments, scFv further comprises a peptide linker between the VH and VL domains that enables the scFv to form the required structure for antigen-binding. In a specific embodiment, the scFv is arranged from N-terminus to C-terminus in the following order: VL-linker-VH.

The term "Fc domain" or "Fc" is used herein to define the C-terminal region of an immunoglobulin heavy chain, which comprises at least part of a constant region. The term includes natural sequence Fc and variant Fc. The C-terminal lysine (Lys447) of the Fc may or may not be present. Unless otherwise stated, amino acid residues in the Fc or constant region are numbered according to the EU numbering system, also referred to as the EU index, as described in Kabat, E.A. et al., Sequences of Proteins of Immunological Interest, 5th Ed., Public Health Service, National Institutes of Health, Bethesda, MD (1991), NIH Publication 91-3242. As used herein, one of "Fc polypeptides" of an Fc domain refers to one of the two polypeptides that form a dimeric Fc domain. For example, the Fc polypeptide of an IgG Fc domain comprises IgG CH2 and IgG CH3.

The term "treating" or "treatment" means administering the compound (e.g., a monospecific antibody or a bispecific antibody) or pharmaceutical composition described in the present disclosure to prevent, ameliorate, or eliminate a disease or one or more symptoms associated with the disease, including but not limited to: (i) preventing the occurrence of a disease or disease state in a mammal, particularly when such a mammal is predisposed to the disease state but has not yet been diagnosed with it; (ii) inhibiting a disease or disease state, i.e., arresting its progression; (iii) alleviating a disease or disease state, i.e., causing its regression; and (iv) reducing any direct or indirect pathological consequences of a disease or disease state.

The term "therapeutically effective amount" refers to an amount of the compound of the present disclosure for (i) treating or preventing a specific disease, condition, or disorder; (ii) relieving, ameliorating, or eliminating one or more symptoms of a specific disease, condition, or disorder, or (iii) preventing or delaying the onset of one or more symptoms of the specific disease, condition, or disorder described herein. The amount of the monospecific antibody, the bispecific antibody, the antibody-drug conjugate, the engineered immune effector cell, or the pharmaceutical composition of the present disclosure that constitutes a "therapeutically effective amount" may vary depending on factors such as the compound or the pharmaceutical composition and its ability to elicit a desired response in an individual, the disease state and its severity, the mode of administration, and the age, sex, and weight of the mammal to be treated. The therapeutically effective amount may also be determined routinely by those skilled in the art following their knowledge and the present disclosure.

The term "pharmaceutically acceptable" is used herein for those compounds, materials, compositions, and/or dosage forms, etc., that are, within the scope of sound medical judgment, suitable for use in contact with the tissues of human beings and animals without excessive toxicity, irritation, allergic response, or other problems or complications, and commensurate with a reasonable benefit/risk ratio.

The term "excipient" refers to any ingredient other than the active ingredient (e.g., the antibody of the present disclosure). The selection of the excipient will largely depend on factors such as the particular mode of administration, the effect of the excipient on solubility and stability, and the nature of the dosage form.

The term "isolated" means that a target compound, such as an antibody or an antigen-binding fragment thereof, or a nucleic acid, has been isolated from its natural environment.

The terms "$X_n$" and "Xaa" are equivalent and refer to an unspecified amino acid whose scope is specified by the subsequent definitions in the relevant description.

As used herein, the term "$EC_{50}$", i.e., the half-maximal effective concentration, refers to the concentration of an antibody at which the induced response reaches 50% of the maximal response, representing the midpoint between the maximal response and baseline. $EC_{50}$ can be measured by ELISA or FACS analysis or any other method known in the art.

"$K_D$" refers to the equilibrium dissociation constant, which is derived from the ratio of the dissociation rate constant ($k_d$) to the association rate constant ($k_a$) (i.e., $k_d/k_a$), and is expressed in molar concentration (M). The $K_D$ value of an antibody may be determined using methods well established in the art. A preferred method for determining the $K_D$ of an antibody is surface plasmon resonance (SPR), preferably using a biosensor system such as the Biacore surface plasmon resonance system for analysis.

The term "identity" is also referred to as consistency. The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. Alignment of amino acid sequences for identity can be performed in a variety of ways within the skill in the art, such as BLAST, BLAST-2, ALIGN, or Megalign (DNASTAR) software. Those skilled in the art can determine suitable parameters for aligning sequences, including any algorithms required to obtain maximum alignment for the full length of sequences being compared.

The term "subject" includes any human or non-human animal. The term "non-human animal" includes all vertebrates, e.g., mammals and non-mammals, such as non-human primates, sheep, dogs, cats, horses, cows, chickens, amphibians, and reptiles. Preferably, the subject according to the present disclosure is a human. The terms "patient" and "subject" are used interchangeably unless otherwise indicated. "Subjects in need" include those with a disease or condition, those at risk of developing the disease or condition, and those who may have the disease or condition and whose purpose is to prevent, delay, or alleviate the disease or condition.

As used herein, "about" means being within an acceptable error range determined by those of ordinary skill in the art for a specific value, which will depend in part on how the value is measured or determined, i.e., the limitation by the measurement system. For example, "about" may mean being within 1 or more than 1 standard deviation, as practiced in the art. Alternatively, "about" may mean a range of up to $\pm 5\%$, for example, fluctuating within a specific numerical range given $\pm 2\%$, $\pm 1\%$, or $\pm 0.5\%$. Where a specific value is given in the scope of the present disclosure, unless otherwise stated, "about" should be considered to mean being within an acceptable error range for that specific value. As used herein, unless otherwise stated, the values of the parameters or conditions in a step are all modified by "about" by default.

The terms "comprise", "comprises", "comprising", and equivalents thereof (e.g., contain, contains, containing, include, includes, and including) are to be construed as "including, but not limited to", meaning that additional unspecified elements, components, and steps may be included in addition to the enumerated elements, components, and steps.

As used herein, unless otherwise specified clearly in the context, singular terms encompass plural referents, and vice versa.

_II. Monospecific antibody or antigen-binding portion thereof_

The present disclosure provides a monospecific antibody binding to GPRC5D or an antigen-binding portion thereof, comprising: an HCDR1 comprising the amino acid sequence set forth in SEQ ID NO: 1, 9, 17, 25, or 33, an HCDR2 comprising the amino acid sequence set forth in SEQ ID NO: 59, 10, 18, 26, or 34, an HCDR3 comprising the amino acid sequence set forth in SEQ ID NO: 3, 11, 19, 27, or 35, an LCDR1 comprising the amino acid sequence set forth in SEQ ID NO: 4, 12, 20, 28, or 36, an LCDR2 comprising the amino acid sequence set forth in SEQ ID NO: 5, 13, 21, 29, or 37, and an LCDR3 comprising the amino acid sequence set forth in SEQ ID NO: 6, 14, 22, 30, or 38.

In some embodiments, the monospecific antibody binding to GPRC5D or an antigen-binding portion thereof comprises: an HCDR1 comprising the amino acid sequence set forth in SEQ ID NO: 1, an HCDR2 comprising the amino acid sequence set forth in SEQ ID NO: 59, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, wherein $X_1$ is selected from D and E, and $X_2$ is selected from G and A. In some specific embodiments, $X_1$ is D, and $X_2$ is G. In some specific embodiments, $X_1$ is D, and $X_2$ is A. In some specific embodiments, $X_1$ is E, and $X_2$ is G.

In some embodiments, the monospecific antibody binding to GPRC5D or an antigen-binding portion thereof comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the monospecific antibody binding to GPRC5D or an antigen-binding portion thereof comprises: an HCDR1 comprising the amino acid sequence set forth in SEQ ID NO: 1, an HCDR2 comprising the amino acid sequence set forth in SEQ ID NO: 60, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the monospecific antibody binding to GPRC5D or an antigen-binding portion thereof comprises: an HCDR1 comprising the amino acid sequence set forth in SEQ ID NO: 1, an HCDR2 comprising the amino acid sequence set forth in SEQ ID NO: 61, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the monospecific antibody binding to GPRC5D or an antigen-binding portion thereof comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the monospecific antibody binding to GPRC5D or an antigen-binding portion thereof comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the monospecific antibody binding to GPRC5D or an antigen-binding portion thereof comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the monospecific antibody binding to GPRC5D or an antigen-binding portion thereof comprises: an HCDR1 comprising the amino acid sequence set forth in SEQ ID NO: 33, an HCDR2 comprising the amino acid sequence set forth in SEQ ID NO: 34, an HCDR3 comprising the amino acid sequence set forth in SEQ ID NO: 35, an LCDR1 comprising the amino acid sequence set forth in SEQ ID NO: 36, an LCDR2 comprising the amino acid sequence set forth in SEQ ID NO: 37, and an LCDR3 comprising the amino acid sequence set forth in SEQ ID NO: 38.

The present disclosure further provides a monospecific antibody binding to GPRC5D or an antigen-binding portion thereof comprising: an HCDR1, an HCDR2, and an HCDR3 in a heavy chain variable region comprising the amino acid sequence set forth in SEQ ID NO: 7, 15, 23, 31, 39, 66, or 68, and an LCDR1, an LCDR2, and an LCDR3 in a light chain variable region comprising the amino acid sequence set forth in SEQ ID NO: 8, 16, 24, 32, 40, 67, or 69.

In some embodiments, the monospecific antibody binding to GPRC5D or an antigen-binding portion thereof comprises: an HCDR1, an HCDR2, and an HCDR3 in a heavy chain variable region having the amino acid sequence set forth in SEQ ID NO: 7, and an LCDR1, an LCDR2, and an LCDR3 in a light chain variable region having the amino acid sequence set forth in SEQ ID NO: 8.

In some embodiments, the monospecific antibody binding to GPRC5D or an antigen-binding portion thereof comprises: an HCDR1, an HCDR2, and an HCDR3 in a heavy chain variable region having the amino acid sequence set forth in SEQ ID NO: 15, and an LCDR1, an LCDR2, and an LCDR3 in a light chain variable region having the amino acid sequence set forth in SEQ ID NO: 16.

In some embodiments, the monospecific antibody binding to GPRC5D or an antigen-binding portion thereof comprises: an HCDR1, an HCDR2, and an HCDR3 in a heavy chain variable region having the amino acid sequence set forth in SEQ ID NO: 23, and an LCDR1, an LCDR2, and an LCDR3 in a light chain variable region having the amino acid sequence set forth in SEQ ID NO: 24.

In some embodiments, the monospecific antibody binding to GPRC5D or an antigen-binding portion thereof comprises: an HCDR1, an HCDR2, and an HCDR3 in a heavy chain variable region having the amino acid sequence set forth in SEQ ID NO: 31, and an LCDR1, an LCDR2, and an LCDR3 in a light chain variable region having the amino acid sequence set forth in SEQ ID NO: 32.

In some embodiments, the monospecific antibody binding to GPRC5D or an antigen-binding portion thereof comprises: an HCDR1, an HCDR2, and an HCDR3 in a heavy chain variable region having the amino acid sequence set forth in SEQ ID NO: 39, and an LCDR1, an LCDR2, and an LCDR3 in a light chain variable region having the amino acid sequence set forth in SEQ ID NO: 40.

In some embodiments, the monospecific antibody binding to GPRC5D or an antigen-binding portion thereof comprises: an HCDR1, an HCDR2, and an HCDR3 in a heavy chain variable region having the amino acid sequence set forth in SEQ ID NO: 62, and an LCDR1, an LCDR2, and an LCDR3 in a light chain variable region having the amino acid sequence set forth in SEQ ID NO: 67.

In some embodiments, the monospecific antibody binding to GPRC5D or an antigen-binding portion thereof comprises: an HCDR1, an HCDR2, and an HCDR3 in a heavy chain variable region having the amino acid sequence set forth in SEQ ID NO: 63, and an LCDR1, an LCDR2, and an LCDR3 in a light chain variable region having the amino acid sequence set forth in SEQ ID NO: 67.

In some embodiments, the monospecific antibody binding to GPRC5D or an antigen-binding portion thereof comprises: an HCDR1, an HCDR2, and an HCDR3 in a heavy chain variable region having the amino acid sequence set forth in SEQ ID NO: 64, and an LCDR1, an LCDR2, and an LCDR3 in a light chain variable region having the amino acid sequence set forth in SEQ ID NO: 67.

In some embodiments, the monospecific antibody binding to GPRC5D or an antigen-binding portion thereof comprises: an HCDR1, an HCDR2, and an HCDR3 in a heavy chain variable region having the amino acid sequence set forth in SEQ ID NO: 65, and an LCDR1, an LCDR2, and an LCDR3 in a light chain variable region having the amino acid sequence set forth in SEQ ID NO: 67.

In some embodiments, the monospecific antibody binding to GPRC5D or an antigen-binding portion thereof comprises: an HCDR1, an HCDR2, and an HCDR3 in a heavy chain variable region having the amino acid sequence set forth in SEQ ID NO: 66, and an LCDR1, an LCDR2, and an LCDR3 in a light chain variable region having the amino acid sequence set forth in SEQ ID NO: 67, wherein $X_3$ is selected from Q and V, $X_4$ is selected from P and A, $X_5$ is selected from L and V, $X_6$ is selected from K and A, $X_7$ is selected from I and M, $X_8$ is selected from D and E, $X_9$ is selected from G and A, $X_{10}$ is selected from K and R, $X_{11}$ is selected from A and V, $X_{12}$ is selected from K and T, $X_{13}$ is selected from S and A, $X_{14}$ is selected from T and R, $X_{15}$ is selected from S and T, and $X_{16}$ is selected from A and Q.

In some embodiments, the monospecific antibody binding to GPRC5D or an antigen-binding portion thereof comprises: an HCDR1, an HCDR2, and an HCDR3 in a heavy chain variable region having the amino acid sequence set forth in SEQ ID NO: 68, and an LCDR1, an LCDR2, and an LCDR3 in a light chain variable region having the amino acid sequence set forth in SEQ ID NO: 69.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, 15, 23, 31, 39, 62, 63, 64, 65, 66, or 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8, 16, 24, 32, 40, 67, or 69.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8. Further, in some of such embodiments, the monospecific antibody binding to GPRC5D or an antigen-binding portion thereof further comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16. Further, in some of such embodiments, the monospecific antibody binding to GPRC5D or an antigen-binding portion thereof further comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 23, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24. Further, in some of such embodiments, the monospecific antibody binding to GPRC5D or an antigen-binding portion thereof further comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 31, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32. Further, in some of such embodiments, the monospecific antibody binding to GPRC5D or an antigen-binding portion thereof further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 39, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40. Further, in some of such embodiments, the monospecific antibody binding to GPRC5D or an antigen-binding portion thereof further comprises: an HCDR1 comprising the amino acid sequence set forth in SEQ ID NO: 33, an HCDR2 comprising the amino acid sequence set forth in SEQ ID NO: 34, an HCDR3 comprising the amino acid sequence set forth in SEQ ID NO: 35, an LCDR1 comprising the amino acid sequence set forth in SEQ ID NO: 36, an LCDR2 comprising the amino acid sequence set forth in SEQ ID NO: 37, and an LCDR3 comprising the amino acid sequence set forth in SEQ ID NO: 38.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67. Further, in some of such embodiments, the monospecific antibody binding to GPRC5D or an antigen-binding portion thereof further comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67. Further, in some of such embodiments, the monospecific antibody binding to GPRC5D or an antigen-binding portion thereof further comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67. Further, in some of such embodiments, the monospecific antibody binding to GPRC5D or an antigen-binding portion thereof further comprises: an HCDR1 comprising the amino acid sequence set forth in SEQ ID NO: 1, an HCDR2 comprising the amino acid sequence set forth in SEQ ID NO: 60, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67. Further, in some of such embodiments, the monospecific antibody binding to GPRC5D or an antigen-binding portion thereof further comprises: an HCDR1 comprising the amino acid sequence set forth in SEQ ID NO: 1, an HCDR2 comprising the amino acid sequence set forth in SEQ ID NO: 61, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 66, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, wherein $X_3$ is selected from Q and V, $X_4$ is selected from P and A, $X_5$ is selected from L and V, $X_6$ is selected from K and A, $X_7$ is selected from I and M, $X_8$ is selected from D and E, $X_9$ is selected from G and A, $X_{10}$ is selected from K and R, $X_{11}$ is selected from A and V, $X_{12}$ is selected from K and T, $X_{13}$ is selected from S and A, $X_{14}$ is selected from T and R, $X_{15}$ is selected from S and T, and $X_{16}$ is selected from A and Q. Further, in some of such embodiments, the monospecific antibody binding to GPRC5D or an antigen-binding portion thereof further comprises: an HCDR1 comprising the amino acid sequence set forth in SEQ ID NO: 1, an HCDR2 comprising the amino acid sequence set forth in SEQ ID NO: 59, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, wherein $X_1$ is selected from D and E, and $X_2$ is selected from G and A.

In some embodiments, the monospecific antibody binding to GPRC5D or an antigen-binding portion thereof comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69. Further, in some of such embodiments, the monospecific antibody binding to GPRC5D or an antigen-binding portion thereof further comprises: an HCDR1 comprising the amino acid sequence set forth in SEQ ID NO: 33, an HCDR2 comprising the amino acid sequence set forth in SEQ ID NO: 34, an HCDR3 comprising the amino acid sequence set forth in SEQ ID NO: 35, an LCDR1 comprising the amino acid sequence set forth in SEQ ID NO: 36, an LCDR2 comprising the amino acid sequence set forth in SEQ ID NO: 37, and an LCDR3 comprising the amino acid sequence set forth in SEQ ID NO: 38.

In some embodiments, the monospecific antibody binding to GPRC5D or an antigen-binding portion thereof comprises: a heavy chain variable region comprising the amino acid sequence set forth in SEQ ID NO: 7 and a light chain variable region comprising the amino acid sequence set forth in SEQ ID NO: 8.

In some embodiments, the monospecific antibody binding to GPRC5D or an antigen-binding portion thereof comprises: a heavy chain variable region comprising the amino acid sequence set forth in SEQ ID NO: 15 and a light chain variable region comprising the amino acid sequence set forth in SEQ ID NO: 16.

In some embodiments, the monospecific antibody binding to GPRC5D or an antigen-binding portion thereof comprises: a heavy chain variable region comprising the amino acid sequence set forth in SEQ ID NO: 23 and a light chain variable region comprising the amino acid sequence set forth in SEQ ID NO: 24.

In some embodiments, the monospecific antibody binding to GPRC5D or an antigen-binding portion thereof comprises: a heavy chain variable region comprising the amino acid sequence set forth in SEQ ID NO: 31 and a light chain variable region comprising the amino acid sequence set forth in SEQ ID NO: 32.

In some embodiments, the monospecific antibody binding to GPRC5D or an antigen-binding portion thereof comprises: a heavy chain variable region comprising the amino acid sequence set forth in SEQ ID NO: 39 and a light chain variable region comprising the amino acid sequence set forth in SEQ ID NO: 40.

In some embodiments, the monospecific antibody binding to GPRC5D or an antigen-binding portion thereof comprises: a heavy chain variable region comprising the amino acid sequence set forth in SEQ ID NO: 62 and a light chain variable region comprising the amino acid sequence set forth in SEQ ID NO: 67.

In some embodiments, the monospecific antibody binding to GPRC5D or an antigen-binding portion thereof comprises: a heavy chain variable region comprising the amino acid sequence set forth in SEQ ID NO: 63 and a light chain variable region comprising the amino acid sequence set forth in SEQ ID NO: 67.

In some embodiments, the monospecific antibody binding to GPRC5D or an antigen-binding portion thereof comprises: a heavy chain variable region comprising the amino acid sequence set forth in SEQ ID NO: 64 and a light chain variable region comprising the amino acid sequence set forth in SEQ ID NO: 67.

In some embodiments, the monospecific antibody binding to GPRC5D or an antigen-binding portion thereof comprises: a heavy chain variable region comprising the amino acid sequence set forth in SEQ ID NO: 65 and a light chain variable region comprising the amino acid sequence set forth in SEQ ID NO: 67.

In some embodiments, the monospecific antibody binding to GPRC5D or an antigen-binding portion thereof comprises: a heavy chain variable region comprising the amino acid sequence set forth in SEQ ID NO: 66 and a light chain variable region comprising the amino acid sequence set forth in SEQ ID NO: 67.

In some embodiments, the monospecific antibody binding to GPRC5D or an antigen-binding portion thereof comprises: a heavy chain variable region comprising the amino acid sequence set forth in SEQ ID NO: 68 and a light chain variable region comprising the amino acid sequence set forth in SEQ ID NO: 69.

In some specific embodiments, the monospecific antibody binding to GPRC5D or an antigen-binding portion thereof comprises a heavy chain variable region having the amino acid sequence set forth in SEQ ID NO: 7 and a light chain variable region having the amino acid sequence set forth in SEQ ID NO: 8.

In some specific embodiments, the monospecific antibody binding to GPRC5D or an antigen-binding portion thereof comprises a heavy chain variable region having the amino acid sequence set forth in SEQ ID NO: 15 and a light chain variable region having the amino acid sequence set forth in SEQ ID NO: 16.

In some specific embodiments, the monospecific antibody binding to GPRC5D or an antigen-binding portion thereof comprises a heavy chain variable region having the amino acid sequence set forth in SEQ ID NO: 23 and a light chain variable region having the amino acid sequence set forth in SEQ ID NO: 24.

In some specific embodiments, the monospecific antibody binding to GPRC5D or an antigen-binding portion thereof comprises a heavy chain variable region having the amino acid sequence set forth in SEQ ID NO: 31 and a light chain variable region having the amino acid sequence set forth in SEQ ID NO: 32.

In some specific embodiments, the monospecific antibody binding to GPRC5D or an antigen-binding portion thereof comprises a heavy chain variable region having the amino acid sequence set forth in SEQ ID NO: 39 and a light chain variable region having the amino acid sequence set forth in SEQ ID NO: 40.

In some specific embodiments, the monospecific antibody binding to GPRC5D or an antigen-binding portion thereof comprises a heavy chain variable region having the amino acid sequence set forth in SEQ ID NO: 62 and a light chain variable region having the amino acid sequence set forth in SEQ ID NO: 67.

In some specific embodiments, the monospecific antibody binding to GPRC5D or an antigen-binding portion thereof comprises a heavy chain variable region having the amino acid sequence set forth in SEQ ID NO: 63 and a light chain variable region having the amino acid sequence set forth in SEQ ID NO: 67.

In some specific embodiments, the monospecific antibody binding to GPRC5D or an antigen-binding portion thereof comprises a heavy chain variable region having the amino acid sequence set forth in SEQ ID NO: 64 and a light chain variable region having the amino acid sequence set forth in SEQ ID NO: 67.

In some specific embodiments, the monospecific antibody binding to GPRC5D or an antigen-binding portion thereof comprises a heavy chain variable region having the amino acid sequence set forth in SEQ ID NO: 65 and a light chain variable region having the amino acid sequence set forth in SEQ ID NO: 67.

In some specific embodiments, the monospecific antibody binding to GPRC5D or an antigen-binding portion thereof comprises a heavy chain variable region having the amino acid sequence set forth in SEQ ID NO: 66 and a light chain variable region having the amino acid sequence set forth in SEQ ID NO: 67.

In some specific embodiments, the monospecific antibody binding to GPRC5D or an antigen-binding portion thereof comprises a heavy chain variable region having the amino acid sequence set forth in SEQ ID NO: 68 and a light chain variable region having the amino acid sequence set forth in SEQ ID NO: 69.

In the present disclosure, the monospecific antibody binding to GPRC5D or the antigen-binding portion thereof is murine, chimeric, or humanized. Humanization may reduce immunogenicity. Therefore, in some embodiments, the monospecific antibody binding to GPRC5D or the antigen-binding portion thereof is humanized.

In some embodiments, the monospecific antibody binding to GPRC5D of the present disclosure may further comprise a constant region of an immunoglobulin, or a fragment, analog, variant or derivative of the constant region. In some embodiments, the constant region comprises a heavy chain constant region and a light chain constant region. In some embodiments, the heavy chain constant region is derived from a human immunoglobulin heavy chain, for example, a heavy chain of IgG1, IgG2, IgG3, IgG4, or other types of immunoglobulins. In some embodiments, the light chain constant region is derived from a human immunoglobulin light chain, for example, a κ light chain or a λ light chain of a human immunoglobulin. In some embodiments, the constant region may comprise any modification disclosed herein or well-known in the art, such as an insertion, a deletion, a substitution, or a chemical modification of an amino acid. In some embodiments, the constant region comprises a mutation that alters the effector function. In some embodiments, any amino acid residue of the constant region may be substituted by an amino acid residue of any allotype. In some embodiments, the C-terminal lysine (Lys447) of the heavy chain constant region may or may not be present. In some specific embodiments, the monospecific antibody binding to GPRC5D comprises: a heavy chain comprising the amino acid sequence set forth in SEQ ID NO: 70 and a light chain comprising the amino acid sequence set forth in SEQ ID NO: 78. In some specific embodiments, the monospecific antibody binding to GPRC5D comprises: a heavy chain comprising the amino acid sequence set forth in SEQ ID NO: 72 and a light chain comprising the amino acid sequence set forth in SEQ ID NO: 78. In some specific embodiments, the monospecific antibody binding to GPRC5D comprises: a heavy chain comprising the amino acid sequence set forth in SEQ ID NO: 74 and a light chain comprising the amino acid sequence set forth in SEQ ID NO: 78. In some specific embodiments, the monospecific antibody binding to GPRC5D comprises: a heavy chain comprising the amino acid sequence set forth in SEQ ID NO: 76 and a light chain comprising the amino acid sequence set forth in SEQ ID NO: 78. In some specific embodiments, the monospecific antibody binding to GPRC5D comprises: a heavy chain comprising the amino acid sequence set forth in SEQ ID NO: 80 and a light chain comprising the amino acid sequence set forth in SEQ ID NO: 82. In some embodiments, the C-terminal lysine of the heavy chain described above is absent.

Tables S1 and S2 provide amino acid sequence information about CDRs and variable regions of some exemplary monospecific antibodies that bind to GPRC5D (e.g., chimeric antibodies Chi-89, Chi-105, Chi-128, Chi-131, and Chi-169, and humanized antibodies hu131-v1, hu131-v2, hu131-v3, hu131-v4, and hu128-v1). These exemplary antibodies can be used to construct the bispecific antibodies of the present disclosure.

Table S1. Amino acid sequences of CDRs and variable regions (SEQ ID NOs:)

| Name | HCDR1 | HCDR2 | HCDR3 | VH | LCDR1 | LCDR2 | LCDR3 | VL |
|---|---|---|---|---|---|---|---|---|
| Chi-89 | 17 | 18 | 19 | 23 | 20 | 21 | 22 | 24 |
| Chi-105 | 25 | 26 | 27 | 31 | 28 | 29 | 30 | 32 |
| Chi-128 | 33 | 34 | 35 | 39 | 36 | 37 | 38 | 40 |
| hu128-v1 | | | | 68 | | | | 69 |
| Chi-131 | 1 | 2 | 3 | 7 | 4 | 5 | 6 | 8 |
| hu131-v1 | | | | 62 | | | | 67 |
| hu131-v2 | | | | 63 | | | | |
| hu131-v3 | | 60 | | 64 | | | | |
| hu131-v4 | | 61 | | 65 | | | | |
| Chi-169 | 9 | 10 | 11 | 15 | 12 | 13 | 14 | 16 |

The monospecific antibody binding to GPRC5D or the antigen-binding portion thereof of the present disclosure can bind to human GPRC5D or/and monkey GPRC5D or/and mouse GPRC5D. In some embodiments, the monospecific antibody or the antigen-binding portion thereof does not bind or substantially does not bind to GPRC5A, GPRC5B, and GPRC5C.

*Isolated nucleic acid*

The present disclosure provides an isolated nucleic acid comprising a nucleotide sequence encoding the monospecific antibody binding to GPRC5D or the antigen-binding portion thereof described in the present disclosure. Some nucleotide sequences encoding the monospecific antibody binding to GPRC5D or the antigen-binding portion thereof are illustratively listed in the sequence listing.

*Vector*

The present disclosure provides a vector comprising the nucleic acid. In some embodiments, the vector is a cloning vector; in some other embodiments, the vector is an expression vector; in a specific embodiment, the expression vector is pcDNA3.1(+). The expression vector is optionally any expression vector capable of expressing the monospecific antibody binding to GPRC5D described herein.

*Host cell*

The present disclosure provides a host cell comprising the nucleic acid of the present disclosure or the vector of the present disclosure. In some embodiments, the host cell is a suitable host cell for cloning or expressing the monospecific antibody or the antigen-binding portion thereof. In some embodiments, the host cell is a prokaryotic cell. In some other embodiments, the host cell is a eukaryotic cell. In some embodiments, the host cell is selected from a yeast cell, a mammalian cell, and other cells suitable for preparing a monospecific antibody or an antigen-binding portion thereof. Mammalian cells are, for example, Chinese hamster ovary (CHO) cells or CHO-S cells.

*Antibody-drug conjugate (ADC)*

The present disclosure provides an antibody-drug conjugate comprising the monospecific antibody binding to GPRC5D or the antigen-binding portion thereof of the present disclosure. In some embodiments, the antibody-drug conjugate comprises the monospecific antibody binding to GPRC5D or the antigen-binding portion thereof, and a cytotoxic drug. The monospecific antibody binding to GPRC5D or the antigen-binding portion thereof and the cytotoxic drug are preferably linked by a linker, and the linker is a cleavable linker or a non-cleavable linker.

*Chimeric antigen receptor (CAR)*

The present disclosure further provides an anti-GPRC5D chimeric antigen receptor (CAR) comprising the monospecific antibody binding to GPRC5D or the antigen-binding portion thereof of the present disclosure. The anti-GPRC5D CAR may comprise: (a) an extracellular antigen-binding domain that binds to GPRC5D; (b) a transmembrane domain; and (c) an intracellular signaling domain.

The present disclosure further provides an engineered immune effector cell, which comprises the CAR of the present disclosure. In some embodiments, the immune effector cell is a T cell, an NK cell, a peripheral blood mononuclear cell (PBMC), a hematopoietic stem cell, a pluripotent stem cell, or an embryonic stem cell.

*Pharmaceutical composition*

The present disclosure provides a pharmaceutical composition comprising the monospecific antibody binding to GPRC5D or the antigen-binding portion thereof, the antibody-drug conjugate, or the engineered immune effector cell of the present disclosure, and one or more pharmaceutically acceptable excipients. The pharmaceutically acceptable excipient includes, for example, an excipient, a diluent, an encapsulating material, a filler, a buffering agent, or other reagents.

*Method for preparing monospecific antibody or antigen-binding portion thereof*

In some embodiments, the present disclosure provides a method for preparing a monospecific antibody binding to GPRC5D or an antigen-binding portion thereof, which comprises: culturing the host cell to express the monospecific antibody or the antigen-binding portion thereof, and isolating and purifying the monospecific antibody or the antigen-binding portion thereof in a system. To produce the monospecific antibody or the antigen-binding portion thereof, a nucleic acid encoding the monospecific antibody or the antigen-binding portion thereof is isolated and inserted into one or more vectors for further cloning or/and expression in a host cell. The nucleic acid can be obtained using various methods known in the art, such as gene splicing and chemical synthesis.

The purity of the monospecific antibody or the antigen-binding portion thereof can be determined by any of a variety of well-known analytical methods, such as gel electrophoresis, high performance liquid chromatography, and size exclusion chromatography.

The physicochemical properties or/and biological activity of the monospecific antibody or the antigen-binding portion thereof of the present disclosure can be identified, screened, or characterized by a variety of assay methods known in the art.

*Use*

The present disclosure provides use of the monospecific antibody binding to GPRC5D or the antigen-binding portion thereof of the present disclosure. In some specific embodiments, the monospecific antibody used may be Chi-89, Chi-105, Chi-128, Chi-131, Chi-169, hu131-v1, hu131-v2, hu131-v3, hu131-v4, or/and hu128-v1. The present disclosure provides use of the monospecific antibody or the antigen-binding portion thereof, the antibody-drug conjugate, the engineered immune effector cell, or the pharmaceutical composition for preparing a medicament for treating a GPRC5D-expressing disease. In some embodiments, the monospecific antibody or the antigen-binding portion thereof, the antibody-drug conjugate, the engineered immune effector cell, or the pharmaceutical composition is used in combination with one or more additional therapeutic agents to prepare the medicament. The additional therapeutic agent may be a therapeutic agent for a tumor or a therapeutic agent for an autoimmune disease known in the art.

The present disclosure provides a method for treating a GPRC5D-expressing disease comprising administering to a subject in need the monospecific antibody or the antigen-binding portion thereof, the antibody-drug conjugate, the engineered immune effector cell, or the pharmaceutical composition. The present disclosure provides a method for treating a GPRC5D-expressing disease comprising administering to a subject in need a therapeutically effective amount of the monospecific antibody or the antigen-binding portion thereof, the antibody-drug conjugate, the engineered immune effector cell, or the pharmaceutical composition. In some embodiments, the disease is a tumor or an autoimmune disease.

In some embodiments, the tumor is a non-solid tumor. In some embodiments, the tumor is a hematological tumor. In some embodiments, the tumor is B-cell lymphoma. In some embodiments, the tumor is a B-cell lymphoma expressing GPRC5D. In some embodiments, the tumor is multiple myeloma (MM). In some embodiments, the tumor is multiple myeloma expressing GPRC5D.

In some embodiments, the autoimmune disease is, for example, systemic lupus erythematosus and/or rheumatoid arthritis.

*III. Bispecific antibody*

The present disclosure provides a bispecific antibody comprising a first antigen-binding portion binding to GPRC5D and a second antigen-binding portion binding to CD3, wherein the first antigen-binding portion comprises: an HCDR1 comprising the amino acid sequence set forth in SEQ ID NO: 1, 9, 17, 25, or 33, an HCDR2 comprising the amino acid sequence set forth in SEQ ID NO: 59, 10, 18, 26, or 34, an HCDR3 comprising the amino acid sequence set forth in SEQ ID NO: 3, 11, 19, 27, or 35, an LCDR1 comprising the amino acid sequence set forth in SEQ ID NO: 4, 12, 20, 28, or 36, an LCDR2 comprising the amino acid sequence set forth in SEQ ID NO: 5, 13, 21, 29, or 37, and an LCDR3 comprising the amino acid sequence set forth in SEQ ID NO: 6, 14, 22, 30, or 38.

In some embodiments, the first antigen-binding portion comprises: an HCDR1 comprising the amino acid sequence set forth in SEQ ID NO: 1, an HCDR2 comprising the amino acid sequence set forth in SEQ ID NO: 59, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, wherein $X_1$ is selected from D and E, and $X_2$ is selected from G and A. In some specific embodiments, $X_1$ is D, and $X_2$ is G. In some specific embodiments, $X_1$ is D, and $X_2$ is A. In some specific embodiments, $X_1$ is E, and $X_2$ is G.

In some embodiments, the first antigen-binding portion comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the first antigen-binding portion comprises: an HCDR1 comprising the amino acid sequence set forth in SEQ ID NO: 1, an HCDR2 comprising the amino acid sequence set forth in SEQ ID NO: 60, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the first antigen-binding portion comprises: an HCDR1 comprising the amino acid sequence set forth in SEQ ID NO: 1, an HCDR2 comprising the amino acid sequence set forth in SEQ ID NO: 61, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the first antigen-binding portion comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the first antigen-binding portion comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the first antigen-binding portion comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the first antigen-binding portion comprises: an HCDR1 comprising the amino acid sequence set forth in SEQ ID NO: 33, an HCDR2 comprising the amino acid sequence set forth in SEQ ID NO: 34, an HCDR3 comprising the amino acid sequence set forth in SEQ ID NO: 35, an LCDR1 comprising the amino acid sequence set forth in SEQ ID NO: 36, an LCDR2 comprising the amino acid sequence set forth in SEQ ID NO: 37, and an LCDR3 comprising the amino acid sequence set forth in SEQ ID NO: 38.

The present disclosure further provides a bispecific antibody, comprising a first antigen-binding portion binding to GPRC5D and a second antigen-binding portion binding to CD3, wherein the first antigen-binding portion comprises: an HCDR1, an HCDR2, and an HCDR3 in a heavy chain variable region having the amino acid sequence set forth in SEQ ID NO: 7, 15, 23, 31, 39, 66, or 68, and an LCDR1, an LCDR2, and an LCDR3 in a light chain variable region having the amino acid sequence set forth in SEQ ID NO: 8, 16, 24, 32, 40, 67, or 69.

In some embodiments, the first antigen-binding portion comprises: an HCDR1, an HCDR2, and an HCDR3 in a heavy chain variable region having the amino acid sequence set forth in SEQ ID NO: 7, and an LCDR1, an LCDR2, and an LCDR3 in a light chain variable region having the amino acid sequence set forth in SEQ ID NO: 8.

In some embodiments, the first antigen-binding portion comprises: an HCDR1, an HCDR2, and an HCDR3 in a heavy chain variable region having the amino acid sequence set forth in SEQ ID NO: 15, and an LCDR1, an LCDR2, and an LCDR3 in a light chain variable region having the amino acid sequence set forth in SEQ ID NO: 16.

In some embodiments, the first antigen-binding portion comprises: an HCDR1, an HCDR2, and an HCDR3 in a heavy chain variable region having the amino acid sequence set forth in SEQ ID NO: 23, and an LCDR1, an LCDR2, and an LCDR3 in a light chain variable region having the amino acid sequence set forth in SEQ ID NO: 24.

In some embodiments, the first antigen-binding portion comprises: an HCDR1, an HCDR2, and an HCDR3 in a heavy chain variable region having the amino acid sequence set forth in SEQ ID NO: 31, and an LCDR1, an LCDR2, and an LCDR3 in a light chain variable region having the amino acid sequence set forth in SEQ ID NO: 32.

In some embodiments, the first antigen-binding portion comprises: an HCDR1, an HCDR2, and an HCDR3 in a heavy chain variable region having the amino acid sequence set forth in SEQ ID NO: 39, and an LCDR1, an LCDR2, and an LCDR3 in a light chain variable region having the amino acid sequence set forth in SEQ ID NO: 40.

In some embodiments, the first antigen-binding portion comprises: an HCDR1, an HCDR2, and an HCDR3 in a heavy chain variable region having the amino acid sequence set forth in SEQ ID NO: 62, and an LCDR1, an LCDR2, and an LCDR3 in a light chain variable region having the amino acid sequence set forth in SEQ ID NO: 67.

In some embodiments, the first antigen-binding portion comprises: an HCDR1, an HCDR2, and an HCDR3 in a heavy chain variable region having the amino acid sequence set forth in SEQ ID NO: 63, and an LCDR1, an LCDR2, and an LCDR3 in a light chain variable region having the amino acid sequence set forth in SEQ ID NO: 67.

In some embodiments, the first antigen-binding portion comprises: an HCDR1, an HCDR2, and an HCDR3 in a heavy chain variable region having the amino acid sequence set forth in SEQ ID NO: 64, and an LCDR1, an LCDR2, and an LCDR3 in a light chain variable region having the amino acid sequence set forth in SEQ ID NO: 67.

In some embodiments, the first antigen-binding portion comprises: an HCDR1, an HCDR2, and an HCDR3 in a heavy chain variable region having the amino acid sequence set forth in SEQ ID NO: 65, and an LCDR1, an LCDR2, and an LCDR3 in a light chain variable region having the amino acid sequence set forth in SEQ ID NO: 67.

In some embodiments, the first antigen-binding portion comprises: an HCDR1, an HCDR2, and an HCDR3 in a heavy chain variable region having the amino acid sequence set forth in SEQ ID NO: 66, and an LCDR1, an LCDR2, and an LCDR3 in a light chain variable region having the amino acid sequence set forth in SEQ ID NO: 67, wherein $X_3$ is selected from Q and V, $X_4$ is selected from P and A, $X_5$ is selected from L and V, $X_6$ is selected from K and A, $X_7$ is selected from I and M, $X_8$ is selected from D and E, $X_9$ is selected from G and A, $X_{10}$ is selected from K and R, $X_{11}$ is selected from A and V, $X_{12}$ is selected from K and T, $X_{13}$ is selected from S and A, $X_{14}$ is selected from T and R, $X_{15}$ is selected from S and T, and $X_{16}$ is selected from A and Q.

In some embodiments, the first antigen-binding portion comprises: an HCDR1, an HCDR2, and an HCDR3 in a heavy chain variable region having the amino acid sequence set forth in SEQ ID NO: 68, and an LCDR1, an LCDR2, and an LCDR3 in a light chain variable region having the amino acid sequence set forth in SEQ ID NO: 69.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, 15, 23, 31, 39, 62, 63, 64, 65, 66, or 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8, 16, 24, 32, 40, 67, or 69.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8. Further, in some of such embodiments, the first antigen-binding portion further comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16. Further, in some of such embodiments, the first antigen-binding portion further comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 23, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24. Further, in some of such embodiments, the first antigen-binding portion further comprises: an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 31, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32. Further, in some of such embodiments, the first antigen-binding portion further comprises: an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 39, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40. Further, in some of such embodiments, the first antigen-binding portion further comprises: an HCDR1 comprising the amino acid sequence set forth in SEQ ID NO: 33, an HCDR2 comprising the amino acid sequence set forth in SEQ ID NO: 34, an HCDR3 comprising the amino acid sequence set forth in SEQ ID NO: 35, an LCDR1 comprising the amino acid sequence set forth in SEQ ID NO: 36, an LCDR2 comprising the amino acid sequence set forth in SEQ ID NO: 37, and an LCDR3 comprising the amino acid sequence set forth in SEQ ID NO: 38.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67. Further, in some of such embodiments, the first antigen-binding portion further comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67. Further, in some of such embodiments, the first antigen-binding portion further comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67. Further, in some of such embodiments, the first antigen-binding portion further comprises: an HCDR1 comprising the amino acid sequence set forth in SEQ ID NO: 1, an HCDR2 comprising the amino acid sequence set forth in SEQ ID NO: 60, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67. Further, in some of such embodiments, the first antigen-binding portion further comprises: an HCDR1 comprising the amino acid sequence set forth in SEQ ID NO: 1, an HCDR2 comprising the amino acid sequence set forth in SEQ ID NO: 61, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 66, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, wherein $X_3$ is selected from Q and V, $X_4$ is selected from P and A, $X_5$ is selected from L and V, $X_6$ is selected from K and A, $X_7$ is selected from I and M, $X_8$ is selected from D and E, $X_9$ is selected from G and A, $X_{10}$ is selected from K and R, $X_{11}$ is selected from A and V, $X_{12}$ is selected from K and T, $X_{13}$ is selected from S and A, $X_{14}$ is selected from T and R, $X_{15}$ is selected from S and T, and $X_{16}$ is selected from A and Q. Further, in some of such embodiments, the first antigen-binding portion further comprises: an HCDR1 comprising the amino acid sequence set forth in SEQ ID NO: 1, an HCDR2 comprising the amino acid sequence set forth in SEQ ID NO: 59, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, wherein $X_1$ is selected from D and E, and $X_2$ is selected from G and A.

In some embodiments, the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69. Further, in some of such embodiments, the first antigen-binding portion further comprises: an HCDR1 comprising the amino acid sequence set forth in SEQ ID NO: 33, an HCDR2 comprising the amino acid sequence set forth in SEQ ID NO: 34, an HCDR3 comprising the amino acid sequence set forth in SEQ ID NO: 35, an LCDR1 comprising the amino acid sequence set forth in SEQ ID NO: 36, an LCDR2 comprising the amino acid sequence set forth in SEQ ID NO: 37, and an LCDR3 comprising the amino acid sequence set forth in SEQ ID NO: 38.

In some embodiments, the first antigen-binding portion comprises: a heavy chain variable region comprising the amino acid sequence set forth in SEQ ID NO: 7 and a light chain variable region comprising the amino acid sequence set forth in SEQ ID NO: 8.

In some embodiments, the first antigen-binding portion comprises: a heavy chain variable region comprising the amino acid sequence set forth in SEQ ID NO: 15 and a light chain variable region comprising the amino acid sequence set forth in SEQ ID NO: 16.

In some embodiments, the first antigen-binding portion comprises: a heavy chain variable region comprising the amino acid sequence set forth in SEQ ID NO: 23 and a light chain variable region comprising the amino acid sequence set forth in SEQ ID NO: 24.

In some embodiments, the first antigen-binding portion comprises: a heavy chain variable region comprising the amino acid sequence set forth in SEQ ID NO: 31 and a light chain variable region comprising the amino acid sequence set forth in SEQ ID NO: 32.

In some embodiments, the first antigen-binding portion comprises: a heavy chain variable region comprising the amino acid sequence set forth in SEQ ID NO: 39 and a light chain variable region comprising the amino acid sequence set forth in SEQ ID NO: 40.

In some embodiments, the first antigen-binding portion comprises: a heavy chain variable region comprising the amino acid sequence set forth in SEQ ID NO: 62 and a light chain variable region comprising the amino acid sequence set forth in SEQ ID NO: 67.

In some embodiments, the first antigen-binding portion comprises: a heavy chain variable region comprising the amino acid sequence set forth in SEQ ID NO: 63 and a light chain variable region comprising the amino acid sequence set forth in SEQ ID NO: 67.

In some embodiments, the first antigen-binding portion comprises: a heavy chain variable region comprising the amino acid sequence set forth in SEQ ID NO: 64 and a light chain variable region comprising the amino acid sequence set forth in SEQ ID NO: 67.

In some embodiments, the first antigen-binding portion comprises: a heavy chain variable region comprising the amino acid sequence set forth in SEQ ID NO: 65 and a light chain variable region comprising the amino acid sequence set forth in SEQ ID NO: 67.

In some embodiments, the first antigen-binding portion comprises: a heavy chain variable region comprising the amino acid sequence set forth in SEQ ID NO: 66 and a light chain variable region comprising the amino acid sequence set forth in SEQ ID NO: 67.

In some embodiments, the first antigen-binding portion comprises: a heavy chain variable region comprising the amino acid sequence set forth in SEQ ID NO: 68 and a light chain variable region comprising the amino acid sequence set forth in SEQ ID NO: 69.

In some specific embodiments, the first antigen-binding portion comprises a heavy chain variable region having the amino acid sequence set forth in SEQ ID NO: 7 and a light chain variable region having the amino acid sequence set forth in SEQ ID NO: 8.

In some specific embodiments, the first antigen-binding portion comprises a heavy chain variable region having the amino acid sequence set forth in SEQ ID NO: 15 and a light chain variable region having the amino acid sequence set forth in SEQ ID NO: 16.

In some specific embodiments, the first antigen-binding portion comprises a heavy chain variable region having the amino acid sequence set forth in SEQ ID NO: 23 and a light chain variable region having the amino acid sequence set forth in SEQ ID NO: 24.

In some specific embodiments, the first antigen-binding portion comprises a heavy chain variable region having the amino acid sequence set forth in SEQ ID NO: 31 and a light chain variable region having the amino acid sequence set forth in SEQ ID NO: 32.

In some specific embodiments, the first antigen-binding portion comprises a heavy chain variable region having the amino acid sequence set forth in SEQ ID NO: 39 and a light chain variable region having the amino acid sequence set forth in SEQ ID NO: 40.

In some specific embodiments, the first antigen-binding portion comprises a heavy chain variable region having the amino acid sequence set forth in SEQ ID NO: 62 and a light chain variable region having the amino acid sequence set forth in SEQ ID NO: 67.

In some specific embodiments, the first antigen-binding portion comprises a heavy chain variable region having the amino acid sequence set forth in SEQ ID NO: 63 and a light chain variable region having the amino acid sequence set forth in SEQ ID NO: 67.

In some specific embodiments, the first antigen-binding portion comprises a heavy chain variable region having the amino acid sequence set forth in SEQ ID NO: 64 and a light chain variable region having the amino acid sequence set forth in SEQ ID NO: 67.

In some specific embodiments, the first antigen-binding portion comprises a heavy chain variable region having the amino acid sequence set forth in SEQ ID NO: 65 and a light chain variable region having the amino acid sequence set forth in SEQ ID NO: 67.

In some specific embodiments, the first antigen-binding portion comprises a heavy chain variable region having the amino acid sequence set forth in SEQ ID NO: 66 and a light chain variable region having the amino acid sequence set forth in SEQ ID NO: 67.

In some specific embodiments, the first antigen-binding portion comprises a heavy chain variable region having the amino acid sequence set forth in SEQ ID NO: 68 and a light chain variable region having the amino acid sequence set forth in SEQ ID NO: 69.

In the present disclosure, the first antigen-binding portion is murine, chimeric, or humanized. Humanization may reduce immunogenicity. Thus, in some embodiments, the first antigen-binding portion is humanized.

The exemplary monospecific antibodies provided in Tables S1 and S2 can be used to construct the bispecific antibodies of the present disclosure. For example, in some embodiments, the CDRs of the first antigen-binding portion of the bispecific antibody are selected from six CDRs of any one of the exemplary monospecific antibodies that bind to GPRC5D. For example, in some embodiments, the variable regions of the first antigen-binding portion of the bispecific antibody are selected from a heavy chain variable region and a light chain variable region of any one of the exemplary monospecific antibodies that bind to GPRC5D.

In the bispecific antibody of the present disclosure, the second antigen-binding portion provides the ability to target CD3. The second antigen-binding portion may be a Fab, an scFv, or an scFab (single-chain Fab). In some embodiments, the second antigen-binding portion is an scFv binding to CD3.

In some embodiments, the second antigen-binding portion is murine, chimeric, or humanized. In some embodiments, the second antigen-binding portion is humanized. In some specific embodiments, both the first antigen-binding portion and the second antigen-binding portion are humanized.

In some embodiments, the second antigen-binding portion comprises: an HCDR1 comprising the amino acid sequence set forth in SEQ ID NO: 94, an HCDR2 comprising the amino acid sequence set forth in SEQ ID NO: 95, an HCDR3 comprising the amino acid sequence set forth in SEQ ID NO: 96, an LCDR1 comprising the amino acid sequence set forth in SEQ ID NO: 97, an LCDR2 comprising the amino acid sequence set forth in SEQ ID NO: 98, and an LCDR3 comprising the amino acid sequence set forth in SEQ ID NO: 99.

In some embodiments, the second antigen-binding portion comprises: an HCDR1, an HCDR2, and an HCDR3 in a heavy chain variable region having the amino acid sequence set forth in SEQ ID NO: 100, and an LCDR1, an LCDR2, and an LCDR3 in a light chain variable region having the amino acid sequence set forth in SEQ ID NO: 101.

In some embodiments, the second antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 100, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 101. In some embodiments, the second antigen-binding portion comprises: a heavy chain variable region comprising the amino acid sequence set forth in SEQ ID NO: 100 and a light chain variable region comprising the amino acid sequence set forth in SEQ ID NO: 101. In some embodiments, the second antigen-binding portion comprises a heavy chain variable region having the amino acid sequence set forth in SEQ ID NO: 100 and a light chain variable region having the amino acid sequence set forth in SEQ ID NO: 101.

The bispecific antibody of the present disclosure can bind to GPRC5D. In some embodiments, the bispecific antibody can bind to human GPRC5D or/and monkey GPRC5D or/and mouse GPRC5D. In some embodiments, the bispecific antibody does not bind or substantially does not bind to GPRC5A, GPRC5B, and GPRC5C.

In some specific examples, the bispecific antibody of the present disclosure may be GC35 or GC39.

The bispecific antibody of the present disclosure can target GPRC5D expressed on the surface of tumor cells, and can also bind to T cells, induce the activation of T cells, and activate the tumor killing activity of the T cells. The bispecific antibody of the present disclosure exhibits excellent anti-tumor properties such as lysing tumor cells or inhibiting tumor cell proliferation. In some embodiments, the bispecific antibody has excellent anti-tumor performance against a tumor such as multiple myeloma.

The molecular design of the bispecific antibody of the present disclosure is easy to express and purify.

The bispecific antibody of the present disclosure has good safety.

*Bispecific antibody configurations*

The bispecific antibody of the present disclosure may have no Fc domain, and the first antigen-binding portion and the second antigen-binding portion are fused via a suitable linker.

The bispecific antibody disclosed herein may have an Fc domain, and the Fc domain may prolong the half-life, provide the Fc domain-related effector, and the like. In some embodiments, the first antigen-binding portion or/and the second antigen-binding portion of the bispecific antibody of the present disclosure can be fused to the N-terminus of the Fc domain.

In the bispecific antibody of the present disclosure, the first antigen-binding portion and the second antigen-binding portion may take any suitable structural form. In some embodiments, the first antigen-binding portion and the second antigen-binding portion may each independently be a Fab, scFv, scFab, or other structures.

The bispecific antibody of the present disclosure may be polyvalent, e.g., bivalent or tetravalent. In some embodiments, the bispecific antibody is bivalent, i.e., the first antigen-binding portion and the second antigen-binding portion each provide monovalent binding to the corresponding antigen.

In some embodiments, the first antigen-binding portion is a Fab and the second antigen-binding portion is an scFv.

In some embodiments, the bispecific antibody of the present disclosure further comprises an Fc domain composed of two Fc polypeptides.

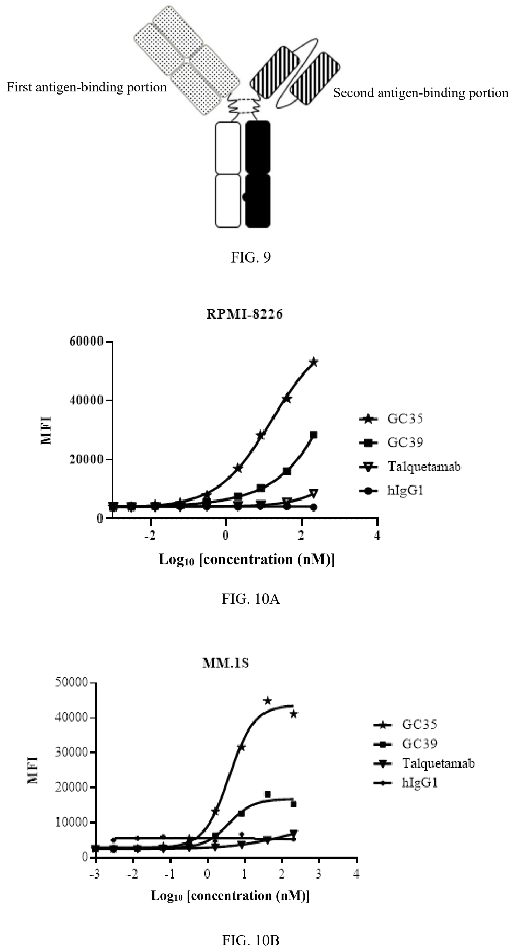

In some embodiments, the first antigen-binding portion is a Fab, and the second antigen-binding portion is an scFv; the first antigen-binding portion, at the C-terminus of its Fab heavy chain, is fused to the N-terminus of one of the Fc polypeptides of the Fc domain, and the second antigen-binding portion, at the C-terminus, is fused to the N-terminus of the other Fc polypeptide of the Fc domain. Such configurations are schematically depicted in FIG. 9. For the embodiment, in further more specific embodiments, the bispecific antibody consists of three polypeptide chains, wherein the first polypeptide chain comprises a Fab light chain of the first antigen-binding portion described above, the second polypeptide chain comprises a Fab heavy chain of the first antigen-binding portion described above and one of the Fc polypeptides of the Fc domain, and the third polypeptide chain comprises the second antigen-binding portion described above and the other Fc polypeptide of the Fc domain.

In the bispecific antibody of the present disclosure, the heavy chain variable region and the light chain variable region of the second antigen-binding portion are linked by a peptide linker to form an scFv. The peptide linker may be any suitable, e.g., electrically charged and/or flexible, linker.

In some embodiments, the second antigen-binding portion is an scFv and has a structure of VH-VL from N-terminus to C-terminus. In some embodiments, the second antigen-binding portion is an scFv and has a structure of VL-VH from N-terminus to C-terminus. Optionally, the heavy chain variable region of the second antigen-binding portion is fused to the light chain variable region of the second antigen-binding portion via a peptide linker.

In some specific embodiments, the peptide linker consists of 1 to 50 amino acids linked by peptide bonds, wherein the amino acids may be selected from 20 naturally occurring amino acids. In some more preferred embodiments, the 1 to 50 amino acids are selected from glycine, alanine, proline, serine, asparagine, glutamine, and lysine. Thus, exemplary peptide linkers may be polyglycines (particularly $(Gly)_4$ and $(Gly)_5$), poly(Gly-Ser), $(Gly)_3AsnGlySer(Gly)_2$, $(Gly)_3Cys(Gly)_4$, GlyProAsnGlyGly, or those disclosed in Table 4 of patent application WO2019195535, etc.

In some embodiments, the peptide linker may be a peptide linker consisting of glycine and serine. In some embodiments, the peptide linker may comprise 0, 1, 2, 3, 4, 5, 6, 7, 8, 9, 10, 11, 12, 13, 14, 15, 16, 17, 18, 19, 20 or more amino acids. In some embodiments, the peptide linker is a peptide linker containing GGGGS as a unit. In some embodiments, the peptide linker comprising GGGGS as a unit is $(GGGGS)_n$, wherein n is any number between 1 and 10, i.e., 1, 2, 3, 4, 5, 6, 7, 8, 9, and 10, or any range defined by any two of the aforementioned numbers, e.g., 1-5, 2-5, 3-6, 2-4, and 1-4. In some specific embodiments, the peptide linker is a linker polypeptide comprising GGGGS (SEQ ID NO: 104), $(GGGGS)_2$, $(GGGGS)_3$, or $(GGGGS)_4$. In some specific embodiments, the heavy chain variable region of the second antigen-binding portion is fused to the light chain variable region of the second antigen-binding portion via a peptide linker $(GGGGS)_3$.

In some specific embodiments, the first antigen-binding portion is a Fab, the second antigen-binding portion is an scFv, the first antigen-binding portion, at the C-terminus of its Fab heavy chain, is fused to the N-terminus of one of the Fc polypeptides of the Fc domain, the heavy chain variable region of the second antigen-binding portion, at the C-terminus, is fused to the N-terminus of the other Fc polypeptide of the Fc domain, and the light chain variable region of the second antigen-binding portion, at the C-terminus, is fused to the heavy chain variable region of the second antigen-binding portion; preferably, the heavy chain variable region of the second antigen-binding portion is fused to the light chain variable region of the second antigen-binding portion via a peptide linker $(GGGGS)_3$.

When the antigen-binding portion is fused to an Fc, it is typically fused via a hinge region. In one embodiment, the first antigen-binding portion is fused to one of the Fc polypeptides of the Fc domain via a first hinge, and the second antigen-binding portion is fused to another Fc polypeptide of the Fc domain via a second hinge. In some embodiments, the first hinge and the second hinge can form a covalent bond, such as a disulfide bond. The first hinge or/and the second hinge may comprise amino acids of the hinge region of human IgG, and the hinge region of human IgG comprises a native hinge region or a variant thereof. In some embodiments, the first hinge or/and the second hinge comprises/comprise amino acids of the hinge region of human IgG1. In some embodiments, the first hinge or/and the second hinge comprises/comprise amino acids of the hinge region of human IgG4. In some specific embodiments, the first hinge comprises EPKSCDKTHTCPPCP (SEQ ID NO: 102) and the second hinge comprises GEPKSSDKTHTCPPCP (SEQ ID NO: 103).

In some other embodiments, the first antigen-binding portion is a Fab, and the second antigen-binding portion is an scFv; the first antigen-binding portion, at the C-terminus of its Fab light chain, is fused to the N-terminus of one of the Fc polypeptides of the Fc domain, and the second antigen-binding portion, at the C-terminus, is fused to the N-terminus of the other Fc polypeptide of the Fc domain.

*Fc domain*

An Fc domain of a bispecific antibody consists of a pair of polypeptide chains comprising a heavy chain domain of an immunoglobulin molecule. For example, the Fc domain of immunoglobulin G (IgG) molecule is a dimer, and each of Fc polypeptides comprises CH2 and CH3 of the IgG heavy chain constant regions. The two Fc polypeptides of the Fc domain are capable of stable association with each other. In some embodiments, the bispecific antibody of the present disclosure comprises one Fc domain.

In some embodiments, the Fc domain of the bispecific antibody is an IgG Fc domain. In some embodiments, the Fc domain is an IgG1 Fc domain. In some embodiments, the Fc domain is a human IgG Fc domain. In some specific embodiments, the Fc domain is a human IgG1 Fc domain.

In some embodiments, the Fc domain comprises a modification, such as an amino acid substitution. The modification may be, for example, a modification that promotes heterodimerization, a modification that alters effector function, a modification that alters the binding ability to protein A, or the like.

In some embodiments, the Fc comprises a modification that promotes heterodimerization.

The bispecific antibody of the present disclosure comprises different antigen-binding portions fused to one or the other of the two Fc polypeptides in the Fc domain, thus, the two Fc polypeptides are typically comprised in two different polypeptide chains. Several possible combinations of the two polypeptides are generated by recombinant co-expression and subsequent dimerization of these polypeptides. In order to increase the yield and purity of the bispecific antibody in recombinant production, it would be advantageous to introduce a modification that promotes the binding of the desired polypeptides into the Fc domain of the bispecific antibody. Thus, in a specific embodiment, the Fc domain comprises an amino acid substitution that promotes association of the two Fc polypeptides of the Fc domain.

The site for the most extensive protein-protein interaction between the two Fc polypeptides of the human IgG Fc domain is in the CH3 domain of the Fc domain. Thus, in one embodiment, the modification is in the CH3 domain of the Fc domain.

In a specific embodiment, the modification is a so-called "knob-into-hole" modification, which comprises a "knob" modification in one of the two Fc polypeptides of the Fc domain and a "hole" modification in the other one of the two Fc polypeptides of the Fc domain. Generally, the method involves introducing a protuberance ("knob") at the interface of one Fc polypeptide and a corresponding cavity ("hole") at the interface of the other Fc polypeptide, such that the protuberance can be positioned in the cavity to promote heterodimer formation and to interfere with homodimer formation. The protuberance is constructed by substituting a small amino acid side chain from the interface of one Fc polypeptide with a larger side chain (e.g., tyrosine or tryptophan, etc.). The complementary cavity having the same or similar size as the protuberance is created in the interface of the other Fc polypeptide by substituting a large amino acid side chain with a smaller amino acid side chain (e.g., alanine or threonine, etc.).

Thus, in a specific embodiment, an amino acid residue is substituted with an amino acid residue having a larger side chain volume in the CH3 domain of one Fc polypeptide of the bispecific antibody, thereby creating a protuberance within the CH3 domain of the Fc polypeptide that can be positioned in a cavity within the CH3 domain of the other Fc polypeptide, and an amino acid residue is substituted with an amino acid residue having a smaller side chain volume in the CH3 domain of the other Fc polypeptide, thereby creating a cavity within the CH3 domain of the Fc polypeptide.

In some embodiments, according to the EU numbering, one Fc polypeptide of the Fc domain comprises amino acid substitution(s) 354C or/and 366Y/W, and the other Fc polypeptide comprises amino acid substitution(s) 349C, 366S, 368A, or/and 407T/V. In some specific embodiments, according to the EU numbering, one of the Fc polypeptides of the Fc domain comprises amino acid substitutions 354C and 366Y/W, and the other Fc polypeptide comprises amino acid substitutions 349C, 366S, 368A, and 407T/V. In some more specific embodiments, according to the EU numbering, one of the Fc polypeptides of the Fc domain comprises amino acid substitutions 354C and 366W, and the other Fc polypeptide comprises amino acid substitutions 349C, 366S, 368A, and 407V. In the embodiments described above, the Fc may be human IgG1 Fc. In a specific embodiment, according to the EU numbering, one of the Fc polypeptides of the Fc domain comprises amino acid substitutions S354C and T366W, and the other Fc polypeptide comprises amino acid substitutions Y349C, T366S, L368A, and Y407Y.

In some embodiments, the Fc domain comprises a modification that alters effector function. In some specific embodiments, the Fc domain of the bispecific antibody is modified to reduce the binding affinity of the Fc domain to an Fc receptor and/or reduce effector function as compared to an Fc not subjected to the modification. Reducing the binding affinity of the Fc domain to an Fc receptor and/or reducing effector function is beneficial to improve cytokine release and reduce side effects.

In some embodiments, the modification that reduces the binding affinity of the Fc domain to an Fc receptor and/or reduces effector function is an amino acid substitution. In some embodiments, the Fc domain comprises an amino acid substitution at one or more positions selected from 233, 234, 235, 297, 331, and 329. In some embodiments, the Fc domain comprises an amino acid substitution at one or more positions selected from 234, 235, and 329. In some embodiments, the Fc domain comprises amino acid substitutions 234A and 235A. In one such embodiment, the Fc is IgG1 Fc, particularly human IgG1 Fc. In some embodiments, the Fc domain comprises an amino acid substitution at position 329. In a more specific embodiment, the amino acid substitution is 329A, 329R, or 329G. In some embodiments, the Fc domain comprises an amino acid substitution at position 329 and an additional amino acid substitution at a position selected from 233, 234, 235, 297, and 331. In a more specific embodiment, the additional amino acid substitution is 233P, 234A, 235A, 235E, 297A, 297D, or/and 331S. In some embodiments, the Fc domain comprises amino acid substitutions at positions 329, 234, and 235. In a more specific embodiment, the Fc domain comprises amino acid substitutions 234A, 235A, and 329G. In another more specific embodiment, the Fc domain comprises amino acid substitutions 234A, 235A, and 329A. In one such embodiment, the Fc is IgG1 Fc, particularly human IgG1 Fc. In a specific embodiment, the Fc domain comprises amino acid substitutions L234A, L235A, and P329G. In another specific embodiment, the Fc domain comprises amino acid substitutions L234A, L235A, and P329A. In some specific embodiments, the Fc domain is a human IgG1 Fc domain comprising amino acid substitutions L234A, L235A, and P329A/G/R. Further, the Fc domain is a human IgG1 Fc domain comprising amino acid substitutions L234A, L235A, and P329A.

The amino acid substitution that reduces the binding affinity of the Fc domain to an Fc receptor and/or reduces effector function described above occurs on two polypeptide chains of the Fc domain.

In some embodiments, the Fc domain comprises a modification that reduces or eliminates the binding of a CH3 region of one Fc polypeptide in the Fc domain to Protein A (from Staphylococcus aureus). In some embodiments, the Fc domain comprises an amino acid substitution that reduces or eliminates the binding of a CH3 region of one Fc polypeptide in the Fc domain to Protein A. In some embodiments, according to the EU numbering, the Fc domain comprises an amino acid substitution (a) 435R or (b) 435R and 436F, which occurs only in one Fc polypeptide rather than in the other Fc polypeptide. In some embodiments, according to the EU numbering, the Fc domain comprises an amino acid substitution (a) 435R or (b) 435R and 436F, which occurs only in one of the Fc polypeptides. In some specific embodiments, according to the EU numbering, the Fc domain comprises amino acid substitutions H435R and Y436F, which occur only in one of the Fc polypeptides. In some specific embodiments, according to the EU numbering, the Fc domain comprises an amino acid substitution H435R, which occurs only in one of the Fc polypeptides. In the embodiments described above, the Fc is IgG1 Fc, particularly human IgG1 Fc.

In the bispecific antibody disclosed herein, the Fc domain may comprise one, two, or three of a modification that promotes heterodimerization, a modification that alters effector function, and a modification that reduces or eliminates the binding of the CH3 region of one Fc polypeptide in the Fc domain to Protein A. In some embodiments, the Fc comprises a modification that promotes heterodimerization, a modification that alters effector function, and a modification that reduces or eliminates the binding of the CH3 region of one Fc polypeptide in the Fc domain to Protein A. Combinations of the embodiments described above of the different modification types are possible. For example, in a specific embodiment, according to the EU numbering, the Fc domain comprises the following groups of amino acid substitutions:

    i. 349C, 366S, 368A, 407T/V, 354C, and 366Y/W, wherein amino acid substitutions 354C and 366Y/W are on the same Fc polypeptide, and are not on the same Fc polypeptide with other amino acid substitutions in (i);
    ii. 234A, 235A and 329A; and
    iii. (a) 435R or (b) 435R and 436F, which occurs only in one of the Fc polypeptides.

In a more specific embodiment, according to the EU numbering, one of the Fc polypeptides of the Fc domain comprises amino acid substitutions L234A, L235A, P329A, Y349C, T366S, L368A, and Y407V, and the other Fc polypeptide comprises amino acid substitutions L234A, L235A, P329A, S354C, T366W, and H435R. In one such specific embodiment, the Fc is IgG1 Fc, particularly human IgG1 Fc.

In another more specific embodiment, according to the EU numbering, one of the Fc polypeptides of the Fc domain comprises amino acid substitutions L234A, L235A, P329A, Y349C, T366S, L368A, and Y407V, and the other Fc polypeptide comprises amino acid substitutions L234A, L235A, P329A, S354C, T366W, H435R, and Y436F. In one such specific embodiment, the Fc is IgG1 Fc, particularly human IgG1 Fc.

In the context of the present disclosure, amino acid substitutions are represented as: original amino acid-position-substituted amino acid, using three-letter (Xaa) or single-letter (X) codes to represent amino acid residues, and the original amino acid may be omitted. Thus, for example, "H435R" or "435R" means that amino acid H at position 435 or the original amino acid is substituted with amino acid R, and more than 1 substituted amino acid may be contained, wherein for example, "T366Y/W" means that amino acid T at position 366 is substituted with amino acid Y or W.

In some embodiments, the bispecific antibody consists of three polypeptide chains, wherein one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 84, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 78, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 88.

In some embodiments, the bispecific antibody consists of three polypeptide chains, wherein one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 86, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 82, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 88.

In some embodiments, the bispecific antibody consists of three polypeptide chains, wherein one polypeptide chain comprises the amino acid sequence set forth in SEQ ID NO: 84, another polypeptide chain comprises the amino acid sequence set forth in SEQ ID NO: 78, and the other polypeptide chain comprises the amino acid sequence set forth in SEQ ID NO: 88.

In some embodiments, the bispecific antibody consists of three polypeptide chains, wherein one polypeptide chain comprises the amino acid sequence set forth in SEQ ID NO: 86, another polypeptide chain comprises the amino acid sequence set forth in SEQ ID NO: 82, and the other polypeptide chain comprises the amino acid sequence set forth in SEQ ID NO: 88.

The present disclosure provides exemplary bivalent bispecific antibodies.

In one example, the bispecific antibody consists of three polypeptide chains, the amino acid sequences of which are set forth in SEQ ID NOs: 84, 78, and 88, respectively. In some embodiments, the nucleotide sequences encoding the three polypeptide chains are set forth in SEQ ID NOs: 85, 79 and 89, respectively.

In one example, the bispecific antibody consists of three polypeptide chains, the amino acid sequences of which are set forth in SEQ ID NOs: 86, 82, and 88, respectively. In some embodiments, the nucleotide sequences encoding the three polypeptide chains are set forth in SEQ ID NOs: 87, 83 and 89, respectively.

## Isolated nucleic acid

The present disclosure provides an isolated nucleic acid comprising a nucleotide sequence encoding the bispecific antibody described herein. Some nucleotide sequences encoding the bispecific antibodies are illustratively listed in the sequence listing.

## Vector

The present disclosure provides a vector comprising the nucleic acid. In some embodiments, the vector is a cloning vector; in some other embodiments, the vector is an expression vector; in a specific embodiment, the expression vector is pcDNA3.1(+). The expression vector is optionally any expression vector capable of expressing the bispecific antibody described herein.

## Host cell

The present disclosure provides a host cell comprising the nucleic acid of the present disclosure or the vector of the present disclosure. In some embodiments, the host cell is a suitable host cell for cloning or expressing the bispecific antibody. In some embodiments, the host cell is a prokaryotic cell. In some other embodiments, the host cell is a eukaryotic cell. In some embodiments, the host cell is selected from a yeast cell, a mammalian cell, or other cells suitable for preparing a bispecific antibody. Mammalian cells are, for example, Chinese hamster ovary (CHO) cells or CHO-S cells.

## Pharmaceutical composition

The present disclosure provides a pharmaceutical composition comprising the bispecific antibody of the present disclosure and further comprising one or more pharmaceutically acceptable excipients. The pharmaceutically acceptable excipient includes, for example, an excipient, a diluent, an encapsulating material, a filler, a buffering agent, or other reagents.

*Method for preparing bispecific antibody*

In some embodiments, the present disclosure provides a method for preparing a bispecific antibody, comprising culturing the host cell to express the bispecific antibody, and isolating and purifying the bispecific antibody in a system. To produce the bispecific antibody, a nucleic acid encoding the bispecific antibody is isolated and inserted into one or more vectors for further use in cloning or/and expression in a host cell. The nucleic acid can be obtained using various methods known in the art, such as gene splicing and chemical synthesis.

The prepared bispecific antibody can be purified by techniques known in the art, such as high performance liquid chromatography, ion exchange chromatography, gel chromatography, affinity chromatography, size exclusion chromatography, ceramic hydroxyapatite (CHT) chromatography, and the like. The actual conditions used to purify a particular protein further depend in part on factors such as net charge, hydrophobicity and hydrophilicity, which would have been apparent to those skilled in the art. For affinity chromatography, purification can be carried out using antibodies, ligands, receptors, or antigens that bind to the bispecific antibody. For example, for the affinity chromatography of the bispecific antibody of the present disclosure, a matrix with protein A or protein G can be used for purification. The bispecific antibody of the present disclosure can be purified by affinity chromatography, ion exchange chromatography, gel chromatography, or/and CHT chromatography sequentially.

The purity of the bispecific antibody can be determined by any of a variety of well-known analytical methods, such as gel electrophoresis, high performance liquid chromatography, and size exclusion chromatography. The physicochemical properties or/and biological activity of the bispecific antibody of the present disclosure can be identified, screened, or characterized by a variety of assay methods known in the art.

*Use*

The present disclosure provides use of the bispecific antibody of the present disclosure. In some specific embodiments, the bispecific antibody used may be GC35 or/and GC39.

The present disclosure provides use of the bispecific antibody or the pharmaceutical composition for preparing a medicament for treating a GPRC5D-expressing disease. In some embodiments, the bispecific antibody or the pharmaceutical composition is used in combination with one or more additional therapeutic agents to prepare the medicament. The additional therapeutic agent may be a therapeutic agent for a tumor or a therapeutic agent for an autoimmune disease known in the art.

The present disclosure provides a method for treating a GPRC5D-expressing disease comprising administering to a subject in need the bispecific antibody or the pharmaceutical composition. The present disclosure provides a method for treating a GPRC5D-expressing disease comprising administering to a subject in need a therapeutically effective amount of the bispecific antibody or the pharmaceutical composition. In some embodiments, the disease is a tumor or an autoimmune disease.

In some embodiments, the tumor is a non-solid tumor. In some embodiments, the tumor is a hematological tumor. In some embodiments, the tumor is B-cell lymphoma. In some embodiments, the tumor is a B-cell lymphoma expressing GPRC5D. In some embodiments, the tumor is multiple myeloma (MM). In some embodiments, the tumor is multiple myeloma expressing GPRC5D.

In some embodiments, the autoimmune disease is, for example, systemic lupus erythematosus and/or rheumatoid arthritis.

**DETAILED DESCRIPTION**

The present disclosure further provides the following specific embodiments, which, however, are not intended to limit the scope of the present disclosure:

Embodiment 1. A bispecific antibody, comprising a first antigen-binding portion binding to GPRC5D and a second antigen-binding portion binding to CD3, wherein the first antigen-binding portion comprises: an HCDR1 comprising the amino acid sequence set forth in SEQ ID NO: 1, 9, 17, 25, or 33, an HCDR2 comprising the amino acid sequence set forth in SEQ ID NO: 2, 59, 10, 18, 26, or 34, an HCDR3 comprising the amino acid sequence set forth in SEQ ID NO: 3, 11, 19, 27, or 35, an LCDR1 comprising the amino acid sequence set forth in SEQ ID NO: 4, 12, 20, 28, or 36, an LCDR2 comprising the amino acid sequence set forth in SEQ ID NO: 5, 13, 21, 29, or 37, and an LCDR3 comprising the amino acid sequence set forth in SEQ ID NO: 6, 14, 22, 30, or 38.

Embodiment 2. The bispecific antibody according to embodiment 1, wherein the first antigen-binding portion comprises:

(1) an HCDR1 comprising the amino acid sequence set forth in SEQ ID NO: 1, an HCDR2 comprising the amino acid sequence set forth in SEQ ID NO: 59, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, wherein $X_1$ is selected from D and E, and $X_2$ is selected from G and A;

(2) an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;

(3) an HCDR1 comprising the amino acid sequence set forth in SEQ ID NO: 1, an HCDR2 comprising the amino acid sequence set forth in SEQ ID NO: 60, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;

(4) an HCDR1 comprising the amino acid sequence set forth in SEQ ID NO: 1, an HCDR2 comprising the amino acid sequence set forth in SEQ ID NO: 61, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;

(5) an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14;

(6) an HCDR1 comprising the amino acid sequence set forth in SEQ ID NO: 17, an HCDR2 comprising the amino acid sequence set forth in SEQ ID NO: 18, an HCDR3 comprising the amino acid sequence set forth in SEQ ID NO: 19, an LCDR1 comprising the amino acid sequence set forth in SEQ ID NO: 20, an LCDR2 comprising the amino acid sequence set forth in SEQ ID NO: 21, and an LCDR3 comprising the amino acid sequence set forth in SEQ ID NO: 22;

(7) an HCDR1 comprising the amino acid sequence set forth in SEQ ID NO: 25, an HCDR2 comprising the amino acid sequence set forth in SEQ ID NO: 26, an HCDR3 comprising the amino acid sequence set forth in SEQ ID NO: 27, an LCDR1 comprising the amino acid sequence set forth in SEQ ID NO: 28, an LCDR2 comprising the amino acid sequence set forth in SEQ ID NO: 29, and an LCDR3 comprising the amino acid sequence set forth in SEQ ID NO: 30; or

(8) an HCDR1 comprising the amino acid sequence set forth in SEQ ID NO: 33, an HCDR2 comprising the amino acid sequence set forth in SEQ ID NO: 34, an HCDR3 comprising the amino acid sequence set forth in SEQ ID NO: 35, an LCDR1 comprising the amino acid sequence set forth in SEQ ID NO: 36, an LCDR2 comprising the amino acid sequence set forth in SEQ ID NO: 37, and an LCDR3 comprising the amino acid sequence set forth in SEQ ID NO: 38.

Embodiment 3. A bispecific antibody, comprising a first antigen-binding portion binding to GPRC5D and a second antigen-binding portion binding to CD3, wherein the first antigen-binding portion comprises: an HCDR1, an HCDR2, and an HCDR3 in a heavy chain variable region having the amino acid sequence set forth in SEQ ID NO: 7, 15, 23, 31, 39, 66, or 68, and an LCDR1, an LCDR2, and an LCDR3 in a light chain variable region having the amino acid sequence set forth in SEQ ID NO: 8, 16, 24, 32, 40, 67, or 69.

Embodiment 4. The bispecific antibody according to embodiment 3, wherein the first antigen-binding portion comprises:

(1) an HCDR1, an HCDR2, and an HCDR3 in a heavy chain variable region having the amino acid sequence set forth in SEQ ID NO: 7, and an LCDR1, an LCDR2, and an LCDR3 in a light chain variable region having the amino acid sequence set forth in SEQ ID NO: 8;

(2) an HCDR1, an HCDR2, and an HCDR3 in a heavy chain variable region having the amino acid sequence set forth in SEQ ID NO: 15, and an LCDR1, an LCDR2, and an LCDR3 in a light chain variable region having the amino acid sequence set forth in SEQ ID NO: 16;

(3) an HCDR1, an HCDR2, and an HCDR3 in a heavy chain variable region having the amino acid sequence set forth in SEQ ID NO: 23, and an LCDR1, an LCDR2, and an LCDR3 in a light chain variable region having the amino acid sequence set forth in SEQ ID NO: 24;

(4) an HCDR1, an HCDR2, and an HCDR3 in a heavy chain variable region having the amino acid sequence set forth in SEQ ID NO: 31, and an LCDR1, an LCDR2, and an LCDR3 in a light chain variable region having the amino acid sequence set forth in SEQ ID NO: 32;

(5) an HCDR1, an HCDR2, and an HCDR3 in a heavy chain variable region having the amino acid sequence set forth in SEQ ID NO: 39, and an LCDR1, an LCDR2, and an LCDR3 in a light chain variable region having the amino acid sequence set forth in SEQ ID NO: 40;

(6) an HCDR1, an HCDR2, and an HCDR3 in a heavy chain variable region having the amino acid sequence set forth in SEQ ID NO: 62, and an LCDR1, an LCDR2, and an LCDR3 in a light chain variable region having the amino acid sequence set forth in SEQ ID NO: 67;

(7) an HCDR1, an HCDR2, and an HCDR3 in a heavy chain variable region having the amino acid sequence set forth in SEQ ID NO: 63, and an LCDR1, an LCDR2, and an LCDR3 in a light chain variable region having the amino acid sequence set forth in SEQ ID NO: 67;

(8) an HCDR1, an HCDR2, and an HCDR3 in a heavy chain variable region having the amino acid sequence set forth in SEQ ID NO: 64, and an LCDR1, an LCDR2, and an LCDR3 in a light chain variable region having the amino acid sequence set forth in SEQ ID NO: 67;

(9) an HCDR1, an HCDR2, and an HCDR3 in a heavy chain variable region having the amino acid sequence set forth in SEQ ID NO: 65, and an LCDR1, an LCDR2, and an LCDR3 in a light chain variable region having the amino acid sequence set forth in SEQ ID NO: 67;

(10) an HCDR1, an HCDR2, and an HCDR3 in a heavy chain variable region having the amino acid sequence set forth in SEQ ID NO: 66, and an LCDR1, an LCDR2, and an LCDR3 in a light chain variable region having the amino acid sequence set forth in SEQ ID NO: 67, wherein $X_3$ is selected from Q and V, $X_4$ is selected from P and A, $X_5$ is selected from L and V, $X_6$ is selected from K and A, $X_7$ is selected from I and M, $X_8$ is selected from D and E, $X_9$ is selected from G and A, $X_{10}$ is selected from K and R, $X_{11}$ is selected from A and V, $X_{12}$ is selected from K and T, $X_{13}$ is selected from S and A, $X_{14}$ is selected from T and R, $X_{15}$ is selected from S and T, and $X_{16}$ is selected from A and Q; or

(11) an HCDR1, an HCDR2, and an HCDR3 in a heavy chain variable region having the amino acid sequence set forth in SEQ ID NO: 68, and an LCDR1, an LCDR2, and an LCDR3 in a light chain variable region having the amino acid sequence set forth in SEQ ID NO: 69.

Embodiment 5. The bispecific antibody according to any one of embodiments 1-4, wherein the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, 15, 23, 31, 39, 62, 63, 64, 65, 66, or 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8, 16, 24, 32, 40, 67, or 69.

Embodiment 6. The bispecific antibody according to any one of embodiments 1-5, wherein the first antigen-binding portion comprises:

(1) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;

(2) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;

(3) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 23, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24;

(4) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 31, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32;

(5) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 39, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40;

(6) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(7) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(8) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(9) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(10) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 66, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67; or

(11) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69.

Embodiment 7. The bispecific antibody according to any one of embodiments 1-6, wherein the first antigen-binding portion comprises:

(1) a heavy chain variable region comprising the amino acid sequence set forth in SEQ ID NO: 7, and a light chain variable region comprising the amino acid sequence set forth in SEQ ID NO: 8;

(2) a heavy chain variable region comprising the amino acid sequence set forth in SEQ ID NO: 15, and a light chain variable region comprising the amino acid sequence set forth in SEQ ID NO: 16;

(3) a heavy chain variable region comprising the amino acid sequence set forth in SEQ ID NO: 23, and a light chain variable region comprising the amino acid sequence set forth in SEQ ID NO: 24;

(4) a heavy chain variable region comprising the amino acid sequence set forth in SEQ ID NO: 31, and a light chain variable region comprising the amino acid sequence set forth in SEQ ID NO: 32;

(5) a heavy chain variable region comprising the amino acid sequence set forth in SEQ ID NO: 39, and a light chain variable region comprising the amino acid sequence set forth in SEQ ID NO: 40;

(6) a heavy chain variable region comprising the amino acid sequence set forth in SEQ ID NO: 62, and a light chain variable region comprising the amino acid sequence set forth in SEQ ID NO: 67;

(7) a heavy chain variable region comprising the amino acid sequence set forth in SEQ ID NO: 63, and a light chain variable region comprising the amino acid sequence set forth in SEQ ID NO: 67;

(8) a heavy chain variable region comprising the amino acid sequence set forth in SEQ ID NO: 64, and a light chain variable region comprising the amino acid sequence set forth in SEQ ID NO: 67;

(9) a heavy chain variable region comprising the amino acid sequence set forth in SEQ ID NO: 65, and a light chain variable region comprising the amino acid sequence set forth in SEQ ID NO: 67;

(10) a heavy chain variable region comprising the amino acid sequence set forth in SEQ ID NO: 66, and a light chain variable region comprising the amino acid sequence set forth in SEQ ID NO: 67; or

(11) a heavy chain variable region comprising the amino acid sequence set forth in SEQ ID NO: 68, and a light chain variable region comprising the amino acid sequence set forth in SEQ ID NO: 69.

Embodiment 8. The bispecific antibody according to embodiment 1, wherein the first antigen-binding portion comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6.

Embodiment 9. The bispecific antibody according to embodiment 8, wherein the first antigen-binding portion comprises: a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67; preferably, the first antigen-binding portion comprises: a heavy chain variable region comprising the amino acid sequence set forth in SEQ ID NO: 62, and a light chain variable region comprising the amino acid sequence set forth in SEQ ID NO: 67.

Embodiment 10. The bispecific antibody according to embodiment 1, wherein the first antigen-binding portion comprises: an HCDR1 comprising the amino acid sequence set forth in SEQ ID NO: 33, an HCDR2 comprising the amino acid sequence set forth in SEQ ID NO: 34, an HCDR3 comprising the amino acid sequence set forth in SEQ ID NO: 35, an LCDR1 comprising the amino acid sequence set forth in SEQ ID NO: 36, an LCDR2 comprising the amino acid sequence set forth in SEQ ID NO: 37, and an LCDR3 comprising the amino acid sequence set forth in SEQ ID NO: 38.

Embodiment 11. The bispecific antibody according to embodiment 10, wherein the first antigen-binding portion comprises: a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69; preferably, the first antigen-binding portion comprises: a heavy chain variable region comprising the amino acid sequence set forth in SEQ ID NO: 68, and a light chain variable region comprising the amino acid sequence set forth in SEQ ID NO: 69.

Embodiment 12. The bispecific antibody according to any one of embodiments 1-11, wherein the second antigen-binding portion comprises: an HCDR1 comprising the amino acid sequence set forth in SEQ ID NO: 94, an HCDR2 comprising the amino acid sequence set forth in SEQ ID NO: 95, an HCDR3 comprising the amino acid sequence set forth in SEQ ID NO: 96, an LCDR1 comprising the amino acid sequence set forth in SEQ ID NO: 97, an LCDR2 comprising the amino acid sequence set forth in SEQ ID NO: 98, and an LCDR3 comprising the amino acid sequence set forth in SEQ ID NO: 99.

Embodiment 13. The bispecific antibody according to any one of embodiments 1-11, wherein the second antigen-binding portion comprises: an HCDR1, an HCDR2, and an HCDR3 in a heavy chain variable region variable region having the amino acid sequence set forth in SEQ ID NO: 100, and an LCDR1, an LCDR2, and an LCDR3 in a light chain variable region having the amino acid sequence set forth in SEQ ID NO: 101.

Embodiment 14. The bispecific antibody according to any one of embodiments 1-13, wherein the second antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 100, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 101.

Embodiment 15. The bispecific antibody according to any one of embodiments 1-13, wherein the second antigen-binding portion comprises: a heavy chain variable region comprising the amino acid sequence set forth in SEQ ID NO: 100 and a light chain variable region comprising the amino acid sequence set forth in SEQ ID NO: 101.

Embodiment 16. The bispecific antibody according to any one of embodiments 1-15, wherein the first antigen-binding portion is murine, chimeric, or humanized.

Embodiment 17. The bispecific antibody according to any one of embodiments 1-16, wherein the second antigen-binding portion is murine, chimeric, or humanized.

Embodiment 18. The bispecific antibody according to any one of embodiments 1-17, wherein both the first antigen-binding portion and the second antigen-binding portion are each independently a Fab, an scFv, or an scFab.

Embodiment 19. The bispecific antibody according to embodiment 18, wherein the first antigen-binding portion is a Fab, and the second antigen-binding portion is an scFv.

Embodiment 20. The bispecific antibody according to any one of embodiments 1-19, wherein the bispecific antibody further comprises an Fc domain composed of two Fc polypeptides.

Embodiment 21. The bispecific antibody according to embodiment 20, wherein the first antigen-binding portion is a Fab, and the second antigen-binding portion is an scFv; the first antigen-binding portion, at the C-terminus of its Fab heavy chain, is fused to the N-terminus of one of the Fc polypeptides of the Fc domain, and the second antigen-binding portion, at the C-terminus, is fused to the N-terminus of the other Fc polypeptide of the Fc domain.

Embodiment 22. The bispecific antibody according to embodiment 21, wherein the structure of the second antigen-binding portion from the N-terminus to the C-terminus is VH-VL or VL-VH; optionally, the heavy chain variable region of the second antigen-binding portion is fused to the light chain variable region of the second antigen-binding portion via a peptide linker, and preferably, the peptide linker is $(GGGGS)_3$.

Embodiment 23. The bispecific antibody according to any one of embodiments 20-22, wherein the Fc domain is an IgG Fc domain, preferably an IgG1 Fc domain.

Embodiment 24. The bispecific antibody according to embodiment 23, wherein the IgG Fc domain is a human IgG Fc domain, preferably a human IgG1 Fc domain.

Embodiment 25. The bispecific antibody according to any one of embodiments 20-24, wherein the Fc domain comprises an amino acid substitution that promotes association of the two Fc polypeptides of the Fc domain.

Embodiment 26. The bispecific antibody according to embodiment 25, wherein according to the EU numbering, one of the Fc polypeptides of the Fc domain comprises amino acid substitutions 354C and 366Y/W, and the other Fc polypeptide comprises amino acid substitutions 349C, 366S, 368A, and 407T/V.

Embodiment 27. The bispecific antibody according to any one of embodiments 20-26, wherein the Fc domain comprises an amino acid substitution that reduces the binding affinity of the Fc domain to an Fc receptor and/or reduces effector function.

Embodiment 28. The bispecific antibody according to embodiment 27, wherein according to the EU numbering, the amino acid substitution that reduces the binding affinity of the Fc domain to an Fc receptor and/or reduces effector function is at one or more positions selected from the group consisting of 234, 235, and 329; preferably, the two Fc polypeptides of the Fc domain comprise amino acid substitutions 234A, 235A, and 329G, or comprise amino acid substitutions 234A, 235A, and 329A.

Embodiment 29. The bispecific antibody according to any one of embodiments 20-28, wherein the Fc domain comprises an amino acid substitution that reduces or eliminates the binding of a CH3 region of one Fc polypeptide in the Fc domain to Protein A.

Embodiment 30. The bispecific antibody according to embodiment 29, wherein according to the EU numbering, the Fc domain comprises an amino acid substitution (a) 435R or (b) 435R and 436F, which occurs only in one of the Fc polypeptides.

Embodiment 31. The bispecific antibody according to any one of embodiments 20-24, wherein according to the EU numbering, one of the Fc polypeptides of the Fc domain comprises amino acid substitutions L234A, L235A, P329A, Y349C, T366S, L368A, and Y407V, and the other Fc polypeptide comprises amino acid substitutions (a) L234A, L235A, P329A, S354C, T366W, and H435R, or (b) L234A, L235A, P329A, S354C, T366W, H435R, and Y436F.

Embodiment 32. The bispecific antibody according to any one of embodiments 1-31, wherein the bispecific antibody is bivalent.

Embodiment 33. The bispecific antibody of any one of embodiments 1-32, wherein the bispecific antibody consists of three polypeptide chains, wherein:

(1) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 84, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 78, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 88; or

(2) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 86, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 82, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 88.

Embodiment 34. A bispecific antibody, wherein the bispecific antibody consists of three polypeptide chains, wherein:

(1) one polypeptide chain comprises the amino acid sequence set forth in SEQ ID NO: 84, another polypeptide chain comprises the amino acid sequence set forth in SEQ ID NO: 78, and the other polypeptide chain comprises the amino acid sequence set forth in SEQ ID NO: 88; or
(2) one polypeptide chain comprises the amino acid sequence set forth in SEQ ID NO: 86, another polypeptide chain comprises the amino acid sequence set forth in SEQ ID NO: 82, and the other polypeptide chain comprises the amino acid sequence set forth in SEQ ID NO: 88.

Embodiment 35. An isolated nucleic acid, comprising a nucleotide sequence encoding the bispecific antibody according to any one of embodiments 1-34.

Embodiment 36. A vector, comprising the nucleic acid according to embodiment 35.

Embodiment 37. A host cell, comprising the nucleic acid according to embodiment 35 or the vector according to embodiment 36.

Embodiment 38. A method for preparing the bispecific antibody according to any one of embodiments 1-34, comprising culturing the host cell according to embodiment 37 to express the bispecific antibody, and isolating and purifying the bispecific antibody in a system.

Embodiment 39. A pharmaceutical composition, comprising the bispecific antibody according to any one of embodiments 1-34 and a pharmaceutically acceptable excipient.

Embodiment 40. Use of the bispecific antibody according to any one of embodiments 1-34 or the pharmaceutical composition according to embodiment 39 in preparing a medicament for treating a GPRC5D-expressing disease.

Embodiment 41. The use according to embodiment 40, wherein the disease is an autoimmune disease or a tumor; the tumor is preferably a non-solid tumor, more preferably multiple myeloma.

Embodiment 42. The use according to embodiment 40 or 41, wherein the bispecific antibody or the pharmaceutical composition is used in combination with one or more additional therapeutic agents to prepare the medicament.

Embodiment 43. A method for treating a GPRC5D-expressing disease, comprising administering to a subject in need a therapeutically effective amount of the bispecific antibody according to any one of embodiments 1-34 or the pharmaceutical composition according to embodiment 39.

Embodiment 44. The method according to embodiment 43, wherein the disease is an autoimmune disease or a tumor; the tumor is preferably a non-solid tumor, more preferably multiple myeloma.

Embodiment 45. A monospecific antibody binding to GPRC5D or an antigen-binding portion thereof, comprising a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, 63, 64, 65, 66, or 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67 or 69.

Embodiment 46. The monospecific antibody or the antigen-binding portion thereof according to embodiment 45, comprising:

(1) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;
(2) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;
(3) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;
(4) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(5) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 66, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69; or

(6) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69.

Embodiment 47. The monospecific antibody or the antigen-binding portion thereof according to embodiment 45 or 46, comprising:

(1) a heavy chain variable region comprising the amino acid sequence set forth in SEQ ID NO: 62, and a light chain variable region comprising the amino acid sequence set forth in SEQ ID NO: 67;
(2) a heavy chain variable region comprising the amino acid sequence set forth in SEQ ID NO: 63, and a light chain variable region comprising the amino acid sequence set forth in SEQ ID NO: 67;
(3) a heavy chain variable region comprising the amino acid sequence set forth in SEQ ID NO: 64, and a light chain variable region comprising the amino acid sequence set forth in SEQ ID NO: 67;
(4) a heavy chain variable region comprising the amino acid sequence set forth in SEQ ID NO: 65, and a light chain variable region comprising the amino acid sequence set forth in SEQ ID NO: 67; or
(5) a heavy chain variable region comprising the amino acid sequence set forth in SEQ ID NO: 68, and a light chain variable region comprising the amino acid sequence set forth in SEQ ID NO: 69.

Embodiment 48. An antibody-drug conjugate, comprising the monospecific antibody or the antigen-binding portion thereof according to any one of embodiments 45-47.
Embodiment 49. An anti-GPRC5D chimeric antigen receptor, comprising the monospecific antibody or the antigen-binding portion thereof according to any one of embodiments 45-47.
Embodiment 50. An engineered immune effector cell, comprising the anti-GPRC5D chimeric antigen receptor according to embodiment 49.

For clarity, the present disclosure is further described with the following examples, which are, however, not intended to limit the scope of the present disclosure. The reagents used in the present disclosure are commercially available and can be used without further purification.

### Example 1: Antigen immunization

1. DNA antigen immunization

A cDNA sequence encoding human GPRC5D protein (with the amino acid sequence set forth in SEQ ID NO: 41) was obtained by gene synthesis and subcloned into expression vector pcDNA3.1(+) to construct a plasmid pcDNA3.1-hGPRC5D. A large quantity of plasmid was prepared according to the instructions for EndoFree Plasmid Giga Kit (QIAGEN, Cat. No. 12391).
The plasmid pcDNA3.1-hGPRC5D was used as the antigen to immunize A/J mice (Model Animal Research Center of Nanjing University), BALB/c mice (Shanghai Lingchang), and SJL mice (Beijing Vital River) separately. Firstly, hyaluronidase (Sigma, Cat. No. H4272) was pre-injected into the left and right hind limb muscles of each mouse for pretreatment. Then, CpG (InvivoGen, Cat. No. tlrl-1826) and the plasmid pcDNA3.1-hGPRC5D were mixed well in a mass ratio of 1:1, and the well-mixed antigen complex was injected into the pretreated site of the muscle in the mice with an *in vivo* gene transfer instrument (Shanghai Teresa Biotechnology Co., Ltd., type II). The immunization procedures described above were repeated once every 2 to 3 weeks for a total of 4 immunizations. After the completion of the immunizations, the serum was collected from each mouse.

2. Mouse serum titer assessment

The antibody titer in the mouse serum was detected by FACS to assess the immune response of anti-human GPRC5D antibodies produced in mice immunized with DNA antigens. Mice with a higher serum titer were selected for final boost before subsequent splenocyte fusion.

3. Cell antigen immunization

The plasmid pcDNA3.1-hGPRC5D was transfected into CHO-K1 cells according to the instructions for lipofectamine3000 transfection reagent (Thermo, Cat. No. L3000015) to give a cell line CHO-K1-hGPRC5D$^{hi}$ with stable high expression of hGPRC5D. Mice were subjected to final boost with the CHO-K1-hGPRC5D$^{hi}$ cell described above as the antigen: 3-4 days before splenocyte fusion, a CHO-K1-hGPRC5D$^{hi}$ cell suspension at a final cell concentration of $5 \times 10^7$ cells/mL and CpG (InvivoGen, Cat. No. tlrl-1826) at a final concentration of 0.5 mg/mL were mixed well, and then intraperitoneally injected into the mice at 100 $\mu$L/mouse.

**Example 2: Screening of mouse anti-human GPRCSD antibodies**

1. Preparation of hybridoma cells

On the day of fusion, the mouse spleen was collected aseptically and ground before a red cell lysis buffer (Sigma, Cat. No. R7757) was added. After the cells were washed with a PBS solution, the splenocytes were resuspended in an electrofusion buffer (BTX, Cat. No. 47-0001) and mixed well with mouse myeloma cells SP2/0 in a cell number ratio of 2:1. The splenocyte fusion was performed on an electrofusion system (BTX). The fused cells were diluted by adding a hybridoma medium (Gibco, Cat. No. 12045-076) containing $1\times$ HAT (Gibco, Cat. No. 21060-017), and then cultured at 37°C with 5% $CO_2$ for 7-10 days to prepare hybridoma cells. The hybridoma cell culture supernatant was collected for screening of mouse anti-human GPRC5D antibodies.

2. Construction of stable cell lines

The following cell lines were constructed to assess the species cross-binding activity of the mouse anti-human GPRC5D antibodies at the cellular level: cDNAs encoding full-length monkey GPRC5D (with amino acid sequence set forth in SEQ ID NO: 42) and mouse GPRC5D (with amino acid sequence set forth in SEQ ID NO: 43) were obtained by gene synthesis, and then subcloned into a vector pcDNA3.1(+) to give pcDNA3.1-cynoGPRC5D and pcDNA3.1-murineGPRC5D recombinant plasmids. Plasmids pcDNA3.1-cynoGPRCSD and pcDNA3.1-murineGPRC5D were separately transfected into CHO-K1 cells according to the instructions for lipofectamine3000 transfection reagent (Thermo, Cat. No. L3000015) to give stable cell lines CHO-K1-cynoGPRC5D and CHO-K1-murineGPRC5D.

3. Preliminary cell screening by ELISA

CHO-K1-hGPRC5D$^{hi}$ cells in the logarithmic growth phase were collected and resuspended in a DMEM/F-12 (Gibco, Cat. No. 11320033) medium containing 10% (by volume) of FBS. The cell concentration was adjusted to $1 \times 10^6$ cells/mL. The cells were plated into a 96-well flat bottom plate at 100 $\mu$L/well, and then incubated overnight at 37°C with 5% $CO_2$. On the next day, the cell plate was washed with PBS before 4% (by volume) Paraformaldehyde Fix Solution (Beyotime, Cat. No. P0099) was added, and let stand at room temperature for 30 min. After the plate was washed with PBS, a PBS solution containing 3% (by volume) of BSA was added at 200 $\mu$L/well. The mixture was blocked at room temperature for 2 h. After blocking, the hybridoma cell culture supernatant was added to the cell plate at 100 $\mu$L/well, and the mixture was incubated at room temperature for 2 h. The plate was washed with PBS. Then, an HRP-conjugated goat anti-mouse IgG+IgM(H+L) antibody (Jackson Immuno Research, Cat. No. 115-035-068) was added, and the mixture was incubated at room temperature for 1 h. After the plate was washed with PBS, a TMB reaction solution (Solarbio, Cat. No. RP1200) was added at 100 $\mu$L/well, and the mixture was incubated at room temperature in the dark for 5 min. The reaction was stopped with 0.5 M $H_2SO_4$. The OD$_{450\,nm}$ value was read on a Bio-rad iMark microplate reader. Hybridoma cells corresponding to the culture supernatant with an absorbance greater than 1.0 were selected as the positive clones for subsequent screening by FACS.

4. Antibody screening by flow cytometry FACS

A CHO-K1-cynoGPRC5D cell suspension at a cell concentration of $5 \times 10^5$ cells/mL was plated into a 96-well U-bottom plate at 100 $\mu$L/well. The culture supernatant of the positive clones was added at 100 $\mu$L/well. The mixture was mixed well, and then incubated at 4°C for 1 h. The cells were washed with PBS containing 2% (by volume) of FBS. An AlexaFluor488-conjugated goat anti-mouse IgG+IgM(H+L) antibody (Jackson Immuno Research, Cat. No. 115-545-044) was added, and the mixture was incubated at 4°C for 1 h. The cells were washed with PBS containing 2% (by volume) of FBS and then resuspended. Fluorescence signals were detected on a flow cytometer (Sartorius, iQue3). The binding of the anti-hGPRC5D antibodies to CHO-K1-cynoGPRC5D cells was analyzed by the mean fluorescence intensity (MFI) of staining.

5. Preparation of subclones

Hybridoma cells that were positively for binding to both CHO-K1-hGPRC5D$^{hi}$ and CHO-K1-cynoGPRC5D after the cell screening by ELISA and FACS were selected for expansion culture. The expanded cells were mixed well with a semi-solid medium D (Stemcell, Cat. No. 3810) in a volume ratio of 1:10, and then incubated at 37°C with 5% $CO_2$ for 5 days. The monoclonal cell mass was pipetted under 4-fold microscopy and transferred to a 96-well plate plated with a hybridoma medium (Gibco, Cat. No. 12045-076). Then the monoclonal cell mass was placed at 37°C with 5% $CO_2$ for 2 days to continue the culture. The subcloned cell culture supernatant of each well was collected for further screening of subclones.

6. Screening of subclones

(1) Screening of anti-hGPRC5D antibodies by FACS

An NCI-H929 cell suspension at a cell concentration of $3 \times 10^5$ cells/mL was plated into a 96-well U-bottom plate at 100 $\mu$L/well. The subcloned cell culture supernatant was added at 100 $\mu$L/well or an equal volume of medium was added. The mixture was mixed well, and then incubated at 4°C for 1 h. The cells were washed with PBS containing 2% (by volume) of FBS. An AlexaFluor488-conjugated goat anti-mouse IgG+IgM(H+L) antibody (Jackson Immuno Research, Cat. No. 115-545-044) was added, and the mixture was incubated at 4°C for 1 h. The cells were washed with PBS containing 2% (by volume) of FBS and then resuspended. Fluorescence signals were detected on a flow cytometer (Sartorius, iQue3). The binding of the mouse anti-hGPRC5D antibody to NCI-H929 cells was analyzed by the mean fluorescence intensity (MFI) of staining. Part of the results are shown in Table 1.

Table 1. Binding assay of mouse anti-hGPRC5D antibodies to NCI-H929 cells by FACS

| Clone No. | MFI | Clone No. | MFI | Clone No. | HMFI |
|-----------|--------|-----------|-------|-----------|-------|
| mu-7 | 53151 | mu-74.3 | 18078 | mu-131 | 12826 |
| mu-9 | 10704 | mu-89 | 11919 | mu-153 | 46867 |
| mu-45 | 21606 | mu-105 | 13248 | mu-164 | 14184 |
| mu-38.1 | 75956 | mu-125 | 11670 | mu-169 | 59318 |
| mu-35 | 54719 | mu-126 | 10271 | mu-180 | 26579 |
| mu-51 | 95531 | mu-128 | 15661 | mu-183 | 27008 |
| mu-58 | 464150 | mu-129 | 7904 | Medium | 8242 |

(2) Cell ELISA screening of anti-hGPRC5D antibodies

CHO-K1-hGPRC5D$^{hi}$ cells, CHO-K1-cynoGPRC5D cells, CHO-K1-murineGPRC5D cells, and CHO-K1 cells in the logarithmic growth phase were separately collected and resuspended in a DMEM/F-12 (Gibco, Cat. No. 11320033) medium containing 10% (by volume) of FBS. The cell concentration was adjusted to $1 \times 10^6$ cells/mL. The cells were plated into a 96-well flat bottom plate at 100 $\mu$L/well, and then incubated overnight at 37°C with 5% $CO_2$. On the next day, the cell plate was washed with PBS before 4% (by volume) Paraformaldehyde Fix Solution (Beyotime, Cat. No. P0099) was added, and let stand at room temperature for 30 min. After the plate was washed with PBS, a PBS solution containing 3% (by volume) of BSA was added at 200 $\mu$L/well. The mixture was blocked at room temperature for 2 h. After blocking, the subcloned cell culture supernatant was added to each cell plate at 50 $\mu$L/well, and the mixture was incubated at room temperature for 2 h. The plate was washed with PBS. Then, an HRP-conjugated goat anti-mouse IgG+IgM(H+L) antibody (Jackson Immuno Research, Cat. No. 115-035-068) was added, and the mixture was incubated at room temperature for 1 h. After the plate was washed with PBS, a TMB reaction solution (Solarbio, Cat. No. RP1200) was added at 100 $\mu$L/well, and the mixture was incubated at room temperature in the dark for 5 min. The reaction was stopped with 0.5 M $H_2SO_4$. The

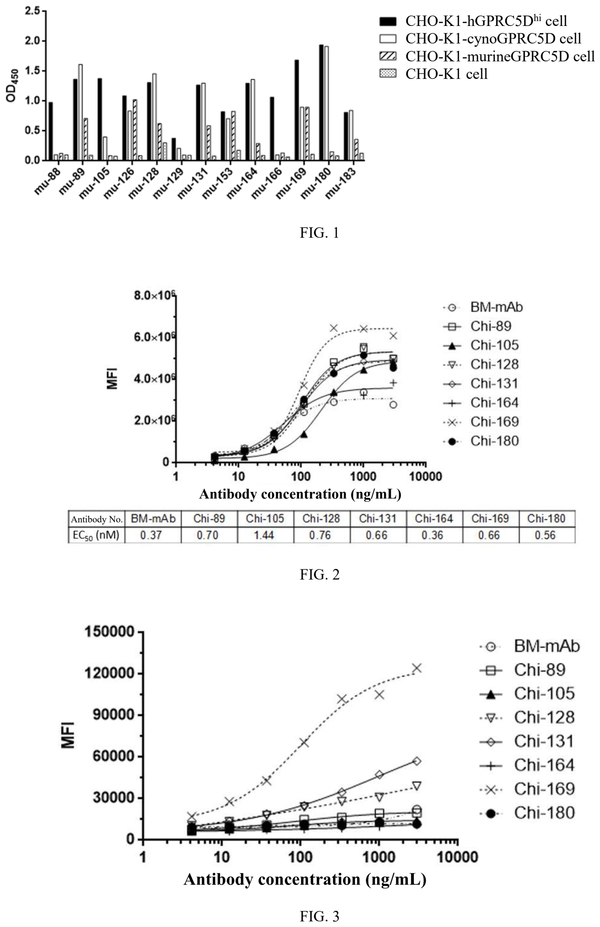

OD$_{450\text{ nm}}$ value was read on a Bio-rad iMark microplate reader. As shown in FIG. 1, typical mouse anti-hGPRC5D antibodies (e.g., mu-89, mu-105, mu-126, mu-128, mu-131, mu-153, mu-164, mu-169, mu-180, and mu-183, etc.) had at least a 2-fold difference in absorbance against CHO-K1-hGPRC5D$^{hi}$ cells, CHO-K1-cynoGPRC5D cells, and/or CHO-K1-murineGPRC5D cells as compared to the control cell CHO-K1.

## Example 3: Preparation of anti-hGPRCSD chimeric antibodies

1. Sequencing of variable regions encoding mouse anti-hGPRC5D antibodies

The total RNA of the hybridoma cells selected was isolated according to the instructions for an RNA extraction kit (Takara, Cat. No. 9767), and a first-strand cDNA was synthesized by a reverse transcription kit (Thermo, Cat. No. K1652). The first-strand cDNA was used as the template, and mixed with a mouse heavy chain constant region primer and a light chain constant region primer separately. The cDNA was then cloned and sequenced by polymerase chain reaction (PCR) to give the sequences of the variable regions of the mouse anti-hGPRC5D antibodies.

2. Construction of expression vectors of chimeric antibodies

The nucleotide sequences of the heavy chain variable region (VH) and the light chain variable region (VL) of the mouse anti-hGPRC5D antibodies were linked to the nucleotide sequences of a human IgG1 heavy chain constant region and a Kappa light chain constant region, respectively, by chemical synthesis. Recombinant human-mouse chimeric antibody expression vectors were constructed using a pcDNA3.1(+) vector for transfection and expression to prepare the chimeric antibodies. The VH/VL amino acid sequences and full-length amino acid sequences of the chimeric antibodies are shown in Table 2.

Table 2. VH/VL and full-length amino acid sequences of chimeric antibodies (numbers in the table show sequence numbers SEQ ID NOs:)

| Clone No. | VH | VL | Heavy chain | Light chain |
|---|---|---|---|---|
| Chi-89 | 23 | 24 | 53 | 54 |
| Chi-105 | 31 | 32 | 55 | 56 |
| Chi-128 | 39 | 40 | 57 | 58 |
| Chi-131 | 7 | 8 | 49 | 50 |
| Chi-169 | 15 | 16 | 51 | 52 |

3. Construction of expression vector of positive reference antibody

GC5B596 was selected as the reference antibody (a monoclonal antibody from WO2018017786A2). The nucleotide sequences of the VH (SEQ ID NO: 44) and VL (SEQ ID NO: 45) of the antibody were obtained by chemical synthesis, and were separately linked to the nucleotide sequences of a human IgG1 heavy chain constant region and a Kappa light chain constant region. An expression vector expressing the positive reference antibody was constructed using a pcDNA3.1(+) vector for transfection and expression to prepare the positive reference antibody. The positive reference antibody (monoclonal antibody) is designated as BM-mAb herein.

4. Preparation of anti-hGPRC5D chimeric antibody and positive reference antibody

The anti-hGPRC5D antibody was transiently transfected into cells according to the instruction manual of the expiCHO expression system (Gibco, Cat. No. A29129). The transfected expiCHO cells were cultured at 37°C with 8% CO$_2$ for 7 days while shaking. The cell culture supernatant was collected, and the clear culture supernatant was loaded onto a protein A column (G.E.Healthcare, Cat. No. 17-5474). The protein A column was washed with 10 column volumes of PBS buffer. The IgG antibodies bound to the protein A column were eluted with an acetic acid buffer (300 mM acetic acid, pH 3.6) and collected. The collected IgG antibody components were transferred into the PBS buffer by an ultrafiltration device (molecular weight cut-off 30 kDa, Millipore, Cat. No. UFC903024) to give anti-hGPRC5D antibody solutions.

## Example 4: Binding activity assay for anti-hGPRCSD antibodies to cells stably expressing human GPRC5D

1. Binding properties of anti-hGPRC5D chimeric antibodies to cells with stable high expression of human GPRC5D

A CHO-K1-hGPRC5D$^{hi}$ (with a GPRC5D expression level of about 2E+06 to 3E+06 antigens/cells) cell suspension at a cell concentration of $3 \times 10^5$ cells/mL was plated into a 96-well U-bottom plate at 100 µL/well. Serially diluted anti-hGPRC5D antibodies (in a final concentration range of 4.1-3000 ng/mL, serially 3-fold diluted) were added. The mixture was mixed well and then incubated at 4°C for 1 h. The cells were washed with PBS containing 2% (by volume) of FBS. A PE-conjugated goat anti-human IgG Fcy antibody (Jackson Immuno Research, Cat. No. 109-116-170) was added, and the mixture was incubated at 4°C for 1 h. The cells were washed with PBS containing 2% (by volume) of FBS and then resuspended. Fluorescence signals were detected on a flow cytometer (Sartorius, iQue3). The binding of the anti-hGPRC5D antibodies to CHO-K1-hGPRC5D$^{hi}$ cells was analyzed by the mean fluorescence intensity (MFI) of staining. The analysis result of $EC_{50}$ calculated is shown in FIG. 2. The chimeric antibodies Chi-89, Chi-105, Chi-128, Chi-131, Chi-164, Chi-169, and Chi-180 all exhibited binding activity in a concentration gradient-dependent manner on cells with high expression of hGPRC5D, and had higher maximum binding amounts to antigens than BM-mAb.

2. Binding properties of anti-hGPRC5D chimeric antibodies to cells with stable low expression of human GPRC5D

The plasmid pcDNA3.1-hGPRC5D was transfected into CHO-K1 cells according to the instructions for lipofectamine3000 transfection reagent (Thermo, Cat. No. L3000015) to give a cell line CHO-K1-hGPRC5D$^{low}$ with stable low expression (GPRC5D expression level of about 2000-2500 antigens/cells). A CHO-K1-hGPRC5D$^{low}$ cell suspension at a cell concentration of $3 \times 10^5$ cells/mL was plated into a 96-well U-bottom plate at 100 µL/well. Serially diluted anti-hGPRC5D antibodies (in a final concentration range of 4.1-3000 ng/mL, serially 3-fold diluted) were added. The mixture was mixed well and then incubated at 4°C for 1 h. The cells were washed with PBS containing 2% (by volume) of FBS. A PE-conjugated goat anti-human IgG Fcy antibody (Jackson Immuno Research, Cat. No. 109-116-170) was added, and the mixture was incubated at 4°C for 1 h. The cells were washed with PBS containing 2% (by volume) of FBS and then resuspended. Fluorescence signals were detected on a flow cytometer (Sartorius, iQue3). The binding of the anti-hGPRC5D antibodies to CHO-K1-hGPRC5D$^{low}$ cells was analyzed by the mean fluorescence intensity (MFI) of staining. As shown in FIG. 3, on the cells with low expression of hGPRC5D, the chimeric antibodies Chi-89, Chi-105, Chi-164, and Chi-180 showed comparable binding levels with BM-mAb, while Chi-128, Chi-131, and Chi-169 exhibited stronger target-binding ability.

Example 5: Binding activity **assay** for anti-hGPRC5D antibodies to tumor cells

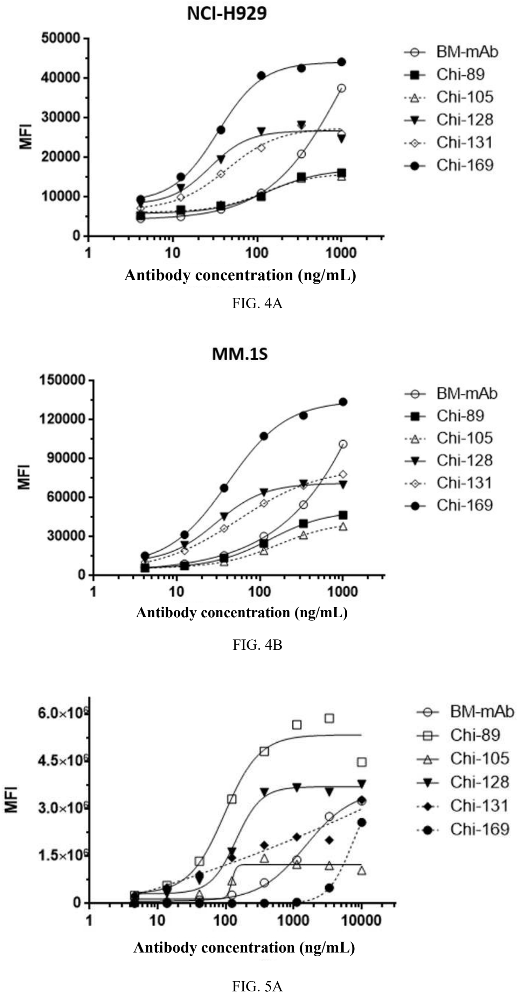

NCI-H929 and MM.1S human myeloma cell suspensions at a cell concentration of $3 \times 10^5$ cells/mL were separately plated into 96-well U-bottom plates at 100 µL/well. Serially diluted anti-hGPRC5D antibodies (in a final concentration range of 4.1-1000 ng/mL, serially 3-fold diluted) were added. The mixture was mixed well and then incubated at 4°C for 1 h. The cells were washed with PBS containing 2% (by volume) of FBS. A PE-conjugated goat anti-human IgG Fcy antibody (Jackson Immuno Research, Cat. No. 109-116-170) was added, and the mixture was incubated at 4°C for 1 h. The cells were washed with PBS containing 2% (by volume) of FBS and then resuspended. Fluorescence signals were detected on a flow cytometer (Sartorius, iQue3). The binding of the anti-hGPRC5D antibodies to human myeloma cells was analyzed by the mean fluorescence intensity (MFI) of staining. The chimeric antibodies Chi-89, Chi-105, Chi-128, Chi-131, and Chi-169 all exhibited binding activity in a concentration gradient-dependent manner on both NCI-H929 and MM.1S cells as shown in FIGs. 4A and 4B.

Example 6: Species cross-activity **assay** for anti-hGPRCSD antibodies

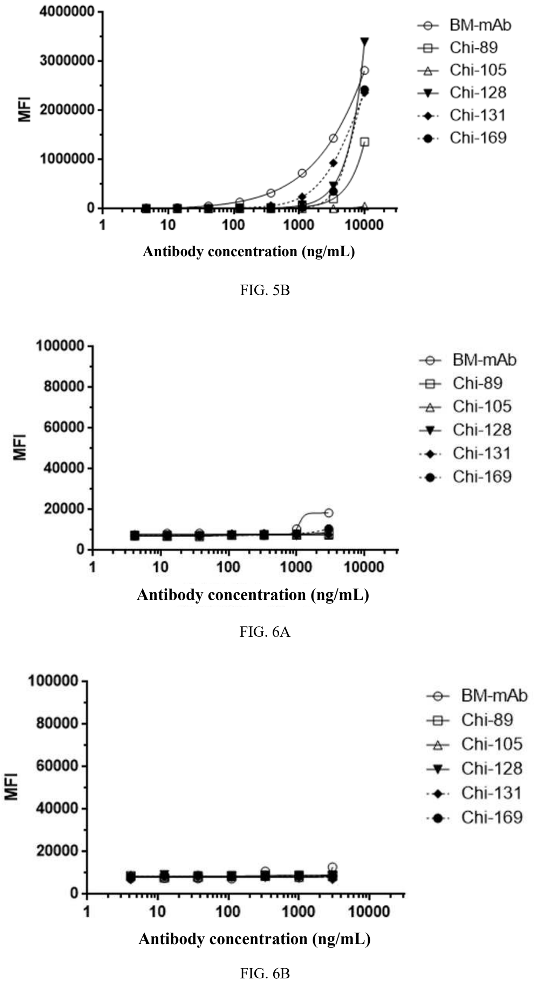

CHO-K1-cynoGPRC5D and CHO-K1-murineGPRC5D cell suspensions at a cell concentration of $5 \times 10^5$ cells/mL were separately plated into 96-well U-bottom plates at 100 µL/well. Serially diluted anti-hGPRC5D antibodies (in a final concentration range of 4.6-10000 ng/mL, serially 3-fold diluted) were added. The mixture was mixed well and then incubated at 4°C for 1 h. The cells were washed with PBS containing 2% (by volume) of FBS. A PE-conjugated goat anti-human IgG Fcy antibody (Jackson Immuno Research, Cat. No. 109-116-170) was added, and the mixture was incubated at 4°C for 1 h. The cells were washed with PBS containing 2% (by volume) of FBS and then resuspended. Fluorescence signals were detected on a flow cytometer (Sartorius, iQue3). The binding of the anti-hGPRC5D antibodies to CHO-K1-cynoGPRC5D and CHO-K1-murineGPRC5D cells was analyzed by the mean fluorescence intensity (MFI) of staining. As shown in FIGs. 5A and 5B, the chimeric antibodies Chi-89, Chi-128, Chi-131, and Chi-169 all exhibited binding activity in a concentration gradient-dependent manner on CHO-K1-cynoGPRC5D and CHO-K1-murineGPRCSD cells, indicating that these anti-hGPRC5D antibodies have cross-binding activity to monkey and mouse GPRC5D compared with BM-mAb, while chimeric antibody Chi-105 has cross-binding activity to monkey GPRC5D only.

## Example 7: Verification of specificity of anti-hGPRCSD antibodies

1. Verification of binding of anti-hGPRC5D chimeric antibodies to other family member proteins at cellular level

It is known that the GPRC family includes GPRC5A (RAIG1), GPRC5B (RAIG2), and GPRC5C (RAIG3) in addition to GPRC5D. cDNAs encoding full-length GPRC5A (SEQ ID NO: 46), GPRC5B (SEQ ID NO: 47), and GPRC5C (SEQ ID NO: 48) were obtained by gene synthesis, and then subcloned into a vector pcDNA3.1(+) to give pcDNA3.1-GPRC5A-GFP, pcDNA3.1-GPRC5B-GFP, and pcDNA3.1-GPRC5C-GFP recombinant plasmids. The DNAs of the recombinant plasmids described above were separately transfected into CHO-K1 cells according to the instructions for lipofectamin3000 transfection reagent (Thermo, Cat. No. L3000015) to give stable cell lines CHO-K1-GPRC5A, CHO-K1-GPRC5B, and CHO-K1-GPRC5C.

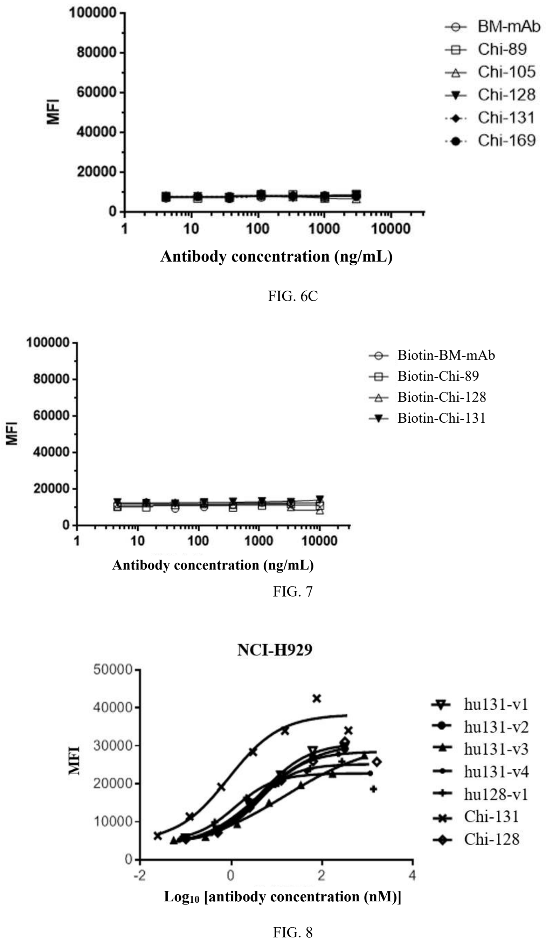

CHO-K1-GPRC5A, CHO-K1-GPRC5B, and CHO-K1-GPRC5C cell suspensions at a cell concentration of $5 \times 10^5$ cells/mL were separately plated into 96-well U-bottom plates at 100 $\mu$L/well. Serially diluted anti-hGPRC5D antibodies (in a final concentration range of 4.1-3000 ng/mL, serially 3-fold diluted) were added. The mixture was mixed well and then incubated at 4°C for 1 h. The cells were washed with PBS containing 2% (by volume) of FBS. An APC-conjugated goat anti-human IgG Fcγ antibody (Jackson Immuno Research, Cat. No. 109-135-098) was added, and the mixture was incubated at 4°C for 1 h. The cells were washed with PBS containing 2% (by volume) of FBS and then resuspended. Fluorescence signals were detected on a flow cytometer (Sartorius, iQue3). The binding of the anti-hGPRC5D antibodies to CHO-K1-GPRC5A, CHO-K1-GPRC5B, and CHO-K1-GPRC5C cells was analyzed by the mean fluorescence intensity (MFI) of staining. As shown in FIGs. 6A, 6B, and 6C, the chimeric antibodies Chi-89, Chi-105, Chi-128, Chi-131, and Chi-169 exhibited no binding activity to CHO-K1-GPRC5A, CHO-K1-GPRC5B, and CHO-K1-GPRC5C cells in the concentration range of 4.1-3000 ng/mL, and the BM-mAb exhibited non-specific binding to CHO-K1-GPRC5A cells at concentrations of greater than 1000 ng/mL.

2. Verification of binding of anti-hGPRC5D chimeric antibodies to CHO-K1 cells

Firstly, biotin-conjugated anti-hGPRC5D antibodies were prepared according to the instructions for EZ-linkNHS-Biotin reagent (Thermo, Cat. No. 20217), designated as Biotin-BM-mAb, Biotin-Chi-89, Biotin-Chi-128, and Biotin-Chi-131, respectively.

A CHO-K1 cell suspension at a cell concentration of $3 \times 10^5$ cells/mL was plated into a 96-well U-bottom plate at 100 $\mu$L/well. Serially diluted biotin-conjugated anti-hGPRC5D antibodies (serially 3-fold diluted, in a final concentration range of 4.6-10000 ng/mL,) were added. The mixture was mixed well and then incubated at 4°C for 1 h. The cells were washed with PBS containing 2% (by volume) of FBS. A PE-conjugated streptavidin (BD, Cat. No. 554061) was added, and the mixture was incubated at 4°C for 1 h. The cells were washed with PBS containing 2% (by volume) of FBS and then resuspended. Fluorescence signals were detected on a flow cytometer (Sartorius, iQue3). The mean fluorescence intensity (MFI) of staining was used to analyze whether the biotin-conjugated anti-hGPRC5D antibodies with amplified MFI signal exhibited non-specific binding to CHO-K1 cells. The results are shown in FIG. 7. The biotin-conjugated chimeric antibodies Chi-89, Chi-128, and Chi-131 all produced non-binding signals to CHO-K1 cells.

## Example 8: Construction, Expression and Purification of Anti-GPRCSD Humanized Antibodies

The anti-GPRC5D chimeric antibodies Chi-131 and Chi-128 were humanized separately. The chimeric antibody Chi-131 was humanized to obtain four humanized antibodies, which were named hu131-v1, hu131-v2, hu131-v3, and hu131-v4. The chimeric antibody Chi-128 was humanized to obtain one humanized antibody, which was named hu128-v1. The amino acid sequences and nucleotide sequences of the heavy chain and light chain of each humanized antibody are shown in Table 3.

Table 3. Sequence information of humanized antibodies (SEQ ID NO:)

| Clone No. | Heavy chain amino acid sequence | Light chain amino acid sequence | Heavy chain nucleotide sequence | Light chain nucleotide sequence |
|---|---|---|---|---|
| hu131-v1 | 70 | 78 | 71 | 79 |
| hu131-v2 | 72 | | 73 | |
| hu131-v3 | 74 | | 75 | |
| hu131-v4 | 76 | | 77 | |
| hu128-v1 | 80 | 82 | 81 | 83 |

Nucleotide sequences encoding the above humanized antibodies were synthesized and separately cloned into pcDNA3.1(+) expression vectors. Each antibody was expressed using an ExpiCHO expression kit (Thermo Fisher, Cat. No. A29133). The constructed expression vector comprising the nucleotide sequence described above was first transfected into ExpiCHO cells (CHO-S, Thermo), and then the cells were cultured in an ExpiCHO expression medium on an orbital shaker platform rotating at 130 rpm in a humidified atmosphere containing 8% $CO_2$ at 37°C. The culture supernatant was collected, and protein purification was performed using protein A magnetic beads (Genscript, Cat. No. L00273). The protein concentration was measured using a UV-Vis spectrophotometer (NanoDrop lite, Thermo Scientific).

Example 9: Assay for Binding Activity of Anti-GPRCSD Chimeric Antibodies and Humanized Antibodies

The binding activity of the chimeric antibodies Chi-131 and Chi-128 and the humanized antibodies thereof to human GPRC5D was determined by binding to NCI-H929 cells (source: Nanjing Cobioer Biosciences).

NCI-H929 human myeloma cell suspensions at a cell concentration of $1 \times 10^6$ cells/mL were plated into 96-well U-bottom plates at 100 μL/well. Serially diluted anti-GPRC5D antibodies (in a final concentration starting range of 310-1586 nM, serially 5-fold diluted) were added. The mixture was mixed well and then incubated at 4°C for 1 h. The cells were washed with pre-cooled running buffer (MACS, Cat. No. 130-091-221). A PE-conjugated goat anti-human IgG Fcγ antibody (Jackson Immuno Research, Cat. No. 109-116-170) was added, and the mixture was incubated at 4°C for 0.5 h. The cells were washed with pre-cooled running buffer and resuspended. Fluorescence signals were detected on a flow cytometer (Sartorius, iQue3). The binding of the anti-hGPRC5D antibodies to human myeloma cells was analyzed by the mean fluorescence intensity (MFI) of staining. The binding activity of the humanized antibodies (hu131-v1, hu131-v2, hu131-v3, hu131-v4, and hu128-v1) and the chimeric antibodies (Chi-131 and Chi-128) to the GPRC5D target antigen of NCI-H929 cells is shown in FIG. 8. The humanized antibodies (hu131-v1, hu131-v2, hu131-v3, hu131-v4, and hu128-v1) and the chimeric antibodies (Chi-131 and Chi-128) all exhibited binding activity in a concentration gradient-dependent manner on NCI-H929 cells.

Example 10: Construction, Expression and Purification of Anti-GPRC5D/Anti-CD3 Bispecific Antibody

(1) Anti-GPRC5D/Anti-CD3 Bispecific Antibodies GC35 and GC39

The anti-GPRC5D/anti-CD3 bispecific antibodies GC35 and GC39 were constructed according to the configuration shown in FIG. 9, wherein the first antigen-binding portion was a Fab binding to GPRC5D, and the second antigen-binding portion was an scFv binding to CD3. Specifically, GC35 and GC39 have 3 polypeptide chains. The amino acid sequences and nucleotide sequences of the 3 polypeptide chains are shown in Table 4-1.

Nucleotide sequences encoding anti-GPRC5D-HC, anti-GPRC5D-LC, and anti-CD3-scFv-Fc were synthesized and separately cloned into pcDNA3.1(+) expression vectors. The expression vector constructed above was co-transfected into ExpiCHO cells at a transfection density of $6 \times 10^6$ cells/mL using an ExpiCHO transfection kit (Thermo Fisher, Cat. No. A29133) according to the ratio of vector anti-GPRC5D-HC:vector anti-GPRC5D-LC:vector anti-CD3-scFv-Fc = 1:1.5:1.5. The culture was continued until day 10-15 after transfection, and the cell culture supernatant was collected by centrifugation. Protein purification was performed by Protein A affinity chromatography, ion exchange chromatography, and gel chromatography using the ÄKTA pure protein purification system (GE Healthcare). The protein concentration was measured using a UV-Vis spectrophotometer (NanoDrop lite, Thermo Scientific). The above bispecific antibody with the expected structure and sequence was confirmed to be obtained by electrophoresis (reduced SDS-PAGE), molecular weight (TOF MS), and sequence analysis.

(2) Benchmark talquetamab

An anti-GPRC5D/anti-CD3 bispecific antibody talquetamab was constructed as a benchmark (BM). Specifically, talquetamab has 4 polypeptide chains. The amino acid sequences are shown in Table 4-2. The nucleotide sequences encoding the polypeptide chains of talquetamab were synthesized and cloned into pcDNA3.1(+) expression vectors. Using an ExpiCHO transfection kit (Thermo Fisher, A29133), anti-GPRC5D-HC and anti-GPRC5D-LC of talquetamab were co-transfected into the first ExpiCHO at a ratio of vector anti-GPRC5D-HC:vector anti-GPRC5D-LC = 1:1.5 to express the anti-GPRC5D monoclonal antibody; anti-CD3-HC and anti-CD3-LC of talquetamab were co-transfected into the second ExpiCHO at a ratio of vector anti-CD3-HC:vector anti-CD3-LC = 1:1.5 to express the anti-CD3 monoclonal antibody. The transfection density of both ExpiCHO samples was $6 \times 10^6$ cells/mL. The culture was continued until day 10-15 after transfection, and the cell culture supernatant was collected by centrifugation.

The obtained supernatant was purified using ÄKTA pure protein purification system (GE Healthcare). The purified anti-GPRC5D monoclonal antibody and anti-CD3 monoclonal antibody were equimolarly assembled *in vitro* to obtain talquetamab. The specific procedures were as follows: The purified monoclonal antibody solution was replaced with PBS (pH 7.4) and concentrated. 2 mg of each monoclonal antibody was taken, and the total amount of the antibody was 4 mg (the molar ratio of the anti-GPRC5D monoclonal antibody to the anti-CD3 monoclonal antibody was about 1:1). PBS was supplemented to 0.9 mL. 6 mg of 2-mercaptoethylamine HCl (2-MEA, with a final concentration of 50 mM) was dissolved in 100 $\mu$L of PBS, which was quickly added to the prepared antibody solution described above to prepare a 1 mL reaction system. The system was well mixed, incubated at 37°C for 90 min, cooled to room temperature, and concentrated by ultrafiltration. 2-MEA was removed by exchange, and the mixture was stored in PBS. After oxidation overnight at 4°C, the antibody concentration was determined.

Table 4-1. Sequence information of anti-GPRC5D/anti-CD3 bispecific antibodies

| Antibody | Polypeptide chain | Amino acid sequence (SEQ ID NO:) | Nucleotide sequence (SEQ ID NO:) |
|---|---|---|---|
| GC35 | anti-GPRC5D-HC | 84 | 85 |
| | anti-GPRC5D-LC | 78 | 79 |
| | anti-CD3-scFv-Fc | 88 | 89 |
| GC39 | anti-GPRC5D-HC | 86 | 87 |
| | anti-GPRC5D-LC | 82 | 83 |
| | anti-CD3-scFv-Fc | 88 | 89 |

Table 4-2. Sequence information of benchmark

| Antibody | Polypeptide chain | Amino acid sequence (SEQ ID NO:) |
|---|---|---|
| Talquetamab | anti GPRC5D-HC | 90 |
| | anti- GPRC5D-LC | 91 |
| | anti-CD3-HC | 92 |
| | anti-CD3-LC | 93 |

**Example** 11: Binding of Anti-GPRCSD/Anti-CD3 Bispecific Antibodies to Multiple Myeloma Cells Expressing GPRC5D

The binding of the anti-GPRC5D/anti-CD3 bispecific antibody (including talquetamab, GC35, and GC39) to GPRC5D on RPMI-8226 cells (source: Cell Resource Center, Institute of Basic Medical Sciences, Chinese Academy of Medical Sciences), MM.1S cells (source: Beina Bio), and NCI-H929 cells (source: Nanjing Cobioer Biosciences) expressing GPRC5D was analyzed by flow cytometry, and the negative control was a hIgG1 monoclonal antibody (Biointron, Cat. No. B117901).

RPMI-8226 cells, MM.1S cells, and NCI-H929 cells in the logarithmic growth phase were taken, adjusted to a cell density of $1 \times 10^6$ - $2 \times 10^6$ cells/mL using RPMI-1640 medium (Hyclone, Cat. No. SH30809.01) containing 2% (v/v) FBS (fetal bovine serum), and seeded in a 96-well U-bottom cell culture plate (Costar, Cat. No. 3799) at 50 $\mu$L per well. Different concentrations of the anti-GPRC5D/anti-CD3 bispecific antibodies were prepared using the medium described above. The highest concentration of the antibody was 200 nM, and a total of 9 concentration gradients were obtained by 5-fold serial dilution. The antibodies at different concentrations were added to the 96-well cell culture plate described above at 50 $\mu$L per well, mixed well and incubated at 4°C for 1 h. The cells were washed with pre-cooled running buffer (MACS, Cat. No. 130-091-221), and then the supernatant was discarded. Pre-cooled PE-conjugated goat anti-human IgG Fc$\gamma$ antibody (Jackson Immuno Research, Cat. No. 109-116-170) was added, and the cells were resuspended at 100 $\mu$L/well. The mixture was mixed well and incubated at 4°C for 30 min. After washing, pre-cooled running buffer was added at 100 $\mu$L/well to resuspend the cells. The mixture was mixed well and then collected while flowing on a flow cytometer (Sartorius, iQue3), followed by data analysis.

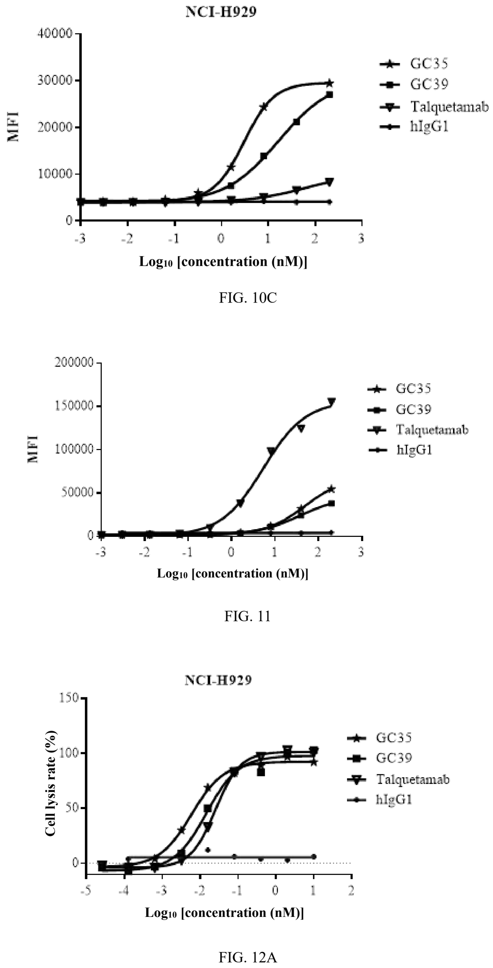

Table 5 and FIGs. 10A-10C show the binding ability of the anti-GPRC5D/anti-CD3 bispecific antibodies to RPMI-8226 cells, MM.1S cells, and NCI-H929 cells. The results show that the binding ability of GC35 and GC39 to target cells RPMI-8226, MM.1S, and NCI-H929 was stronger than that of talquetamab.

Table 5. The $EC_{50}$ values and maximum binding values for the binding of the anti-GPRC5D/anti-CD3 bispecific antibodies to target cells

| Antibody | RPMI-8226 | | MM.1S | | NCI-H929 | |
|---|---|---|---|---|---|---|
| | $EC_{50}$(nM) | Top(MFI) | $EC_{50}$(nM) | Top(MFI) | $EC_{50}$(nM) | Top(MFI) |
| GC35 | 15.88 | 53118 | 3.747 | 44844.5 | 3.035 | 29412 |
| GC39 | / | 28488 | 3.713 | 18235.5 | 16.69 | 27049 |
| Talquetamab | / | 8542 | 158.5 | 6890.5 | 61.26 | 8326 |
| hIgG1 | / | 3862.5 | / | 6770.5 | / | 4181 |
| "/" indicates that the curve could not be fitted to a value. | | | | | | |

Example 12: Binding of Anti-GPRC5D/Anti-CD3 Bispecific Antibodies to T Cells Expressing CD3

The binding of anti-GPRC5D/anti-CD3 bispecific antibodies (including: GC35, GC39, and talquetamab) to T cell surface CD3 was analyzed by flow cytometry. The negative control was a hIgG1 monoclonal antibody (Biointron, Cat. No. B117901).

Human PBMCs (peripheral blood mononuclear cells) were sorted for CD3+ T cells using CD3 magnetic beads (Miltenyi, Cat. No. 130-097-043). The cryopreserved PBMCs were thawed, and washed by centrifugation twice for counting. The running buffer (MACS, Cat. No. 130-091-221) was added at a ratio of 80 μL per $10^7$ cells, and the CD3 magnetic beads were added at a ratio of 20 μL CD3 magnetic beads per $10^7$ cells. The mixture was mixed well and incubated at 4°C for 15 min. The running buffer was added at a ratio of 1-2 mL per $10^7$ cells for washing the cells. The mixture was centrifuged, the supernatant was discarded, and 500 μL of running buffer was added to resuspend the cell pellet. The LS sorting column (Miltenyi, Cat. No. 130-042-401) was placed on the MidiMACS Starting Kit (LS) magnetic rack (Miltenyi, Cat. No. 130-091-051). After rinsing, the cell suspension was added and washed thrice. The LS sorting column was removed from the magnetic rack and placed on a clean centrifuge tube and 5 mL buffer was added to collect the sorted CD3+ T cells.

After counting, the CD3+ T cells were adjusted to a cell density of $1 \times 10^6$ - $2 \times 10^6$ cells/mL using running buffer, and seeded in a 96-well U-bottom cell culture plate (Costar, Cat. No. 3799) at 50 μL per well. Different concentrations of the anti-GPRC5D/anti-CD3 bispecific antibodies were prepared using running buffer. The highest concentration of the antibodies was 200 nM, and the antibodies were serially diluted 5-fold to obtain a total of 10 concentration gradients. The antibodies at different concentrations were added to the 96-well cell culture plate described above at 50 μL per well, mixed well and incubated at 4°C for 1 h. The cells were washed with pre-cooled running buffer (MACS, Cat. No. 130-091-221), and then the supernatant was discarded. Pre-cooled PE-conjugated goat anti-human IgG Fcγ antibody (Jackson Immuno Research, Cat. No. 109-116-170) was added, and the cells were resuspended at 100 μL/well. The mixture was mixed well and incubated at 4°C for 30 min. After washing, pre-cooled running buffer was added at 100 μL/well to resuspend the cells. The mixture was mixed well and then collected while flowing on a flow cytometer (Sartorius, iQue3), followed by data analysis.

Table 6 and FIG. 11 show the binding ability of the anti-GPRC5D/anti-CD3 bispecific antibodies to CD3+ T cells. The results show that GC35 and GC39 were able to bind to CD3+ T cells.

Table 6. The $EC_{50}$ values and maximum binding values for the binding of the anti-GPRC5D/anti-CD3 bispecific antibodies to CD3+ T cells

| Antibody | $EC_{50}$(nM) | Top(MFI) |
|---|---|---|
| GC35 | 48.86 | 54661 |
| GC39 | 37.86 | 37979 |
| Talquetamab | 5.347 | 155310.5 |

Example 13: **Killing of Multiple Myeloma Target Cells by Anti-GPRC5D/Anti-CD3 Bispecific Antibodies**

The killing effects of the anti-GPRC5D/anti-CD3 bispecific antibodies (including GC35, GC39, and talquetamab) on target cells NCI-H929 (source: Nanjing Cobioer Biosciences), MM.1S (source: Beina Bio), and RPMI-8226 (source: Cell Resource Center, Institute of Basic Medical Sciences, Chinese Academy of Medical Sciences) were studied using T cells provided by human PBMCs. The negative control was a hIgG1 monoclonal antibody (Biointron, Cat. No. B117901).

The target cells were adjusted to a cell density of $5 \times 10^5$ cells/mL using RPMI-1640 medium (Hyclone, Cat. No. SH30809.01) containing 2% (v/v) FBS and seeded in a 96-well cell culture plate (eppendorf, Cat. No. 0030730199) at 50 $\mu$L per well. Different concentrations of the anti-GPRC5D/anti-CD3 bispecific antibodies were prepared using the medium described above, with the highest concentration of the antibody being 10 nM. For NCI-H929 and MM.1S cells, the antibody was serially diluted 5-fold to obtain a total of 9 concentration gradients. For RPMI-8226 cells, the antibody was serially diluted 6-fold to obtain a total of 8 concentration gradients. The antibodies at different concentrations were added to the 96-well cell culture plate described above at 50 $\mu$L per well. The effector cells human PBMCs were adjusted to a cell density of $2.5 \times 10^6$ cells/mL using the medium described above and added at 100 $\mu$L per well. An administration group (50 $\mu$L of target cell + 100 $\mu$L of effector cell + 50 $\mu$L of antibody), a target cell group (50 $\mu$L of target cell + 150 $\mu$L of medium), an effector cell group (100 $\mu$L of human PBMC + 100 $\mu$L of medium), a target cell + effector cell group (50 $\mu$L of target cell + 100 $\mu$L of effector cell + 50 $\mu$L of medium), a blank control group (200 $\mu$L of medium), a lysis buffer control group (200 $\mu$L of medium + 20 $\mu$L of lysis buffer), and a target cell maximum release group (50 $\mu$L of target cell + 150 $\mu$L of medium + 20 $\mu$L of lysis buffer) were set, with the effector-to-target cell ratio being 10:1 and the co-incubation time being 24 h. The lysis buffer (Promega, Cat. No. G182A) in the target cell maximum release group and the lysis buffer control group was added 45 min before the assay. CytoTox96® non-radioactive cytotoxicity assay kit (Promega, G1780) was used for assay. Finally, the absorbance at a wavelength of 490 nm was measured in a microplate reader, and the cell lysis rate was calculated.

$$\text{Cell lysis rate}(\%) = \frac{\text{OD}_{\text{Administration group}} - \text{OD}_{\text{Target cell+Effector cell group}}}{\text{OD}_{\text{Target cell maximum release group}} - \text{OD}_{\text{Target cell group}}} \times 100\%$$

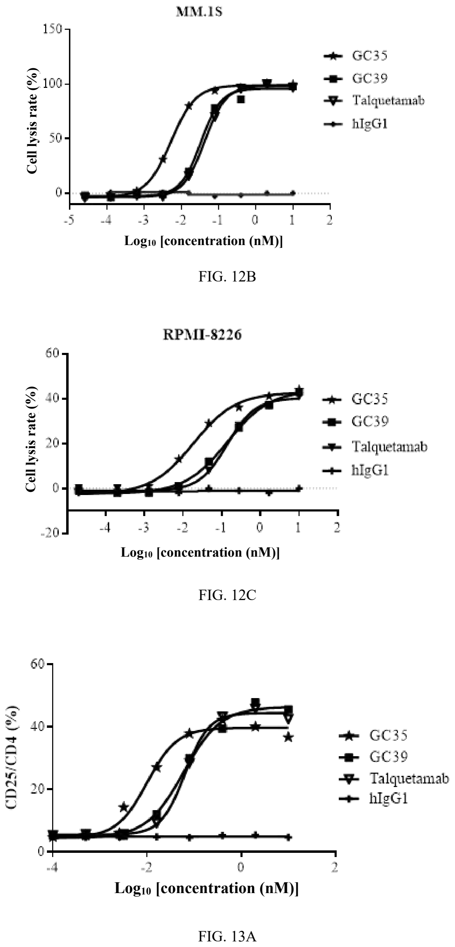

FIGs. 12A-12C and Table 7 show the lysis rate of each tumor cell by the anti-GPRC5D/anti-CD3 bispecific antibodies. The killing activity of the anti-GPRC5D/anti-CD3 bispecific antibody GC35 against NCI-H929, MM.1S, and RPMI-8226 was better than that of GC39 and talquetamab.

Table 7. Lysis $EC_{50}$ value and maximum lysis rate of each antibody to each tumor cell

| Antibody | NCI-H929 | | MM.1S | | RPMI-8226 | |
|---|---|---|---|---|---|---|
| | $EC_{50}$(nM) | Maximum lysis rate | $EC_{50}$(nM) | Maximum lysis rate | $EC_{50}$(nM) | Maximum lysis rate |
| GC35 | 0.005918 | 97% | 0.005335 | 101% | 0.01634 | 44% |
| GC39 | 0.01459 | 103% | 0.03352 | 101% | 0.1364 | 43% |
| Talquetamab | 0.02540 | 103% | 0.04261 | 100% | 0.1351 | 41% |

## Example 14: T Cell Activation Induced by Anti-GPRC5D/Anti-CD3 Bispecific Antibodies

The activation of T cells by the anti-GPRC5D/anti-CD3 bispecific antibodies (including GC35, GC39, and talquetamab) in the presence of myeloma target cells was studied by measuring the expression amounts of the T cell activation markers CD69 and CD25, and the ability to activate T cells was determined according to the degree of concentration-dependent upregulation of the markers. The negative control was a hIgG1 monoclonal antibody (Biointron, Cat. No. B117901).

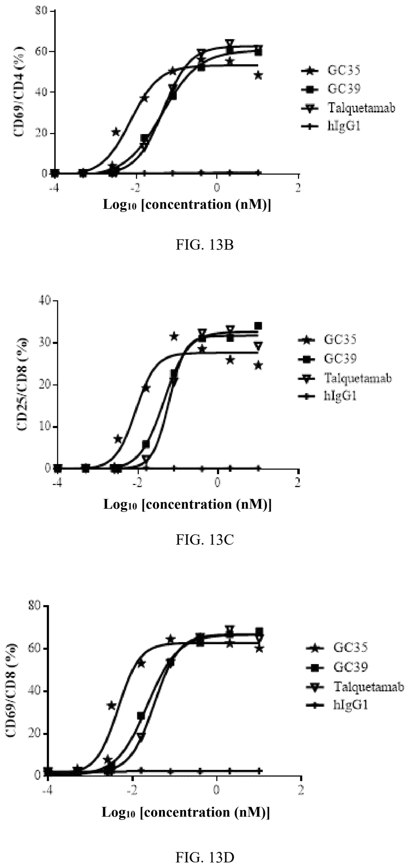

The target cells MM.1S (source: Beina Bio) were adjusted to a cell density of $5 \times 10^5$ cells/mL using RPMI-1640 medium (Hyclone, Cat. No. SH30809.01) containing 2% (v/v) FBS and seeded in a 96-well cell culture plate (eppendorf, Cat. No. 0030730199) at 50 $\mu$L per well. Different concentrations of the anti-GPRC5D/anti-CD3 bispecific antibodies were prepared using the medium described above. The highest concentration of the antibodies was 10 nM, and the antibodies were serially diluted 5-fold to obtain a total of 8 concentration gradients. The antibodies at different concentrations were added to the 96-well cell culture plate described above at 50 $\mu$L per well. The effector cells human PBMCs were adjusted to a cell density of $2.5 \times 10^6$ cells/mL using medium for experiment and added at 100 $\mu$L per well. An administration group (50 $\mu$L of target cell + 100 $\mu$L of effector cell + 50 $\mu$L of antibody), a target cell group (50 $\mu$L of target cell + 150 $\mu$L of medium), an effector cell group (100 $\mu$L of human PBMC + 100 $\mu$L of medium), a target cell + effector cell group (50 $\mu$L of target cell + 100 $\mu$L of effector cell + 50 $\mu$L of medium), and a blank control group (200 $\mu$L of medium) were set, with the effector-to-target cell ratio being 10:1 and the co-incubation time being 24 h. The mixture was centrifuged and the supernatant was removed. The cells were centrifuged and washed with pre-cooled running buffer (MACS, Cat. No. 130-091-221). The supernatant was removed, 100 $\mu$L of the running buffer was added per well, and the cells were mixed well. CD4-BV421 (BD, Cat. No. 564713) and CD8-PE-Cy7 (BD, Cat. No. 557746), with each being 2.5 $\mu$L, and CD25-APC (BD, Cat. No. 555434), and CD69-FITC (BD, Cat. No. 555530), with each being 10 $\mu$L, were added per well, and the mixture was incubated on ice for 30 min. The cells were washed with pre-cooled running buffer, 100 $\mu$L/well of running buffer was added to resuspend the cells, and the cells were mixed well and detected using a flow cytometer. FIGs. 13A-13D and Table 8 show the activation degree of T cells in the presence of MM.1S target cells, wherein CD25/CD4 denotes the proportion of the number of CD25$^+$ cells among the number of CD4$^+$ cells, CD69/CD4 denotes the proportion of the number of CD69$^+$ cells among the number of CD4$^+$ cells, CD25/CD8 denotes CD25/CD8 denotes the proportion of the number of CD25$^+$ cells among the number of CD8$^+$ cells, and CD69/CD8 denotes the proportion of the number of CD69$^+$ cells among the number of CD8$^+$ cells. The expression of CD25 and CD69 was significantly increased relative to the negative control, indicating that T cells were activated; GC35 and GC39 can both effectively activate T cells.

Table 8. The EC$_{50}$ values for the activation of T cells by the anti-GPRC5D/anti-CD3 bispecific antibodies in the presence of MM.1 S

| Antibody | CD25/CD4 EC$_{50}$(nM) | CD69/CD4 EC$_{50}$(nM) | CD25/CD8 EC$_{50}$(nM) | CD69/CD8 EC$_{50}$(nM) |
|---|---|---|---|---|
| GC35 | 0.009988 | 0.007382 | 0.008789 | 0.004582 |
| GC39 | 0.05958 | 0.04376 | 0.04479 | 0.02258 |
| Talquetamab | 0.06372 | 0.04596 | 0.0592 | 0.0329 |

## Example 15: Cytokine Release Induced by Anti-GPRCSD/Anti-CD3 Bispecific Antibodies

Cytokine release induced by the anti-GPRC5D/anti-CD3 bispecific antibodies (including GC35 and talquetamab) in the presence of target cells was studied by measuring the content of IL-2, IL-6, TNF-$\alpha$, and IFN-$\gamma$. The safety of the bispecific antibodies was assessed according to the amount of cytokine released.

The target cells NCI-H929 (source: Nanjing Cobioer Biosciences) were adjusted to a cell density of $5 \times 10^5$ cells/mL using RPMI-1640 medium (Hyclone, Cat. No. SH30809.01) containing 2% (v/v) FBS and seeded in a 96-well cell culture plate (eppendorf, Cat. No. 0030730199) at 50 $\mu$L per well. Different concentrations of the anti-GPRC5D/anti-CD3 bispecific antibodies were prepared using the medium described above. The highest concentration of the antibodies was 10 nM, and the antibodies were serially diluted 5-fold to obtain a total of 9 concentration gradients. The antibodies at different concentrations were added to the 96-well cell culture plate described above at 50 $\mu$L per well. The effector cells human PBMCs were adjusted to a cell density of $2.5 \times 10^6$ cells/mL using medium for experiment and added at 100 $\mu$L per well. An administration group (50 $\mu$L of target cell + 100 $\mu$L of effector cell + 50 $\mu$L of antibody), a target cell group (50 $\mu$L of target cell + 150 $\mu$L of medium), an effector cell group (100 $\mu$L of human PBMC + 100 $\mu$L of medium), a target cell + effector cell group (50 $\mu$L of target cell + 100 $\mu$L of effector cell + 50 $\mu$L of medium), a effector cell + antibody group (100 $\mu$L of effector cell + 50 $\mu$L of antibody + 50 $\mu$L of medium), and a blank control group (200 $\mu$L of medium) were set, with the effector-to-target cell ratio being 10:1 and the co-incubation time being 24 h. The mixture was centrifuged, and the supernatant was collected. The samples were treated using assay kits Human IL-2 Precoated ELISA Kit (Dayou, Cat. No. 1110203), Human IL-6 Precoated ELISA Kit (Dayou, Cat. No. 1110603), Human TNF-$\alpha$ Precoated ELISA Kit (Dayou, Cat. No. 1117203), and Human IFN-$\gamma$ Precoated ELISA Kit (Dayou, Cat. No. 1110003) according to the kit instructions, and assayed and analyzed using a microplate reader (Infinite F50, model: TECAN).

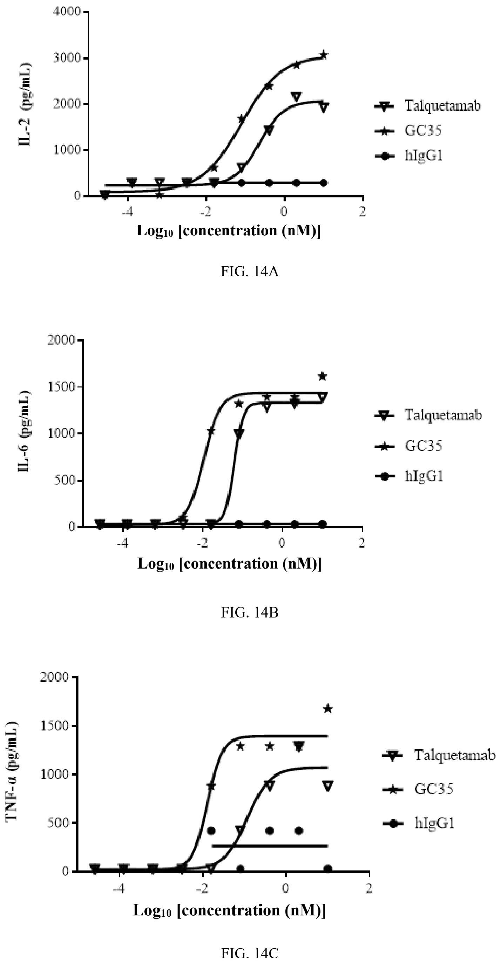

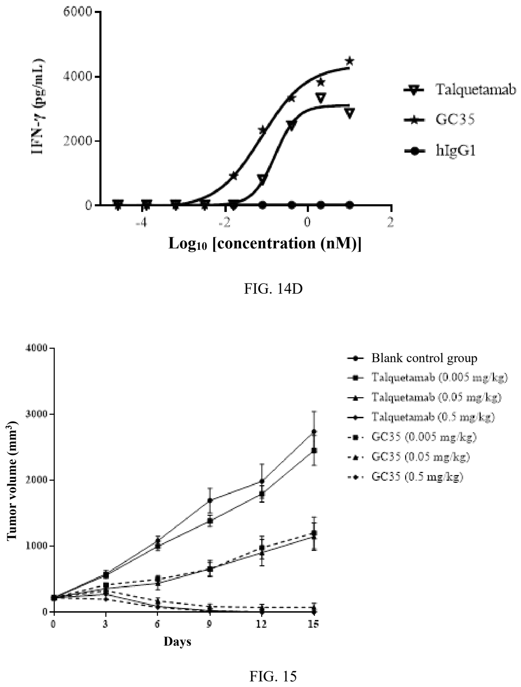

FIGs. 14A-14D and Table 9 show GC35-induced cytokine release in the presence of NCI-H929 cells.

Table 9. The EC$_{50}$ of cytokine release amount and maximum release amount

| Antibody | IL-2 | | IL-6 | | TNF-$\alpha$ | | IFN-y | |
|---|---|---|---|---|---|---|---|---|
| | EC$_{50}$(nM) | Maximum release amount (pg/mL) | EC$_{50}$(nM) | Maximum release amount (pg/mL) | EC$_{50}$(nM) | Maximum release amount (pg/mL) | EC$_{50}$(nM) | Maximum release amount (pg/mL) |
| GC35 | 0.0796 | 3071.27 | 0.0106 | 1616.66 | 0.01301 | 1675.21 | 0.07554 | 4481.89 |
| Talquetamab | 0.2277 | 2161.13 | 0.0609 | 1394.03 | 0.1158 | 1292.61 | 0.1593 | 3342.25 |

## Example 16: Growth Inhibition of NCI-H929 by Anti-GPRC5D/Anti-CD3 Bispecific Antibodies in Mouse Tumor Model

The tumor inhibitory effect of GC35 in the NCI-H929 human multiple myeloma mouse xenograft tumor model was determined. NCI-H929 human myeloma cells were mixed with human PBMCs and suspended to give a suspension of human PBMCs ($0.5 \times 10^7$ cells/mL) and NCI-H929 cells ($7.5 \times 10^7$ cells/mL). Under sterile conditions, the suspension was inoculated at the right-side armpit of B-NDG mice (source: Biocytogen) in a volume of 0.1 mL/mouse. The diameter of the xenograft tumor was measured using a vernier caliper. When the tumors grew to about 200 mm$^3$, the animals were randomly divided into 7 groups with 6 animals in each, and the day of grouping was D0. The treatment groups were dosed via tail vein injection (i.v.) at a volume of 10 mL/kg, and the blank control group was dosed with an equal volume of PBS solution on D1, D4, D7, and D10. A total of 4 administrations were performed. The anti-tumor effect of the antibody was dynamically monitored by measuring the tumor diameter. The tumor volume was measured 2-3 times a week, and meanwhile, the mice were weighed, and the data were recorded. The general behavior of the mice was observed and recorded daily.

The indices were calculated using the following formulas:

$$\text{Tumor volume TV (mm}^3) = 1/2 \times (a \times b^2),$$

wherein a represents the long diameter of the tumor, and b represents the short diameter of the tumor;

$$\text{relative tumor volume RTV} = TV_t/TV_0,$$

wherein $TV_0$ is the tumor volume at D0, and $TV_1$ is the tumor volume at each measurement;

$$\text{relative tumor proliferation rate T/C (\%)} = (T_{RTV}/C_{RTV}) \times 100\%,$$

wherein $T_{RTV}$ is RTV of treatment group, and $C_{RTV}$ is RTV of control group;

tumor growth inhibition rate TGI (%) = (1 - tumor weight of treatment group/tumor weight of control group) $\times$ 100%.

The experimental results are shown in FIG. 15 and Table 10. At high doses, talquetamab and GC35 could significantly inhibit the growth of human multiple myeloma NCI-H929 subcutaneous xenograft tumors, resulting in tumor regression; at medium and low doses, GC35 had better drug effect than that of talquetamab.

Table 10. Effect of anti-GPRC5D/anti-CD3 bispecific antibodies on NCI-H929 human myeloma cell NDG mouse subcutaneous xenograft tumor

| Group | Dose (mg/kg) | Number of animals | | Tumor shrinkage/Tumor regression/Total tumor amount | D15 1-T/C | D15 TGI |
|---|---|---|---|---|---|---|
| | | D0 | D15 | | | |
| Talquetamab | 0.005 | 6 | 6 | 0/0/6 | 9.4% | 12.6% |
| Talquetamab | 0.05 | 6 | 6 | 0/0/6 | 60.2% | 57.3% |
| Talquetamab | 0.5 | 6 | 6 | 6/6/6 | 100.0% | 100.0% |
| GC35 | 0.005 | 6 | 6 | 0/0/6 | 55.5% | 45.8% |
| GC35 | 0.05 | 6 | 6 | 5/4/6 | 96.9% | 95.4% |
| GC35 | 0.5 | 6 | 6 | 6/6/6 | 100.0% | 100.0% |

If the tumor volume was smaller than that at the time of grouping, i.e., $TV_t < TV_0$, it was considered as "tumor shrinkage"; "tumor regression" was complete tumor regression, with TGI (%) = 100%.

The sequence information of the present disclosure is summarized in Table S2 below.

Table S2. Sequence information

| Description<br>Sequence (SEQ ID NO:) |
|---|
| HCDR1 of Chi-131 and its humanized antibodies<br>NYVMH (SEQ ID NO: 1) |
| HCDR2 of Chi-131, hu131-v1, and hu131-v2<br>YFNPYNDGTNYNEKFKG (SEQ ID NO: 2) |
| HCDR3 of Chi-131 and its humanized antibodies<br>GGVRRYFDV (SEQ ID NO: 3) |

(continued)

| Description Sequence (SEQ ID NO:) |
|---|
| LCDR1 of Chi-131 and its humanized antibodies RASQDIGSNLN (SEQ ID NO: 4) |
| LCDR2 of Chi-131 and its humanized antibodies ATFSLDS (SEQ ID NO: 5) |
| LCDR3 of Chi-131 and its humanized antibodies QQYANFPPT (SEQ ID NO: 6) |
| VH of Chi-131 |
| EVQLQQSGPELVKPGASVKMSCKASGYTFTNYVMHWVKQKPGQGLEWIGYFNPYNDGTNYNEKFKGKATLTSDK SSNTAYMELSSLTSEDSAVYYCARGGVRRYFDVWGAGTTVTVSS (SEQ ID NO: 7) |
| VL of Chi-131 DIQMTQSPSSLSASLGERVSLTCRASQDIGSNLNWLQLGPDGTIKRLIYATFSLDSGVPKRFSGSRSGSDYSLTISSLESE DFVDYYCQQYANFPPTFGGGTKLEIK (SEQ ID NO: 8) |
| HCDR1 of Chi-169 GFTFSNYGMS (SEQ ID NO: 9) |
| HCDR2 of Chi-169 SISSGGGRIYYPDNVKG (SEQ ID NO: 10) |
| HCDR3 of Chi-169 HAMDN (SEQ ID NO: 11) |
| LCDR1 of Chi-169 SASSSVSNMY (SEQ ID NO: 12) |
| LCDR2 of Chi-169 DTSKLAS (SEQ ID NO: 13) |
| LCDR3 of Chi-169 QQWSSNPLT (SEQ ID NO: 14) |
| VH of Chi-169 EVKLVESGGGLVKPGASLKLSCAASGFTFSNYGMSWVRQTSDKRLEWVASISSGGGRIYYPDNVKGRFTISRENAKN TLYLQMSSLKSEDTALYYCTRHAMDNWGQGTSVIVSS (SEQ ID NO: 15) |
| VL of Chi-169 QIVLTQSPAIMSASPGEKVTMTCSASSSVSNMYWYQQKSGTSPKRWIYDTSKLASGVPARFSGSGSGTSYSLTISSME AEDASTYYCQQWSSNPLTFGAGTTLELK (SEQ ID NO: 16) |
| HCDR1 of Chi-89 GFTFSSYGMS (SEQ ID NO: 17) |
| HCDR2 of Chi-89 TISSGGSYTYYPDSVKG (SEQ ID NO: 18) |
| HCDR3 of Chi-89 QALRYHMDS (SEQ ID NO: 19) |
| LCDR1 of Chi-89 KASQNVGTNVA (SEQ ID NO: 20) |
| LCDR2 of Chi-89 SASYRYS (SEQ ID NO: 21) |
| LCDR3 of Chi-89 QQYYSYPWT (SEQ ID NO: 22) |
| VH of Chi-89 |

(continued)

| Description Sequence (SEQ ID NO:) |
|---|
| EVQLVESGGDLVKPGGSLKLSCAASGFTFSSYGMSWVRQTPDKRLEWVATISSGGSYTYYPDSVKGRFTFSRDNAKN TLYLQMSSLKSEDTAMYYCVRQALRYHMDSWGQGTSVTVSS (SEQ ID NO: 23) |
| VL of Chi-89 DIVMTQSQKFMSTSVGDRVSVTCKASQNVGTNVAWYQQKPGQSPKALIYSASYRYSGVPDRFTGSGSGTDFTLTISN VQSEDLADFFCQQYYSYPWTFGGGTKLEIK (SEQ ID NO: 24) |
| HCDR1 of Chi-105 GYTFTDYYMN (SEQ ID NO: 25) |
| HCDR2 of Chi-105 YIYPNNGGTGYNQRFKG (SEQ ID NO: 26) |
| HCDR3 of Chi-105 WGGTRLGYYAMDY (SEQ ID NO: 27) |
| LCDR1 of Chi-105 KASQNVGTNVV (SEQ ID NO: 28) |
| LCDR2 of Chi-105 WASTRHT (SEQ ID NO: 29) |
| LCDR3 of Chi-105 QQYSRYPYT (SEQ ID NO: 30) |
| VH of Chi-105 EVQLQQSGPELVKPGASVKMSCKASGYTFTDYYMNWVKQSHGKSLEWIGYIYPNNGGTGYNQRFKGKATLTADKS SSTAYMELRSLTSEDSAVYYCARWGGTRLGYYAMDYWGQGTSVTVSS (SEQ ID NO: 31) |
| VL of Chi-105 DIVMTQSQKFMSTSVGDRVSVTCKASQNVGTNVVWYQQKPGQSPKLLIYWASTRHTGVPDRFTGSGSGTDFTLTIS NVQSEDLADYFCQQYSRYPYTFGGGTKLEIK (SEQ ID NO: 32) |
| HCDRI of Chi-128 and hu128-v1 SFGMH (SEQ ID NO: 33) |
| HCDR2 of Chi-128 and hu128-v1 YISRGSSTIYYADTVKG (SEQ ID NO: 34) |
| HCDR3 of Chi-128 and hu128-v1 SGYGSSSYYFDY (SEQ ID NO: 35) |
| LCDR1 of Chi-128 and hu128-v1 RASSSIRSSYLH (SEQ ID NO: 36) |
| LCDR2 of Chi-128 and hu128-v1 STSNLAS (SEQ ID NO: 37) |
| LCDR3 of Chi-128 and hu128-v1 QQFSGYPLT (SEQ ID NO: 38) |
| VH of Chi-128 DVQLVESGGGLVQPGGSRKVSCAASGFTFSSFGMHWVRQAPEKGLEWVAYISRGSSTIYYADTVKGRFTISRDNPKN TLFLQMTSLRSEDTAMYYCARSGYGSSSYYFDYWGQGTTLTVSS (SEQ ID NO: 39) |
| VL of Chi-128 ENVLTQSPAIMSASPREKVTMTCRASSSIRSSYLHWYQQKSGASPKLWIYSTSNLASGVPARFSGSGSGTSYSLTISSVE AEDAATYYCQQFSGYPLTFGGGTKLEIK (SEQ ID NO: 40) |

(continued)

| Description Sequence (SEQ ID NO:) |
|---|
| Human GPRC5D<br><br>MYKDCIESTGDYFLLCDAEGPWGIILESLAILGIVVTILLLLAFLFLMRKIQDCSQWNVLPTQLLFLLSVLGLFGLAFA FIIELNQQTAPVRYFLFGVLFALCFSCLLAHASNLVKLVRGCVSFSWTTILCIAIGCSLLQIIATEYVTLIMTRGMMFVN MTPCQLNVDFVVLLVYVLFLMALTFFVSKATFCGPCENWKQHGRLIFITVLFSIIIWVVWISMLLRGNPQFQRQPQW DDPVVCIALVTNAWVFLLLYIVPELCILYRSCRQECPLQGNACPVTAYQHSFQVENQELSRARDSDGAEEDVALTSYG TPIQPQTVDPTQECFIPQAKLSPQQDAGGV (SEQ ID NO: 41) |
| Monkey GPRC5D<br><br>MYKDCIESTGDYFLPCDSEGPWGIILESLAILGIVVTILLLLAFLFLMRKIQDCSQWNVLPTQLLFLLSVLGLFGLAFAF IIQLNQQTAPVRYFLFGVLFALCFSCLLAHASNLVKLVRGRVSFSWTTILCIAIGCSLLQVIIAIEYVTLIMTRGMMFVH MTPYQLNVDFVVLLVYVLFLMALTFFVSKATFCGPCENWKQHGRLIFITVLFSIIIWVVWISMLLRGNPQFQRQPQW DDPVVCIALVTNAWVFLLLYIVPELCILYRSCRQECPSQGHACPVTAYQRSFQVENQELSRARDSDGAEEDVALTSFG TPIQPQTVDPTQECFIPRAKLSPQQDAGV (SEQ ID NO: 42) |
| Mouse GPRC5D<br><br>MYEDCVKSTEDYYLFCDNEGPWAIVLESLAVIGIVVTILLLLAFLFLMRKVQDCSQWNVLPTQFLFLLAVLGLFGLTF AFIIQLNHQTAPVRYFLFGVLFAICFSCLLAHASNLVKLVRGRVSFCWTTILFIAIGVSLLQTIIAIEYVTLIMTRGLMFE<br><br>HMTPYQLNVDFVCLLIYVLFLMALTFFVSKATFCGPCENWKQHGRLIFATVLVSIIIWVVWISMLLRGNPQLQRQPH WDDAVICIGLVTNAWVFLLIYIIPELSILYRSCRQECPTQGNVCQVPVYQRSFRMDTQEPTRARDSDGAQEDVALTAY GTPIQLQSADPSREYLIPSATLSPQQDAGL (SEQ ID NO: 43) |
| VH of GC5B596<br><br>QVQLVQSGAEVKKPGASVKVSCKASGYSFTGYTMNWVRQAPGQGLEWMGLINPYNSDTNYAQKLQGRVTMTTDT STSTAYMELRSLRSDDTAVYYCARVALRVALDYWGQGTLVTVSS (SEQ ID NO: 44) |
| VL of GC5B596<br><br>DIQMTQSPSSLSASVGDRVTITCKASQNVATHVGWYQQKPGKAPKRLIYSASYRYSGVPSRFSGSGSGTEFTLTISNLQ PEDFATYYCQQYNRYPYTFGQGTKLEIK (SEQ ID NO: 45) |
| Human GPRC5A<br><br>MATTVPDGCRNGLKSKYYRLCDKAEAWGIVLETVATAGVVTSVAFMLTLPILVCKVQDSNRRKMLPTQFLFLLGVL GIFGLTFAFIIGLDGSTGPTRFFLFGILFSICFSCLLAHAVSLTKLVRGRKPLSLLVILGLAVGFSLVQDVIAIEYIVLTMNR TNVNVFSELSAPRRNEDFVLLLTYVLFLMALTFLMSSFTFCGSFTGWKRHGAHIYLTMLLSIAIWVAWITLLMLPDFD RRWDDTILSSALAANGWVFLLAYVSPEFWLLTKQRNPMDYPVEDAFCKPQLVKKSYGVENRAYSQEEITQGFEETG DTLYAPYSTHFQLQNQPPQKEFSIPRAHAWPSPYKDYEVKKEGS (SEQ ID NO: 46) |
| Human GPRC5B<br><br>MFVASERKMRAHQVLTFLLLFVITSVASENASTSRGCGLDLLPQYVSLCDLDAIWGIVVEAVAGAGALITLLLMLILL VRLPFIKEKEKKSPVGLHFLFLLGTLGLFGLTFAFIIQEDETICSVRRFLWGVLFALCFSCLLSQAWRVRRLVRHGTGPA GWQLVGLALCLMLVQVIIAVEWLVLTVLRDTRPACAYEPMDFVMALIYDMVLLVVTLGLALFTLCGKFKRWKLNG AFLLITAFLSVLIWVAWMTMYLFGNVKLQQGDAWNDPTLAITLAASGWVFVIFHAIPEIHCTLLPALQENTPNYFDTS QPRMRETAFEEDVQLPRAYMENKAFSMDEHNAALRTAGFPNGSLGKRPSGSLGKRPSAPFRSNVYQPTEMAVVLNG GTIPTAPPSHTGRHLW (SEQ ID NO: 47) |
| Human GPRC5C |

(continued)

| Description<br>Sequence (SEQ ID NO:) |
|---|
| MAIHKALVMCLGLPLFLFPGAWAQGHVPPGCSQGLNPLYYNLCDRSGAWGIVLEAVAGAGIVTTFVLTIILVASLPFV QDTKKRSLLGTQVFFLLGTLGLFCLVFACVVKPDFSTCASRRFLFGVLFAICFSCLAAHVFALNFLARKNHGPRGWVI FTVALLLTLVEVIINTEWLIITLVRGSGEGGPQGNSSAGWAVASPCAIANMDFVMALIYVMLLLLGAFLGAWPALCGR YKRWRKHGVFVLLTTATSVAIWVVWIVMYTYGNKQHNSPTWDDPTLAIALAANAWAFVLFYVIPEVSQVTKSSPEQ SYQGDMYPTRGVGYETILKEQKGQSMFVENKAFSMDEPVAAKRPVSPYSGYNGQLLTSVYQPTEMALMHKVPSEG AYDIILPRATANSQVMGSANSTLRAEDMYSAQSHQAATPPKDGKNSQVFRNPYVWD (SEQ ID NO: 48) |
| Heavy chain of Chi-131<br>EVQLQQSGPELVKPGASVKMSCKASGYTFTNYVMHWVKQKPGQGLEWIGYFNPYNDGTNYNEKFKGKATLTSDK SSNTAYMELSSLTSEDSAVYYCARGGVRRYFDVWGAGTTVTVSSASTKGPSVFPLAPSSKSTSGGTAALGCLVKDYF PEPVTVSWNSGALTSGVHTFPAVLQSSGLYSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSRDELTKNQVSLTCLVKGFYPSDIAVEWES NGQPENNYKTTPPVLDSDGSFFLYSKLTVDKSRWQQGNVFSCSVMHEALHNHYTQKSLSLSPGK (SEQ ID NO: 49) |
| Light chain of Chi-131<br>DIQMTQSPSSLSASLGERVSLTCRASQDIGSNLNWLQLGPDGTIKRLIYATFSLDSGVPKRFSGSRSGSDYSLTISSLESE DFVDYYCQQYANFPPTFGGGTKLEIKRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNRGEC (SEQ ID NO: 50) |
| Heavy chain of Chi-169<br>EVKLVESGGGLVKPGASLKLSCAASGFTFSNYGMSWVRQTSDKRLEWVASISSGGGRIYYPDNVKGRFTISRENAKN TLYLQMSSLKSEDTALYYCTRHAMDNWGQGTSVIVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTKNQVSLTCLVKGFYPSDIAVEWESNGQPEN |
| NYKTTPPVLDSDGSFFLYSKLTVDKSRWQQGNVFSCSVMHEALHNHYTQKSLSLSPGK (SEQ ID NO: 51) |
| Light chain of Chi-169<br>QIVLTQSPAIMSASPGEKVTMTCSASSSVSNMYWYQQKSGTSPKRWIYDTSKLASGVPARFSGSGSGTSYSLTISSME AEDASTYYCQQWSSNPLTFGAGTTLELKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVTKSFNRGEC (SEQ ID NO: 52) |
| Heavy chain of Chi-89<br>EVQLVESGGDLVKPGGSLKLSCAASGFTFSSYGMSWVRQTPDKRLEWVATISSGGSYTYYPDSVKGRFTFSRDNAKN TLYLQMSSLKSEDTAMYYCVRQALRYHMDSWGQGTSVTVSSASTKGPSVFPLAPSSKSTSGGTAALGCLVKDYFPE PVTVSWNSGALTSGVHTFPAVLQSSGLYSLSSVVTVPSSSLGTQTYICNVNHKPSNTKVDKKVEPKSCDKTHTCPPCP APELLGGPSVFLFPPKPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPSRDELTKNQVSLTCLVKGFYPSDIAVEWESN GQPENNYKTTPPVLDSDGSFFLYSKLTVDKSRWQQGNVFSCSVMHEALHNHYTQKSLSLSPGK (SEQ ID NO: 53) |
| Light chain of Chi-89<br>DIVMTQSQKFMSTSVGDRVSVTCKASQNVGTNVAWYQQKPGQSPKALIYSASYRYSGVPDRFTGSGSGTDFTLTISN VQSEDLADFFCQQYYSYPWTFGGGTKLEIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPVTKSFNRGEC (SEQ ID NO: 54) |

(continued)

| Description<br>Sequence (SEQ ID NO:) |
|---|
| Heavy chain of Chi-105<br>EVQLQQSGPELVKPGASVKMSCKASGYTFTDYYMNWVKQSHGKSLEWIGYIYPNNGGTGYNQRFKGKATLTADKS SSTAYMELRSLTSEDSAVYYCARWGGTRLGYYAMDYWGQGTSVTVSSASTKGPSVFPLAPSSKSTSGGTAALGCLV KDYFPEPVTVSWNSGALTSGVHTFPAVLQSSGLYSLSSVVTVPSSSLGTQTYICNVNHKPSNTKVDKKVEPKSCDKTH TCPPCPAPELLGGPSVFLFPPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPSRDELTKNQVSLTCLVKGFYPSDIAV EWESNGQPENNYKTTPPVLDSDGSFFLYSKLTVDKSRWQQGNVFSCSVMHEALHNHYTQKSLSLSPGK (SEQ ID NO: 55) |
| Light chain of Chi-105<br>DIVMTQSQKFMSTSVGDRVSVTCKASQNVGTNVVWYQQKPGQSPKLLIYWASTRHTGVPDRFTGSGSGTDFTLTIS NVQSEDLADYFCQQYSRYPYTFGGGTKLEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSPVTKSFNRGEC (SEQ ID NO: 56) |
| Heavy chain of Chi-128<br>DVQLVESGGGLVQPGGSRKVSCAASGFTFSSFGMHWVRQAPEKGLEWVAYISRGSSTIYYADTVKGRFTISRDNPKN TLFLQMTSLRSEDTAMYYCARSGYGSSSYYFDYWGQGTTLTVSSASTKGPSVFPLAPSSKSTSGGTAALGCLVKDYF PEPVTVSWNSGALTSGVHTFPAVLQSSGLYSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSRDELTKNQVSLTCLVKGFYPSDIAVEWES NGQPENNYKTTPPVLDSDGSFFLYSKLTVDKSRWQQGNVFSCSVMHEALHNHYTQKSLSLSPGK (SEQ ID NO: 57) |
| Light chain of Chi-128<br>ENVLTQSPAIMSASPREKVTMTCRASSSIRSSYLHWYQQKSGASPKLWIYSTSNLASGVPARFSGSGSGTSYSLTISSVE AEDAATYYCQQFSGYPLTFGGGTKLEIKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVTKSFNRGEC (SEQ ID NO: 58) |
| HCDR2 of Chi-131 and its humanized antibodies<br>YFNPYNX$_1$X$_2$TNYNEKFKG (SEQ ID NO: 59, wherein X$_1$ is selected from D and E, and X$_2$ is selected from G and A) |
| HCDR2 of hu131-v3<br>YFNPYNDATNYNEKFKG (SEQ ID NO: 60) |
| HCDR2 of hu131-v4<br>YFNPYNEGTNYNEKFKG (SEQ ID NO: 61) |
| VH of hu131-v1<br>EVQLVQSGAEVVKPGASVKMSCKASGYTFTNYVMHWVKQAPGQGLEWMGYFNPYNDGTNYNEKFKGRVTLTSD TSANTAYMELSSLRSEDTAVYYCARGGVRRYFDVWGQGTTVTVSS (SEQ ID NO: 62) |
| VH of hu131-v2<br>EVQLVQSGAEVVKPGASVKMSCKASGYTFTNYVMHWVKQAPGQGLEWIGYFNPYNDGTNYNEKFKGRATLTSDT SANTAYMELSSLRSEDTAVYYCARGGVRRYFDVWGQGTTVTVSS (SEQ ID NO: 63) |
| VH of hu131-v3<br>EVQLVQSGAEVVKPGASVKMSCKASGYTFTNYVMHWVKQAPGQGLEWIGYFNPYNDATNYNEKFKGRATLTSDT SANTAYMELSSLRSEDTAVYYCARGGVRRYFDVWGQGTTVTVSS (SEQ ID NO: 64) |
| VH of hu131-v4 |

| Description |
| --- |
| Sequence (SEQ ID NO:) |
| EVQLVQSGAEVVKPGASVKMSCKASGYTFTNYVMHWVKQAPGQGLEWIGYFNPYNEGTNYNEKFKGRATLTSDT SANTAYMELSSLRSEDTAVYYCARGGVRRYFDVWGQGTTVTVSS (SEQ ID NO: 65) |
| VH of Chi-131 and its humanized antibodies<br><br>EVQLX$_3$QSGX$_4$EX$_5$VKPGASVKMSCKASGYTFTNYVMHWVKQX$_6$PGQGLEWX$_7$GYFNPYNX$_8$X$_9$TNYNEKFKGX$_{10}$X$_{11}$TLTSDX$_{12}$SX$_{13}$NTAYMELSSLX$_{14}$SEDX$_{15}$AVYYCARGGVRRYFDVWGX$_{16}$GTTVTVSS (SEQ ID NO: 66, wherein X$_3$ is selected from Q and V, X$_4$ is selected from P and A, X$_5$ is selected from L and V, X$_6$ is selected from K and A, X$_7$ is selected from I and M, X$_8$ is selected from D and E, X$_9$ is selected from G and A, X$_{10}$ is selected from K and R, X$_{11}$ is selected from A and V, X$_{12}$ is selected from K and T, X$_{13}$ is selected from S and A, X$_{14}$ is selected from T and R, X$_{15}$ is selected from S and T, and X$_{16}$ is selected from A and Q) |
| VL of hu131-v1, hu131-v2, hu131-v3, and hu131-v4<br><br>DIQMTQSPSSLSASVGDRVTITCRASQDIGSNLNWLQLGPGGAIKRLIYATFSLDSGVPSRFSGSRSGSDYTLTISSLQPE DFVDYYCQQYANFPPTFGGGTKVEIK (SEQ ID NO: 67) |
| VH of hu128-v1<br><br>EVQLVESGGGLVQPGGSRRVSCAASGFTFSSFGMHWVRQAPGKGLEWVAYISRGSSTIYYADTVKGRFTISRDNAKN SLYLQMNSLRAEDTAVYYCARSGYGSSSYYFDYWGQGTTVTVSS (SEQ ID NO: 68) |
| VL of hu128-v1<br><br>EIVLTQSPATLSASPGERATMSCRASSSIRSSYLHWYQQKPGQSPRLWIYSTSNLASGVPARFSGSGSGTDYTLTISSLEP EDAAVYYCQQFSGYPLTFGGGTKVEIK (SEQ ID NO: 69) |
| Heavy chain of hu131-v1<br><br>EVQLVQSGAEVVKPGASVKMSCKASGYTFTNYVMHWVKQAPGQGLEWMGYFNPYNDGTNYNEKFKGRVTLTSD TSANTAYMELSSLRSEDTAVYYCARGGVRRYFDVWGQGTTVTVSSASTKGPSVFPLAPSSKSTSGGTAALGCLVKDY FPEPVTVSWNSGALTSGVHTFPAVLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHEALHNHYTQKSLSLSPGK (SEQ ID NO: 70)<br><br>gaagtgcagctggtgcagtctggcgccgaggtggtgaaacctggcgcctctgtcaagatgtcctgcaaggcctccggctacacattcaccaactatgtgatgcactgggtcaagcagg ctcccgggcagggactggaatggatgggctacttcaacccttacaacgacggcaccaattataacgagaagttcaagggcagagtgaccctgacatctgatacctccgccaacaccg cctacatggaactgtccagcctgagatccgaggacaccgccgtgtactactgcgccagaggcggagtgcggagatacttcgacgtgtggggccaaggcaccaccgtgaccgtgtcc tctgccagcaccaagggcccatccgtgtttcctctggcctccttcctccaaatctaccagcggaggcaccgctgctttgggatgcctggtgaaggactactttccagagcccgtgaccgtg tcttggaacagcggcgccctgacctctggcgtgcataccttccctgctgttctgcagtccagcggcctgtactctctgtcctctgtggtgacagtgccctctagctctctgggcacccagac ctacatctgcaacgtgaaccacaagccttctaataccaaagtggacaagaaggtggaacccaagtcttgcgacaagacccacacctgtcctccttgccctgctcctgaactgctgggcg gcccttctgtgtttctgttccccccaaagcctaaggatacactgatgatctctcggacacctgaggtcacatgtgtggtcgtggatgtgtctcacgaggatcctgaagtgaagttcaactggt acgtggatggcgtggaagtgcacaacgccaagaccaagcctagagaggaacagtacaactccacttaccgggtagttagcgtgctgaccgtgctgcaccaggactggctgaacggc aaagagtacaagtgcaaggtgtccaacaaggctctgcctgctcctatcgagaaaaccatctcgaaggccaagggccagcctcgcgagcctcaggtgtacaccctgcccccccagccg ggacgagctgaccaagaaccaggtcagcctgacctgtctggtcaaaggcttctacccttccgacatcgccgtggagtgggagtccaacggtcaacctgagaacaactacaagacaac ccctcctgtgctcgactccgacggctccttcttcctgtattccaagctgaccgtggacaagtccagatggcagcagggcaacgtgttctcctgctccgtgatgcacgaggccctgcataat |
| cactacacccagaagtccctctccctgtctccaggcaag (SEQ ID NO: 71) |
| Heavy chain of hu131-v2 |

| Description Sequence (SEQ ID NO:) |
|---|
| EVQLVQSGAEVVKPGASVKMSCKASGYTFTNYVMHWVKQAPGQGLEWIGYFNPYNDGTNYNEKFKGRATLTSDT SANTAYMELSSLRSEDTAVYYCARGGVRRYFDVWGQGTTVTVSSASTKGPSVFPLAPSSKSTSGGTAALGCLVKDYF PEPVTVSWNSGALTSGVHTFPAVLQSSGLYSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSRDELTKNQVSLTCLVKGFYPSDIAVEWES NGQPENNYKTTPPVLDSDGSFFLYSKLTVDKSRWQQGNVFSCSVMHEALHNHYTQKSLSLSPGK (SEQ ID NO: 72) gaggtgcagctggtgcagagcggagccgaggtcgtgaaacctggcgcctctgtgaagatgtcgtgcaaggcttctggctacaccttcacgaactatgtgatgcactgggtcaagcag gctcctggccaaggactcgaatggatcggctattttaatccttacaacgacggtaccaactacaacgagaagttcaagggcagagctaccctgacctccgatacatctgccaacacagc ctacatggaactcagctccttgcgctctgaggataccgctgtttactactgcgccagaggcggggtccggcggtacttcgacgtgtggggccagggcaccaccgtgacggtttcttctg cctccacaaaggggccctccgtgttccccctggccccatcctccaaatccacatccggcggaaccgctgctctgggctgcctggtgaaggactactttcctgagcctgtgacagtgtctt ggaactctggcgccctgacctctggcgtgcatactttccctgccgtgctgcagtcttctggcctgtactccctgtcctctgttgtgaccgtgccaagctcctctctgggcacccagacctac atctgcaacgtgaaccacaagcctagcaacaccaaagtggacaagaaggtggaacccaagtcctgcgacaagacccacacctgtcctccttgtcccgcacccgagctgctgggcgg cccttctgtgtttctgttccctcccaagcctaaggacaccctgatgatcagcagaaccccgaagtgacctgcgtagtggtggatgtgtctcacgaagaccctgaagtgaagttcaactgg tacgtggatggcgtcgaggtgcacaacgccaagaccaaacctcgggaagagcagtacaacagcacatatagagtggtctccgtgctgacagtgctgcatcaggactggctgaacgg caaggagtacaagtgcaaggtgtccaacaaggccctgcctgctcctatcgagaagaccatctccaaggccaaaggccagcctagagagcccaagtgtacacctgcctccatctcg ggacgagctgaccaagaaccaggtgtccctgacctgtctggtgaagggcttctaccctagcgacatcgccgtcgaatgggagtctaatggacagcctgagaacaactacaagaccac ccctcctgtgctggactccgacggctccttcttcctgtactccaagctgaccgtggataagagtagatggcagcagggcaacgtgttctcctgctccgtgatgcacgaggctctgcacaa tcactacacccagaagtccctgtccctgagcccaggaaaa (SEQ ID NO: 73) |
| Heavy chain of hu131-v3 EVQLVQSGAEVVKPGASVKMSCKASGYTFTNYVMHWVKQAPGQGLEWIGYFNPYNDATNYNEKFKGRATLTSDT SANTAYMELSSLRSEDTAVYYCARGGVRRYFDVWGQGTTVTVSSASTKGPSVFPLAPSSKSTSGGTAALGCLVKDYF PEPVTVSWNSGALTSGVHTFPAVLQSSGLYSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVYTLPPSRDELTKNQVSLTCLVKGFYPSDIAVEWES NGQPENNYKTTPPVLDSDGSFFLYSKLTVDKSRWQQGNVFSCSVMHEALHNHYTQKSLSLSPGK (SEQ ID NO: 74) gaagtgcagctggtgcagtctggtgccgaggtggtcaaacccggcgcctccgtgaagatgtcctgcaaggcttctggatataccttcaccaactatgtgatgcactgggtgaagcagg cccctggccagggcctggaatggatcggctactttaatccttacaacgacgccaccaactacaacgagaagttcaagggcagagctacgctgacctccgacaccagcgccaacacc gcctacatggaactgtccagcctgcggtccgaggataccgctgtgtactactgcgccagaggcggagtgcggagatacttcgacgtgtggggccaaggcaccaccgtgaccgtgtc ctctgcctctaccaagggaccttctgtcttccctctggctccatcttctaagtccaccagcggcggcacagctgctctgggctgcctggtgaaagactacttccctgaacccgtgaccgtct cttggaactctggcgctctgacatctggagttcataccttcctgccgtgctgcagtcctccggcctgtactccctgtcttccgtcgtgacagtgcccagcagcagcctgggcacccagac ctacatctgcaacgtgaaccacaagccttccaacaccaaagtggacaagaaggtggaacctaagtcttgcgacaagacccacacctgtcctccttgtcctgctcctgagctgctgggag gccctagcgtgtttctgttcccccccaagcctaaggacaccctgatgatctcccggacacctgaagtgacctgcgtggtggtggatgtgtctcacgaggatcccgaggtgaagttcaact ggtacgtggacggcgtggaagtgcacaacgccaagaccaagccccgcgaggaacagtacaactcgacatacagagtggtgtctgtgctcacagtactgcaccaggactggctgaa cggcaaagagtacaagtgcaaggtctccaacaaggccctgcctgctcctatcgagaaaaccatctccaaggccaaaggccagcccagagagcccaagtgtacacactgcctccaa gccgggacgagctgaccaagaaccaggtgtccctgacctgtctggtcaagggcttctatccttctgacatcgccgtggagtgggagtccaatggccagcctgagaacaactacaaga caaccccctcctgtgctggactccgatggctccttcttcctctactcaaagctgaccgtggataagtccagatggcagcagggcaacgtgttctcctgctccgtgatgcacgaggccctgc ataatcactacacccagaagtctctgtccctctcctggcaag (SEQ ID NO: 75) |
| Heavy chain of hu131-v4 |

| Description<br>Sequence (SEQ ID NO:) |
| --- |
| EVQLVQSGAEVVKPGASVKMSCKASGYTFTNYVMHWVKQAPGQGLEWIGYFNPYNEGTNYNEKFKGRATLTSDT<br>SANTAYMELSSLRSEDTAVYYCARGGVRRYFDVWGQGTTVTVSSASTKGPSVFPLAPSSKSTSGGTAALGCLVKDYF<br>PEPVTVSWNSGALTSGVHTFPAVLQSSGLYSLSSVVTVPSSSLGTQTYICNVNHKPSNTKVDKKVEPKSCDKTHTCPP<br>CPAPELLGGPSVFLFPPKPKDTLMISRTPEVTCVVVDVSHEDPEVKFNWYVDGVEVHNAKTKPREEQYNSTYRVVSV<br>LTVLHQDWLNGKEYKCKVSNKALPAPIEKTISKAKGQPREPQVYTLPPSRDELTKNQVSLTCLVKGFYPSDIAVEWES<br>NGQPENNYKTTPPVLDSDGSFFLYSKLTVDKSRWQQGNVFSCSVMHEALHNHYTQKSLSLSPGK (SEQ ID NO: 76) |
| gaggtgcagctggtgcagtccggcgccgaggttgtgaagcctggagcctccgtgaagatgtcctgcaaggcctccggctacaccttcaccaactacgtgatgcactgggtgaagcag<br>gcccctggccaaggcctggaatggatcggctatttcaacccttacaacgagggcaccaactataatgaaaagttcaagggcagagccaccctgacctccgacacatctgccaacacc<br>gcctacatggaactgtcctccctgagatctgaggatacagccgtgtactactgcgccagaggcggcgtgcggcggtacttcgacgtgtgggggccagggcacaaccgtgaccgtgtctt<br>ctgcttctaccaagggcccttccgtgttccctctggctccctcttccaagtccacctccggcggcaccgctgctctcggctgtctggtgaaggattactttcctgaacccgtgacggtgtcct<br>ggaacagcggcgctctgacctctggtgtgcatacatttccagctgtgctgcagtcctctggactgtactctttgtcttctgtggtcaccgtgcctagcagctctctgggcacccagacctac<br>atctgcaacgtgaaccacaagccttccaacaccaaagtggacaagaaggtggagcccaaatcttgcgacaaaacccacacctgtcctccttgccctgcacctgagctgctgggcgga<br>ccaagcgtctttctgttcccacctaagcccaaggataccctgatgatctcccggaccccagaagtgacctgtgtggtggtggatgtgtctcacgaggaccctgaggtcaagttcaactgg<br>tacgtggacggagtggaagtgcacaacgccaagaccaagcccagagaggaacagtacaactccacctacagagtggtgtccgttctgaccgtgctgcaccaggactggctgaacg<br>gtaaagagtacaagtgcaaggtgtccaacaaggcccctgcctgctcctatcgagaagaccatctctaaggctaagggccagcctcgcgagcctcaagtatacacactgcctccctctcg<br>ggacgagctgaccaagaaccaggtgtccctgacctgcctggtcaaaggcttctacccctccgacatcgccgtcgaatgggagagtaatggacagcccgagaacaactacaagacca<br>cccctcctgtgctggactccgacggcagcttcttcctgtactccaagctgacagtggataaatctagatggcagcagggcaacgtgttctcctgcagcgtgatgcacgaggccctgcac<br>aatcattacacccagaagagcttgtccctgtctcctggcaag (SEQ ID NO: 77) |
| Light chain of hu131-v1, hu131-v2, hu131-v3, and hu131-v4, and anti-GPRC5D-LC of GC35<br>DIQMTQSPSSLSASVGDRVTITCRASQDIGSNLNWLQLGPGGAIKRLIYATFSLDSGVPSRFSGSRSGSDYTLTISSLQPE<br>DFVDYYCQQYANFPPTFGGGTKVEIKRTVAAPSVFIFPPSDEQLKSGTASVVCLLNNFYPREAKVQWKVDNALQSGN<br>SQESVTEQDSKDSTYSLSSTLTLSKADYEKHKVYACEVTHQGLSSPVTKSFNRGEC (SEQ ID NO: 78) |
| gatatccagatgacccagtcccctccagtctgtctgcttccgtgggcgacagagtgaccatcacctgtcgcgccagccaggacatcggctccaacctgaactggctgcagctgggcc<br>ctggcggcgctatcaagcggctgatctatgctacatttagcctggactccggcgttccttccagattctccggctctcggtccggatctgactacaccctgacgatctccagcctgcagcc<br>tgaggacttcgtggattactactgccagcagtacgccaattttcctcctaccttcggcggaggcaccaaagtggaaatcaaaagaaccgtggctgctcctagcgtgttcatcttccccca<br>tccgacgagcagctgaagtctggaacagcctctgtcgtgtgcctgctgaacaacttctacccccgggaagccaaggtgcagtggaaggtggacaacgccctgcagtctggcaactcc<br>caagagtccgtcaccgagcaagattctaaggactctacctactctctcagctctaccctgacactgtccaaggccgactacgagaagcacaaggtgtacgcctgcgaagtgacccacc<br>agggcctgtcctctcctgtgaccaagtccttcaacagaggcgagtgc (SEQ ID NO: 79) |
| Heavy chain of hu128-v1<br>EVQLVESGGGLVQPGGSRRVSCAASGFTFSSFGMHWVRQAPGKGLEWVAYISRGSSTIYYADTVKGRFTISRDNAKN<br>SLYLQMNSLRAEDTAVYYCARSGYGSSSYYFDYWGQGTTVTVSSASTKGPSVFPLAPSSKSTSGGTAALGCLVKDYF<br>PEPVTVSWNSGALTSGVHTFPAVLQSSGLYSLSSVVTVPSSSLGTQTYICNVNHKPSNTKVDKKVEPKSCDKTHTCPP<br>CPAPELLGGPSVFLFPPKPKDTLMISRTPEVTCVVVDVSHEDPEVKFNWYVDGVEVHNAKTKPREEQYNSTYRVVSV<br>LTVLHQDWLNGKEYKCKVSNKALPAPIEKTISKAKGQPREPQVYTLPPSRDELTKNQVSLTCLVKGFYPSDIAVEWES<br>NGQPENNYKTTPPVLDSDGSFFLYSKLTVDKSRWQQGNVFSCSVMHEALHNHYTQKSLSLSPGK (SEQ ID NO: 80) |

(continued)

| Description<br>Sequence (SEQ ID NO:) |
|---|
| gaggtgcagctggtcgagtccggcggtggcctcgtgcagcctggcggctctcggcgcgtgtcttgtgctgcttctggattcaccttcagctctttcggcatgcactgggtccggcaggc cccaggcaagggcctggaatgggtggcctacatctctagaggctctagcaccatctactacgccgacaccgtgaaaggcagattcaccatctccagagacaacgccaagaactccct gtacctgcagatgaactccctgagagccgaggataccgccgtgtactactgcgccagatccggctacggcagctcctcctactatttcgattactggggccagggcaccaccgtgacc gtttcctccgcctctaccaagggacctagcgtgttccctctggcccccctcttctaagtccacatctggcggaaccgctgctctcggctgcctggtgaaggactactttccagagcctgtga ccgtgtcctggaactctggcgctctgacctccggcgtgcataccttcctgctgtgctgcaatcttctggcctgtactccctgtcttccgtggtgacagtgccttctagtagcctgggcaccc agacctacatctgcaacgtgaaccacaagccctctaacaccaaggtggacaagaaagtggaacccaagagctgcgacaagacccacacctgccctccttgtcctgctcctgaactgct gggcggccccttctgtgtttctgttccccccaaagcctaaggatacactgatgatctctcggacccctgaagtgacctgcgtggtggtggacgtgtctcacgaggaccctgaggtgaagtt caactggtacgtggatggcgtggaagtgcacaacgccaagacaaaacctcgggaagagcagtacaattccacctacagagtggtctccgtgctgactgtgctgcatcaggactggct gaacggcaaagagtacaagtgcaaggtgtccaacaaggccctgcccgctcctatcgagaagaccatctccaaggctaagggccaacctagagaacctcaggtgtacaccctgcctc cttcccgggacgagctgaccaagaaccaggttagcctgacctgtctggtgaagggcttctacccttccgacatcgccgtggagtgggagtctaatggccagcctgagaacaactacaa gaccacacccccgtgctggacagcgacggcagcttcttcctgtattccaagctgacagtcgataaatccagatggcagcagggcaacgtgttcctgctccgtgatgcacgaggcc ctgcacaatcactacacccagaagtctttgtctctgtccccaggaaaa (SEQ ID NO: 81) |
| Light chain of hu128-v1, and anti-GPRC5D-LC of GC39<br>EIVLTQSPATLSASPGERATMSCRASSSIRSSYLHWYQQKPGQSPRLWIYSTSNLASGVPARFSGSGSGTDYTLTISSLEP EDAAVYYCQQFSGYPLTFGGGTKVEIKRTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNRGEC (SEQ ID NO: 82)<br>gagatcgtgctgacccaaagcccagctaccctgagcgccagccccggcgagagagctaccatgtcctgcagagcctccagctctatccggtcctcctacctgcactggtaccagcag |
| aagcctggccagtcccctcggctgtggatctattccaccagcaatctcgcctctggcgtgcctgctaggttttccggctctggatcgggaaccgactacacctgaccatctcctctctgg aacccgaggatgccgccgtgtactactgccagcagttctccggctaccctctgacattcggcggaggcaccaaagtggaaatcaagagaaccgtggctgctcttctgtgttcatcttcc ctccttccgacgagcagctgaagtctggcaccgcttctgtcgtgtgcctgctgaacaacttctacccccagagaggccaaggtgcagtggaaggtggacaacgctctgcagtctggcaa ctcccaagagtctgtcacagaacaggactccaaagattctacctactctctgtcttccacactgacactgtccaaggccgactacgagaagcacaaggtttacgcctgcgaggtgaccc accagggcctgtcctctcctgtgaccaagtccttcaaccggggcgaatgt (SEQ ID NO: 83) |
| Anti-GPRC5D-HC of GC35<br>EVQLVQSGAEVVKPGASVKMSCKASGYTFTNYVMHWVKQAPGQGLEWMGYFNPYNDGTNYNEKFKGRVTLTSD TSANTAYMELSSLRSEDTAVYYCARGGVRRYFDVWGQGTTVTVSSASTKGPSVFPLAPSSKSTSGGTAALGCLVKDY FPEPVTVSWNSGALTSGVHTFPAVLQSSGLYSLSSVVTVPSSSLGTQTYICNVNHKPSNTKVDKKVEPKSCDKTHTCP PCPAPEAAGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALAAPIEKTISKAKGQPREPQVCTLPPSREEMTKNQVSLSCAVKGFYPSDIAVEW ESNGQPENNYKTTPPVLDSDGSFFLVSKLTVDKSRWQQGNVFSCSVMHEALHNHYTQKSLSLSPGK (SEQ ID NO: 84) |

| Description |
| --- |
| Sequence (SEQ ID NO:) |

gaagtgcagctggtgcagtccggcgccgaggttgtgaagcctggagccagcgtcaagatgtcctgcaaggcttctggctacaccttcaccaattatgtgatgcactgggtgaagcagg

cccccggccaaggcctggaatggatgggctacttcaacccttacaacgacggcaccaactacaacgagaagttcaagggcagagtgacactgacctccgacaccagcgccaacac

agcctacatggaactgtcctctctgcggtccgaggataccgccgtgtactactgcgcccggggcggcgtgcggagatacttcgacgtgtggggccaaggaaccaccgtgaccgtctc

ctctgcctctactaagggcccttctgtgttccctctggctccttcttccaagtctacctccggcgcaccgctgcccctgggctgcctggtcaaggactacttccccgaacctgtgacagtgt

cttggaactctggcgctctgacatctggtgtgcataccttcctgccgtgctgcagtcttctggactgtactccctgtctagcgtggtgaccgtgcccagcagctccctgggaacccagac

ctacatctgcaacgtgaaccacaagccatccaacaccaaagtggacaagaaggtcgagcccaaatcttgcgacaagacccacacctgtcctccttgtcctgctcctgaggctgctggtg

gaccttccgtgtttctgttcccacctaaacccaaggacaccctgatgatcagcagaaccccctgaagtgacctgcgtggtggtggatgtgtctcacgaggacccccgaggtgaagttcaact

ggtacgtggatggcgtggaagtgcacaacgctaagaccaagcctagagaggaacagtacaactccacctatagagtcgtgtctgtgctgaccgtgctgcaccaggactggctgaacg

gcaaagagtacaagtgcaaggtgtccaacaaggccctggccgctcccatcgagaaaaccatctccaaagccaagggccagcctcgggagcctcaggtgtgcacgctgcctccatct

cgcgaagagatgaccaagaaccaggtcagcctgagctgtgctgtgaaaaggcttttacccctccgacatcgccgtggagtgggagagcaatggccagcctgagaacaactacaagac

cacacctcctgtgctggactccgacggctccttcttcctggtgtccaagctgaccgtcgataagtccagatggcagcagggcaacgtgttctcctgctctgtgatgcacgaggccctgca

taatcactacacacagaagtccctctccctctccaggcaag (SEQ ID NO: 85)

**Anti-GPRC5D-HC of GC39**

EVQLVESGGGLVQPGGSRRVSCAASGFTFSSFGMHWVRQAPGKGLEWVAYISRGSSTIYYADTVKGRFTISRDNAKN

SLYLQMNSLRAEDTAVYYCARSGYGSSSYYFDYWGQGTTVTVSSASTKGPSVFPLAPSSKSTSGGTAALGCLVKDYF

PEPVTVSWNSGALTSGVHTFPAVLQSSGLYSLSSVVTVPSSSLGTQTYICNVNHKPSNTKVDKKVEPKSCDKTHTCPP

CPAPEAAGGPSVFLFPPKPKDTLMISRTPEVTCVVVDVSHEDPEVKFNWYVDGVEVHNAKTKPREEQYNSTYRVVS

VLTVLHQDWLNGKEYKCKVSNKALAAPIEKTISKAKGQPREPQVCTLPPSREEMTKNQVSLSCAVKGFYPSDIAVEW

ESNGQPENNYKTTPPVLDSDGSFFLVSKLTVDKSRWQQGNVFSCSVMHEALHNHYTQKSLSLSPGK (SEQ ID NO:

86)

gaggtgcagctggtcgaatctggaggcggcctggtgcagcccggcggctctagacgggtgtcttgtgccgcttccggcttcacctttagcagcttcggcatgcactgggtgcggcagg

cccctggcaagggcctcgagtgggtcgcctacatcagccggggatcttctaccatctactacgccgataccgtgaagggccagattcaccatctcccgggacaacgccaagaatagcct

gtacctgcagatgaactccctgagagccgaggacaccgctgtgtactactgcgccagatctggctacggctcttcttcttactatttcgactactggggccaaggcacaaccgtgaccgtt

tcttccgcctctaccaaaggaccttccgtgtttcctctggctccttcctccaaatccacatctggggggtacagctgccctcggctgcttggtgaaggactacttccccgagcccgtgaccgt

gtcctggaactctggcgctctgacctctggagtgcataccttcctgctgtgctgcagtcctccggcctgtactccctgtcctccgtggtgacagtgccttcctctagcctgggcactcaga

cctatatctgcaacgtgaaccacaaaccttccaacaccaaggtggataagaaggtggaacccaagtcctgcgacaaaacccacacctgtcctccttgccctgccccagaggctgctgg

cggaccctctgtctttctgttcccacctaagcctaaggacaccctgatgatctctcggacccctgaagtgacctgcgtggtcgtggacgtgtctcacgaggatcctgaggtgaagttcaac

tggtacgtggacggcgtggaagtgcacaacgctaagaccaagcccagagaagagcagtacaactccacctacagagtggtgtctgtgctgaccgttctgcaccaggactggctgaac

ggcaaagagtacaagtgcaaggtctccaacaaggctctggccgcacccatcgagaagacaatcagcaaggccaagggccagcctagagaacctcaagtgtgcaccctgcctccaa

gccgcgaggaaatgaccaagaaccaggtgtccctgtcctgtgccgtgaaaggcttctacccctccgacatcgccgtggaatgggagtccaatggccagcctgagaacaactacaaga

ccacccctcctgtgctggactccgatggctccttcttcctggtctccaagctgacagtggacaagtctagatggcagcagggcaacgtgttctcgtgctccgtgatgcacgaggccctgc

ataatcactatacccagaagtctctgtctctgagccctggcaag (SEQ ID NO: 87)

**Anti-CD3-scFv-Fc of GC35 and GC39**

ELVVTQEPSLTTSPGGTVTLTCRSSTGAVTTSNYANWVQQKPGQAPRGLIGGTNKRAPGTPARFSGSLLGGKAALTIT

GVQPEDEAEYYCALWYSNLWVFGGGTKLTVLGGGGSGGGGSGGGGSEVQLVESGGGLVQPGGSLRLSCAASGFTF

NTYAMNWVRQAPGKGLEWVARIRSKYNNYATYYADSVKDRFTISRDDSKNTAYLQMNNLKTEDTAMYYCVRHGN

FGNSYVSWFAYWGQGTLVTVSSGEPKSSDKTHTCPPCPAPEAAGGPSVFLFPPKPKDTLMISRTPEVTCVVVDVSHED

PEVKFNWYVDGVEVHNAKTKPREEQYNSTYRVVSVLTVLHQDWLNGKEYKCKVSNKALAAPIEKTISKAKGQPRE

PQVYTLPPCREEMTKNQVSLWCLVKGFYPSDIAVEWESNGQPENNYKTTPPVLDSDGSFFLYSKLTVDKSRWQQGN

VFSCSVMHEALHNRYTQKSLSLSPGK (SEQ ID NO: 88)

(continued)

| Description<br>Sequence (SEQ ID NO:) |
|---|
| gagctggtggtgacccaagagccttccctgaccacctccctggtggcacagtgaccctgacatgcagatcctctaccggcgctgtgacaacgtccaattacgccaattgggtccaacagaagcctggccaggccccagaggcctgatcggaggcaccaacaagagagccccaggcaccctgctcggttctctggctctctgctgggcggcaaggctgccctgaccatcaccggcgtgcagcccgaggacgaggccgagtactactgtgctctgtggtattccaacctgtgggtctttggcggcggcaccaagctgaccgtgctgggaggcggcggctctggcggcggcggcagcggaggcggaggctccgaggtgcagctggtagaatctggcggcggactagtgcagcctggcggcagcctgagactgtcttcgccgcctccggcttcacctttaacacatacgcgatgaactgggtgcggcaggctcctggcaagggcctggaatgggtggcccggatccggtccaagtacaacaactatgctacctattacgccgactccgtgaaggacagattcaccatctcccgggacgacagcaagaacaccgcctacctgcagatgaacaacctgaagaccgaggataccgccatgtactactgcgtgcggcacggcaacttcggtaattcctacgtctcttggttcgcctactggggccagggaacactggtgaccgtgtccagcggcgagcctaagtcctccgataaaacccacacctgtcctccttgccctgccctgaagctgctggcggccctccgtgtttctgttcccccctaagcccaaggacacctgatgatctccagaaccctgaggtgacatgtgtggtggtggacgtgtcccacgaggaccctgaagtgaagttcaactggtacgtggatggcgtggaagtgcacaacgccaagaccaagccccgcgaggaacagtacaactccacctacagagtggtcagcgttctcaccgtgctgcatcaagactggctgaacggcaagagtacaagtgcaaggtgtctaacaaggccctggccgctcctatcgagaagaccatctctaaagccaaaggacagccaagggaacctcaggtctacaccctgcctccatgccgggaagagatgaccaagaaccaggtgtctctctggtgcctggtcaagggcttctacccttctgatatcgccgtggagtgggaatccaatggccagcctgagaacaactacaagacaaccccccctgtgctggactccgacggctccttcttcctgtactctaagctgaccgtggacaagtctagatggcagcagggcaacgtgttctcctgctccgtgatgcacgaggctctgcacaacagatacacccagaaatcgctgtctctgagccctggaaag (SEQ ID NO: 89) |
| Anti-GPRC5D-HC of talquetamab<br>QVQLVQSGAEVKKPGASVKVSCKASGYSFTGYTMNWVRQAPGQGLEWMGLINPYNSDTNYAQKLQGRVTMTTDTSTSTAYMELRSLRSDDTAVYYCARVALRVALDYWGQGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEAAGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFFLYSRLTVDKSRWQEGNVFSCSVMHEALHNHYTQKSLSLSLGK (SEQ ID NO: 90) |
| Anti-GPRC5D-LC of talquetamab<br>DIQMTQSPSSLSASVGDRVTITCKASQNVATHVGWYQQKPGKAPKRLIYSASYRYSGVPSRFSGSGSGTEFTLTISNLQPEDFATYYCQQYNRYPYTFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 91) |
| Anti-CD3-HC of talquetamab<br>EVQLVESGGGLVQPGGSLRLSCAASGFTFNTYAMNWVRQAPGKGLEWVARIRSKYNNYATYYAASVKGRFTISRDDSKNSLYLQMNSLKTEDTAVYYCARHGNFGNSYVSWFAYWGQGTLVTVSSASTKGPSVFPLAPCSRSTSESTAALGCLVKDYFPEPVTVSWNSGALTSGVHTFPAVLQSSGLYSLSSVVTVPSSSLGTKTYTCNVDHKPSNTKVDKRVESKYGPPCPPCPAPEAAGGPSVFLFPPKPKDTLMISRTPEVTCVVVDVSQEDPEVQFNWYVDGVEVHNAKTKPREEQFNSTYRVVSVLTVLHQDWLNGKEYKCKVSNKGLPSSIEKTISKAKGQPREPQVYTLPPSQEEMTKNQVSLTCLVKGFYPSDIAVEWESNGQPENNYKTTPPVLDSDGSFLLYSKLTVDKSRWQEGNVFSCSVMHEALHNHYTQKSLSLSLGK (SEQ ID NO: 92) |
| Anti-CD3-LC of talquetamab<br>QTVVTQEPSLTVSPGGTVTLTCRSSTGAVTTSNYANWVQQKPGQAPRGLIGGTNKRAPGTPARFSGSLLGGKAALTLSGVQPEDEAEYYCALWYSNLWVFGGGTKLTVLGQPKAAPSVTLFPPSSEELQANKATLVCLISDFYPGAVTVAWKADSSPVKAGVETTTPSKQSNNKYAASSYLSLTPEQWKSHRSYSCQVTHEGSTVEKTVAPTECS (SEQ ID NO: 93) |
| HCDR1 of anti-CD3 antibody<br>TYAMN (SEQ ID NO: 94) |
| HCDR2 of anti-CD3 antibody |
| RIRSKYNNYATYYADSVKD (SEQ ID NO: 95) |

(continued)

| Description<br>Sequence (SEQ ID NO:) |
| --- |
| HCDR3 of anti-CD3 antibody<br>HGNFGNSYVSWFAY (SEQ ID NO: 96) |
| LCDR1 of anti-CD3 antibody<br>RSSTGAVTTSNYAN (SEQ ID NO: 97) |
| LCDR2 of anti-CD3 antibody<br>GTNKRAP (SEQ ID NO: 98) |
| LCDR3 of anti-CD3 antibody<br>ALWYSNLWV (SEQ ID NO: 99) |
| VH of anti-CD3 antibody<br>EVQLVESGGGLVQPGGSLRLSCAASGFTFNTYAMNWVRQAPGKGLEWVARIRSKYNNYATYYADSVKDRFTISRDD<br>SKNTAYLQMNNLKTEDTAMYYCVRHGNFGNSYVSWFAYWGQGTLVTVSS (SEQ ID NO: 100) |
| VL of anti-CD3 antibody<br>ELVVTQEPSLTTSPGGTVTLTCRSSTGAVTTSNYANWVQQKPGQAPRGLIGGTNKRAPGTPARFSGSLLGGKAALTIT<br>GVQPEDEAEYYCALWYSNLWVFGGGTKLTVL (SEQ ID NO: 101) |
| First hinge<br>EPKSCDKTHTCPPCP (SEQ ID NO: 102) |
| Second hinge<br>GEPKSSDKTHTCPPCP (SEQ ID NO: 103) |
| Peptide linker<br>GGGGS (SEQ ID NO: 104) |

All patents, patent applications, and other identified publications are explicitly incorporated herein by reference for the purpose of description and disclosure. These publications are provided solely because they were disclosed prior to the filing date of the present disclosure. All statements as to the dates of these documents or descriptions as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art. Although the present disclosure has been described in detail herein above through general explanations and specific embodiments, it will be apparent to those skilled in the art that modifications or improvements can be made based on the present disclosure. Accordingly, such modifications and improvements made without departing from the spirit of the present disclosure all fall within the protection scope of the present disclosure.

**Claims**

1.    A bispecific antibody, comprising a first antigen-binding portion binding to GPRC5D and a second antigen-binding portion binding to CD3, wherein the first antigen-binding portion comprises: HCDR1 comprising the amino acid sequence set forth in SEQ ID NO: 1, 9, 17, 25, or 33, HCDR2 comprising the amino acid sequence set forth in SEQ ID NO: 2, 59, 10, 18, 26, or 34, HCDR3 comprising the amino acid sequence set forth in SEQ ID NO: 3, 11, 19, 27, or 35, LCDR1 comprising the amino acid sequence set forth in SEQ ID NO: 4, 12, 20, 28, or 36, LCDR2 comprising the amino acid sequence set forth in SEQ ID NO: 5, 13, 21, 29, or 37, and LCDR3 comprising the amino acid sequence set forth in SEQ ID NO: 6, 14, 22, 30, or 38.

2.    The bispecific antibody according to claim 1, wherein the first antigen-binding portion comprises:

(1) HCDR1 comprising the amino acid sequence set forth in SEQ ID NO: 1, HCDR2 comprising the amino acid sequence set forth in SEQ ID NO: 59, HCDR3 comprising the amino acid sequence set forth in SEQ ID NO: 3, LCDR1 comprising the amino acid sequence set forth in SEQ ID NO: 4, LCDR2 comprising the amino acid sequence set forth in SEQ ID NO: 5, and LCDR3 comprising the amino acid sequence set forth in SEQ ID NO: 6, wherein $X_1$ is selected from D and E, and $X_2$ is selected from G and A;

(2) HCDR1 comprising the amino acid sequence set forth in SEQ ID NO: 1, HCDR2 comprising the amino acid sequence set forth in SEQ ID NO: 2, HCDR3 comprising the amino acid sequence set forth in SEQ ID NO: 3, LCDR1 comprising the amino acid sequence set forth in SEQ ID NO: 4, LCDR2 comprising the amino acid sequence set forth in SEQ ID NO: 5, and LCDR3 comprising the amino acid sequence set forth in SEQ ID NO: 6;

(3) HCDR1 comprising the amino acid sequence set forth in SEQ ID NO: 1, HCDR2 comprising the amino acid sequence set forth in SEQ ID NO: 60, HCDR3 comprising the amino acid sequence set forth in SEQ ID NO: 3, LCDR1 comprising the amino acid sequence set forth in SEQ ID NO: 4, LCDR2 comprising the amino acid sequence set forth in SEQ ID NO: 5, and LCDR3 comprising the amino acid sequence set forth in SEQ ID NO: 6;

(4) HCDR1 comprising the amino acid sequence set forth in SEQ ID NO: 1, HCDR2 comprising the amino acid sequence set forth in SEQ ID NO: 61, HCDR3 comprising the amino acid sequence set forth in SEQ ID NO: 3, LCDR1 comprising the amino acid sequence set forth in SEQ ID NO: 4, LCDR2 comprising the amino acid sequence set forth in SEQ ID NO: 5, and LCDR3 comprising the amino acid sequence set forth in SEQ ID NO: 6;

(5) HCDR1 comprising the amino acid sequence set forth in SEQ ID NO: 9, HCDR2 comprising the amino acid sequence set forth in SEQ ID NO: 10, HCDR3 comprising the amino acid sequence set forth in SEQ ID NO: 11, LCDR1 comprising the amino acid sequence set forth in SEQ ID NO: 12, LCDR2 comprising the amino acid sequence set forth in SEQ ID NO: 13, and LCDR3 comprising the amino acid sequence set forth in SEQ ID NO: 14;

(6) HCDR1 comprising the amino acid sequence set forth in SEQ ID NO: 17, HCDR2 comprising the amino acid sequence set forth in SEQ ID NO: 18, HCDR3 comprising the amino acid sequence set forth in SEQ ID NO: 19, LCDR1 comprising the amino acid sequence set forth in SEQ ID NO: 20, LCDR2 comprising the amino acid sequence set forth in SEQ ID NO: 21, and LCDR3 comprising the amino acid sequence set forth in SEQ ID NO: 22;

(7) HCDR1 comprising the amino acid sequence set forth in SEQ ID NO: 25, HCDR2 comprising the amino acid sequence set forth in SEQ ID NO: 26, HCDR3 comprising the amino acid sequence set forth in SEQ ID NO: 27, LCDR1 comprising the amino acid sequence set forth in SEQ ID NO: 28, LCDR2 comprising the amino acid sequence set forth in SEQ ID NO: 29, and LCDR3 comprising the amino acid sequence set forth in SEQ ID NO: 30; or

(8) HCDR1 comprising the amino acid sequence set forth in SEQ ID NO: 33, HCDR2 comprising the amino acid sequence set forth in SEQ ID NO: 34, HCDR3 comprising the amino acid sequence set forth in SEQ ID NO: 35, LCDR1 comprising the amino acid sequence set forth in SEQ ID NO: 36, LCDR2 comprising the amino acid sequence set forth in SEQ ID NO: 37, and LCDR3 comprising the amino acid sequence set forth in SEQ ID NO: 38.

3. The bispecific antibody according to claim 1 or 2, wherein the first antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, 63, 64, 65, 66, 7, 15, 23, 31, 39, or 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67, 8, 16, 24, 32, 40, or 69;

preferably, the first antigen-binding portion comprises:

(1) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;

(2) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;

(3) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 23, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 24;

(4) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 31, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 32;

(5) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 39, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 40;

(6) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(7) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(8) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(9) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(10) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 66, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67; or

(11) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69;

further preferably, the first antigen-binding portion comprises:

(1) a heavy chain variable region comprising the amino acid sequence set forth in SEQ ID NO: 7 and a light chain variable region comprising the amino acid sequence set forth in SEQ ID NO: 8;

(2) a heavy chain variable region comprising the amino acid sequence set forth in SEQ ID NO: 15 and a light chain variable region comprising the amino acid sequence set forth in SEQ ID NO: 16;

(3) a heavy chain variable region comprising the amino acid sequence set forth in SEQ ID NO: 23 and a light chain variable region comprising the amino acid sequence set forth in SEQ ID NO: 24;

(4) a heavy chain variable region comprising the amino acid sequence set forth in SEQ ID NO: 31 and a light chain variable region comprising the amino acid sequence set forth in SEQ ID NO: 32;

(5) a heavy chain variable region comprising the amino acid sequence set forth in SEQ ID NO: 39 and a light chain variable region comprising the amino acid sequence set forth in SEQ ID NO: 40;

(6) a heavy chain variable region comprising the amino acid sequence set forth in SEQ ID NO: 62, and a light chain variable region comprising the amino acid sequence set forth in SEQ ID NO: 67;

(7) a heavy chain variable region comprising the amino acid sequence set forth in SEQ ID NO: 63, and a light chain variable region comprising the amino acid sequence set forth in SEQ ID NO: 67;

(8) a heavy chain variable region comprising the amino acid sequence set forth in SEQ ID NO: 64, and a light chain variable region comprising the amino acid sequence set forth in SEQ ID NO: 67;

(9) a heavy chain variable region comprising the amino acid sequence set forth in SEQ ID NO: 65, and a light chain variable region comprising the amino acid sequence set forth in SEQ ID NO: 67;

(10) a heavy chain variable region comprising the amino acid sequence set forth in SEQ ID NO: 66, and a light chain variable region comprising the amino acid sequence set forth in SEQ ID NO: 67; or

(11) a heavy chain variable region comprising the amino acid sequence set forth in SEQ ID NO: 68, and a light chain variable region comprising the amino acid sequence set forth in SEQ ID NO: 69.

4. The bispecific antibody according to claim 1, wherein the first antigen-binding portion comprises: HCDR1 comprising the amino acid sequence set forth in SEQ ID NO: 1, HCDR2 comprising the amino acid sequence set forth in SEQ ID NO: 2, HCDR3 comprising the amino acid sequence set forth in SEQ ID NO: 3, LCDR1 comprising the amino acid sequence set forth in SEQ ID NO: 4, LCDR2 comprising the amino acid sequence set forth in SEQ ID NO: 5, and LCDR3 comprising the amino acid sequence set forth in SEQ ID NO: 6;

preferably, the first antigen-binding portion comprises: a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67; preferably, the first antigen-binding portion comprises: a heavy chain variable region comprising the amino acid sequence set forth in SEQ ID NO: 62, and a light chain variable region comprising the amino acid sequence set forth in SEQ ID NO: 67.

5. The bispecific antibody according to any one of claims 1-4, wherein the second antigen-binding portion comprises: HCDR1 comprising the amino acid sequence set forth in SEQ ID NO: 94, HCDR2 comprising the amino acid sequence set forth in SEQ ID NO: 95, HCDR3 comprising the amino acid sequence set forth in SEQ ID NO: 96, LCDR1 comprising the amino acid sequence set forth in SEQ ID NO: 97, LCDR2 comprising the amino acid sequence set forth in SEQ ID NO: 98, and LCDR3 comprising the amino acid sequence set forth in SEQ ID NO: 99.

6. The bispecific antibody according to any one of claims 1-5, wherein the second antigen-binding portion comprises a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 100, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 101;

preferably, the second antigen-binding portion comprises: a heavy chain variable region comprising the amino acid sequence set forth in SEQ ID NO: 100, and a light chain variable region comprising the amino acid sequence set forth in SEQ ID NO: 101.

7. The bispecific antibody according to any one of claims 1-6, wherein the first antigen-binding portion is murine, chimeric, or humanized;

optionally, the second antigen-binding portion is murine, chimeric, or humanized.

8. The bispecific antibody according to any one of claims 1-7, wherein the bispecific antibody further comprises an Fc domain composed of two Fc polypeptides;

preferably, the Fc domain is an IgG Fc domain, preferably an IgG1 Fc domain;
preferably, the IgG Fc domain is a human IgG Fc domain, preferably a human IgG1 Fc domain.

9. The bispecific antibody according to claim 8, wherein the first antigen-binding portion is a Fab, the second antigen-binding portion is an scFv, the first antigen-binding portion, at the C-terminus of its Fab heavy chain, is fused to the N-terminus of one of the Fc polypeptides of the Fc domain, and the second antigen-binding portion, at the C-terminus thereof, is fused to the N-terminus of the other Fc polypeptide of the Fc domain;

optionally, the structure of the second antigen-binding portion from the N-terminus to the C-terminus is VH-VL or VL-VH; optionally, the heavy chain variable region of the second antigen-binding portion is fused to the light chain variable region of the second antigen-binding portion via a peptide linker, and preferably, the peptide linker is (GGGGS)$_3$.

10. The bispecific antibody according to claim 8 or 9, wherein the Fc domain comprises:

(1) an amino acid substitution that promotes association of the two Fc polypeptides of the Fc domain;
(2) an amino acid substitution that reduces the binding affinity of the Fc domain to an Fc receptor and/or reduces effector function; and/or
(3) an amino acid substitution that reduces or eliminates the binding of a CH3 region of one Fc polypeptide in the Fc domain to Protein A;

preferably, according to the EU numbering, one of the Fc polypeptides of the Fc domain comprises amino acid substitutions L234A, L235A, P329A, Y349C, T366S, L368A, and Y407V, and the other Fc polypeptide comprises amino acid substitutions (a) L234A, L235A, P329A, S354C, T366W, and H435R, or (b) L234A, L235A, P329A, S354C, T366W, H435R, and Y436F.

11. The bispecific antibody of any one of claims 1-10, wherein the bispecific antibody consists of three polypeptide chains, wherein:

(1) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 84, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 78, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 88; or
(2) one polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 86, another polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 82, and the other polypeptide chain comprises an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 88;

preferably, the bispecific antibody consists of three polypeptide chains, wherein:

(1) one polypeptide chain comprises the amino acid sequence set forth in SEQ ID NO: 84, another polypeptide chain comprises the amino acid sequence set forth in SEQ ID NO: 78, and the other polypeptide chain comprises the amino acid sequence set forth in SEQ ID NO: 88; or
(2) one polypeptide chain comprises the amino acid sequence set forth in SEQ ID NO: 86, another polypeptide chain comprises the amino acid sequence set forth in SEQ ID NO: 82, and the other polypeptide chain comprises the amino acid sequence set forth in SEQ ID NO: 88.

12. An isolated nucleic acid, comprising a nucleotide sequence encoding the bispecific antibody according to any one of claims 1-11.

13. A host cell, comprising the nucleic acid according to claim 12.

14. A method for preparing the bispecific antibody according to any one of claims 1-11, comprising culturing the host cell according to claim 13 to express the bispecific antibody, and isolating and purifying the bispecific antibody in a system.

15. A pharmaceutical composition, comprising the bispecific antibody according to any one of claims 1-11 and a pharmaceutically acceptable excipient.

16. A method for treating a GPRC5D-expressing disease, comprising administering to a subject in need a therapeutically effective amount of the bispecific antibody according to any one of claims 1-11 or the pharmaceutical composition according to claim 15;
wherein preferably, the disease is an autoimmune disease or a tumor; the tumor is preferably a non-solid tumor, more preferably multiple myeloma.

17. An antibody binding to GPRC5D or an antigen-binding portion thereof, comprising a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, 63, 64, 65, 66, or 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67 or 69; preferably, the antibody binding to GPRC5D or the antigen-binding portion thereof comprises:

(1) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 62, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(2) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 63, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(3) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 64, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(4) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 65, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 67;

(5) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 66, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69; or

(6) a heavy chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 68, and a light chain variable region which has an amino acid sequence having at least 85%, 86%, 87%, 88%, 89%, 90%, 91%, 92%, 93%, 94%, 95%, 96%, 97%, 98%, 99%, or 100% identity to the amino acid sequence set forth in SEQ ID NO: 69;

further preferably, the antibody binding to GPRC5D or the antigen-binding portion thereof comprises:

(1) a heavy chain variable region comprising the amino acid sequence set forth in SEQ ID NO: 62, and a light chain variable region comprising the amino acid sequence set forth in SEQ ID NO: 67;
(2) a heavy chain variable region comprising the amino acid sequence set forth in SEQ ID NO: 63, and a light chain variable region comprising the amino acid sequence set forth in SEQ ID NO: 67;
(3) a heavy chain variable region comprising the amino acid sequence set forth in SEQ ID NO: 64, and a light chain variable region comprising the amino acid sequence set forth in SEQ ID NO: 67;
(4) a heavy chain variable region comprising the amino acid sequence set forth in SEQ ID NO: 65, and a light chain variable region comprising the amino acid sequence set forth in SEQ ID NO: 67; or
(5) a heavy chain variable region comprising the amino acid sequence set forth in SEQ ID NO: 68, and a light chain variable region comprising the amino acid sequence set forth in SEQ ID NO: 69.

18. An antibody-drug conjugate, comprising the antibody or the antigen-binding portion thereof according to claim 17.

19. An anti-GPRC5D chimeric antigen receptor, comprising the antibody or the antigen-binding portion thereof according to claim 17.

20. An engineered immune effector cell, comprising the anti-GPRC5D chimeric antigen receptor according to claim 19.

FIG. 1

| Antibody No. | BM-mAb | Chi-89 | Chi-105 | Chi-128 | Chi-131 | Chi-164 | Chi-169 | Chi-180 |
|---|---|---|---|---|---|---|---|---|
| $EC_{50}$ (nM) | 0.37 | 0.70 | 1.44 | 0.76 | 0.66 | 0.36 | 0.66 | 0.56 |

FIG. 2

FIG. 3

FIG. 4A

FIG. 4B

FIG. 5A

FIG. 5B

FIG. 6A

FIG. 6B

FIG. 6C

FIG. 7

FIG. 8

First antigen-binding portion

Second antigen-binding portion

FIG. 9

RPMI-8226

FIG. 10A

MM.1S

FIG. 10B

**NCI-H929**

FIG. 10C

FIG. 11

**NCI-H929**

FIG. 12A

**MM.1S**

FIG. 12B

**RPMI-8226**

FIG. 12C

FIG. 13A

FIG. 13B

FIG. 13C

FIG. 13D

FIG. 14A

FIG. 14B

FIG. 14C

FIG. 14D

FIG. 15

## INTERNATIONAL SEARCH REPORT

| International application No. |
| --- |
| **PCT/CN2024/089962** |

| A. | CLASSIFICATION OF SUBJECT MATTER |
| --- | --- |

C07K16/28(2006.01)i; A61K39/395(2006.01)i; A61P35/02(2006.01)i

According to International Patent Classification (IPC) or to both national classification and IPC

| B. | FIELDS SEARCHED |
| --- | --- |

Minimum documentation searched (classification system followed by classification symbols)

IPC:C07K; A61K; A61P

Documentation searched other than minimum documentation to the extent that such documents are included in the fields searched

Electronic data base consulted during the international search (name of data base and, where practicable, search terms used)

CNTXT, USTXT, WOTXT, VEN, CNABS, PubMed, ISI Web of Knowledge, CNKI, NCBI, CJFD, baidu, STN, 中国专利生物序列检索, China Patent Biological Sequence Search: GPRC5D, CD3, 双特异性抗体, 正大天晴药业集团股份有限公司, 张兵, 陆亚敏, 杜敏, 陆臻桢, 陆苗苗, SEQ ID NOs: 1-69, 84-86 and 95-101, 抗原结合部分, bispecific antibody, antigen binding domain

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 109715667 A (JANSSEN PHARMACEUTICA NV) 03 May 2019 (2019-05-03) claims 1, 24, 27, 49 and 52-64 | 1-20 |
| A | CN 111788231 A (F. HOFFMANN-LA ROCHE AG) 16 October 2020 (2020-10-16) abstract, and claims 1-41 | 1-20 |
| A | WO 2021207681 A1 (THE BOARD OF TRUSTEES OF THE LELAND STANFORD JUNIOR UNIVERSITY) 14 October 2021 (2021-10-14) claims 1-21 | 1-20 |
| A | PILLARISETTI, K. et al. "A T-Cell–Redirecting Bispecific G-Protein–Coupled Receptor Class 5 Member D x CD3 Antibody to Treat Multiple Myeloma" *Blood*, Vol. 135, No. (15), 09 April 2020 (2020-04-09), pp. 1232-1243 | 1-20 |

| ☑ Further documents are listed in the continuation of Box C. | ☑ See patent family annex. |
| --- | --- |

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **01 August 2024** | **06 August 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District. Beijing 100088** | |
| | Telephone No. |

Form PCT/ISA/210 (second sheet) (July 2022)

**EP 4 703 384 A1**

**ʃTERNATIONAL SEARCH REPORT**

| International application No. |
| --- |
| **PCT/CN2024/089962** |

| C. | DOCUMENTS CONSIDERED TO BE RELEVANT | |
| --- | --- | --- |
| Category* | Citation of document, with indication, where appropriate, of the relevant passages | Relevant to claim No. |
| A | KODAMA, T. et al. "Anti-GPRC5D/CD3 Bispecific T-Cell--Redirecting Antibody for the Treatment of Multiple Myeloma" *Molecular Cancer Therapeutics*, Vol. 18, No. (9), 03 July 2019 (2019-07-03), pp. 1555-1564 | 1-20 |
| A | LANCMAN, G. et al. "Bispecific Antibodies in Multiple Myeloma: Present and Future" *Blood Cancer Discovery*, Vol. vol. 2, 17 August 2021 (2021-08-17), pp. 423-433 | 1-20 |

Form PCT/ISA/210 (second sheet) (July 2022)

76

| **ITERNATIONAL SEARCH REPORT** | International application No. |
|---|---|
| | **PCT/CN2024/089962** |

**Box No. I   Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

       ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/CN2024/089962**

| **Box No. II** | **Observations where certain claims were found unsearchable (Continuation of item 2 of first sheet)** |

This international search report has not been established in respect of certain claims under Article 17(2)(a) for the following reasons:

1. ☑ Claims Nos.: **16**
because they relate to subject matter not required to be searched by this Authority, namely:

Claim 16 relates to a method for treating a disease expressing GPRC5D, which falls within methods for treatment of diseases as defined in PCT Rule 39.1(iv). However, a search is still performed on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
|---|
| **PCT/CN2024/089962** |

| Patent document cited in search report | | | Publication date (day/month/year) | Patent family member(s) | | | Publication date (day/month/year) |
|---|---|---|---|---|---|---|---|
| CN | 109715667 | A | 03 May 2019 | AR | 109499 | A1 | 19 December 2018 |
| | | | | CR | 20190025 | A | 14 March 2019 |
| | | | | JP | 2024054176 | A | 16 April 2024 |
| | | | | ZA | 201901066 | B | 28 October 2020 |
| | | | | BR | 112019001055 | A2 | 01 October 2019 |
| | | | | NI | 201900005 | A | 10 May 2019 |
| | | | | UY | 37340 | A | 31 January 2018 |
| | | | | TW | 201809005 | A | 16 March 2018 |
| | | | | TWI | 781108 | B | 21 October 2022 |
| | | | | KR | 20190028534 | A | 18 March 2019 |
| | | | | KR | 102572091 | B1 | 31 August 2023 |
| | | | | ECSP | 19012075 | A | 28 February 2019 |
| | | | | EP | 3487882 | A2 | 29 May 2019 |
| | | | | PH | 12019500152 | A1 | 14 October 2019 |
| | | | | US | 2023227548 | A1 | 20 July 2023 |
| | | | | US | 11884722 | B2 | 30 January 2024 |
| | | | | MX | 2023006449 | A | 15 June 2023 |
| | | | | US | 2023084967 | A1 | 16 March 2023 |
| | | | | US | 11685777 | B2 | 27 June 2023 |
| | | | | CL | 2019000146 | A1 | 26 April 2019 |
| | | | | EA | 201990346 | A1 | 28 June 2019 |
| | | | | CA | 3031472 | A1 | 25 January 2018 |
| | | | | KR | 20230128409 | A | 04 September 2023 |
| | | | | SG | 11201900468 | YA | 27 February 2019 |
| | | | | AU | 2017299673 | A1 | 07 February 2019 |
| | | | | IL | 264334 | A | 28 February 2019 |
| | | | | IL | 264334 | B1 | 01 January 2024 |
| | | | | IL | 264334 | B2 | 01 May 2024 |
| | | | | MA | 45712 | A | 29 May 2019 |
| | | | | IL | 298277 | A | 01 January 2023 |
| | | | | JP | 2019527061 | A | 26 September 2019 |
| | | | | JP | 7292200 | B2 | 16 June 2023 |
| | | | | WO | 2018017786 | A2 | 25 January 2018 |
| | | | | WO | 2018017786 | A3 | 01 March 2018 |
| | | | | JP | 2023011774 | A | 24 January 2023 |
| | | | | JP | 7469432 | B2 | 16 April 2024 |
| | | | | US | 2018037651 | A1 | 08 February 2018 |
| | | | | US | 10562968 | B2 | 18 February 2020 |
| | | | | PE | 20190392 | A1 | 13 March 2019 |
| | | | | CO | 2019001114 | A2 | 19 February 2019 |
| | | | | PE | 20240884 | A1 | 24 April 2024 |
| | | | | MX | 2019000825 | A | 04 September 2019 |
| CN | 111788231 | A | 16 October 2020 | US | 2023212308 | A1 | 06 July 2023 |
| | | | | TW | 201936641 | A | 16 September 2019 |
| | | | | TWI | 829667 | B | 21 January 2024 |
| | | | | RU | 2020129004 | A | 09 March 2022 |
| | | | | KR | 20200119833 | A | 20 October 2020 |
| | | | | PE | 20201341 | A1 | 25 November 2020 |
| | | | | EP | 3749692 | A1 | 16 December 2020 |
| | | | | US | 2024067749 | A1 | 29 February 2024 |

Form PCT/ISA/210 (patent family annex) (July 2022)

| Patent document cited in search report | Publication date (day/month/year) | Patent family member(s) | | | Publication date (day/month/year) |
|---|---|---|---|---|---|
| | | CO | 2020008940 | A2 | 31 August 2020 |
| | | IL | 276537 | A | 30 September 2020 |
| | | MX | 2020007012 | A | 07 September 2020 |
| | | MA | 51734 | A | 19 May 2021 |
| | | JP | 2021513334 | A | 27 May 2021 |
| | | JP | 7513521 | B2 | 09 July 2024 |
| | | CL | 2023000907 | A1 | 17 November 2023 |
| | | AU | 2019219061 | A1 | 06 August 2020 |
| | | CR | 20200341 | A | 02 November 2020 |
| | | WO | 2019154890 | A1 | 15 August 2019 |
| | | US | 2021054094 | A1 | 25 February 2021 |
| | | CL | 2020001854 | A1 | 23 October 2020 |
| | | SG | 11202007578 | SA | 29 September 2020 |
| | | BR | 112020015297 | A2 | 08 December 2020 |
| | | CA | 3088730 | A1 | 15 August 2019 |
| | | PH | 12020551211 | A1 | 17 May 2021 |
| | | AR | 117392 | A1 | 04 August 2021 |
| | | JP | 2023159115 | A | 31 October 2023 |
| WO 2021207681 A1 | 14 October 2021 | EP | 4132582 | A1 | 15 February 2023 |

Form PCT/ISA/210 (patent family annex) (July 2022)

## REFERENCES CITED IN THE DESCRIPTION

### Patent documents cited in the description

- CN 202310484445
- WO 2019195535 A
- WO 2018017786 A2

### Non-patent literature cited in the description

- Sequences of Proteins of Immunological Interest. **ELVIN A. KABAT et al.** Public Health Service. National Institutes of Health, 1991
- **CYRUS CHOTHIA et al.** Canonical Structures for the Hypervariable Regions of Immunoglobulins. *J. Mol. Biol.*, 1987, vol. 196, 901-917
- Sequences of Proteins of Immunological Interest. **KABAT, E.A. et al.** Public Health Service. National Institutes of Health, 1991